# EUROPEAN PATENT APPLICATION

(11) **EP 4 467 146 A2**
(43) Date of publication of application: **27.11.2024**
(21) Application number: 24187137.5
(22) Date of filing: 17.09.2018
(51) Int. Cl.: A61K 35/17

(54) **COMPOSITIONS AND METHODS FOR ENHANCING GAMMA DELTA T CELLS IN THE GUT**

(30) Priority: 15.09.2017 US 201762559225 P
(62) Divisional of application: 18782647.4
(71) Applicant: King's College London, London WC2R 2LS (GB)
(72) Inventor: HAYDAY, Adrian, London, SE1 9RT (GB); DART, Robin John Campbell, London, SE1 9RT (GB); PRESCOTT, Natalie, London, SE1 9RT (GB); VANTOUROUT, Pierre, London, SE1 9RT (GB); ZLATAREVA, Iva, London, SE1 9RT (GB)
(74) Representative: Mewburn Ellis LLP

(57) **Abstract**

The invention features compositions and methods useful in treating inflammation of the gut, such as inflammation associated with inflammatory bowel disease, by modulating γδ T cells (e.g., Vy4+ cells). Particular embodiments include Vy4+ cells expressing heterologous protein; polynucleotides containing genes contributing to functional expression of BTNL3, BTNL8, and HNF4A; methods of treating a subject using Vy4+ cells or gene therapy; and methods of identifying a subject having mutations associated with inflammation of the gut. Compositions and methods of the invention can be used in the treatment of inflammation and cancer of the gut.

## Description

This application claims priority from US62/559225, filed 15 Sept 2017, the contents and elements of which are herein incorporated by reference for all purposes.

### FIELD

The present invention relates to the activation and enhancement of γδ T cells in the gut, for example for the treatment of Inflammatory Bowel Disease (IBD).

### BACKGROUND

Inflammatory Bowel Disease (IBD) is characterized by chronic inflammation of the gastrointestinal (GI) tract. Over three million residents in the United States and two-and-a-half million in Europe are estimated to have IBD, and the global prevalence of IBD has risen as IBD has emerged in newly industrialized countries in Asia, South America, and the Middle East. The gut contains a variety of immune cells that maintain intestinal homeostasis, and in response to an abnormal immune cell composition or activity within the gut, IBD can occur.

There is a need in the field for new treatments for IBD that involve modulating the immune response in the gut. Human gut epithelial cells have been reported to express BTNL3 and BTNL8. These factors are reported to jointly induce selective TCR-dependent responses of human colonic Vγ4+ cells (Di Marco Barros (22 September 2016) Cell 167 203-218). However, details of the interaction of BTNL3 and BTNL8 with intraepithelial lymphocytes (IELs) in the gut, including the receptor to which they bind on IELs such as γδ T cells (Chapoval (2013) Mol Immunol 56 819-828), and the role of Vγ4+ cells in IBD and other gut inflammatory disorders, remain to be established.

### SUMMARY

The present inventors have found that atypical levels or activity of Vy4+ cells are associated with human gut inflammation. Atypical levels or activity of Vγ4+ cells may result from reduced Butyrophilin 3 (BTNL3) and/or Butyrophilin 8 (BTNL8) function in the human gut. These findings may be useful for example in the diagnosis and treatment of conditions associated with gut inflammation, such as IBD, for example ulcerative colitis and/or Crohn's disease.

The present invention in various aspects provides compositions and methods for treating inflammation in the gut associated with atypical levels of Vγ4+ cells or Vγ4+ cell activity in the gut (e.g., loss of Vy4+ cell presence or activity associated with a mutation in BTNL3 and/or BTNL8 and/or loss of function of BTNL3 and/or BTNL8 in the gut, e.g., as a result of a mutation or loss of function of HNF4A). Compositions and methods of aspects of the invention involve polynucleotides encoding BTNL3 and BTNL8, and/or HNF4A to adjust expression of BTNL3 and BTNL8, which can impact inflammation of the gut (e.g. IBD) by recruiting, retaining, or otherwise influencing the activity of Vy4+ cells in the gut of a patient (e.g. a subject heterozygous or homozygous for a mutation in a polynucleotide encoding BTNL3, BTNL8, and/or HNF4A). Compositions and methods also involve administration of Vγ4+ cells to treat inflammation of the gut (e.g. IBD, e.g. in a subject heterozygous for a mutation in a polynucleotide encoding BTNL3, BTNL8, and/or HNF4A) or cancer of the gut. The Vγ4+ cells may be recombinant cells, for example cells which express a heterologous protein,

In a first aspect, the invention provides a Vγ4+ cell expressing a heterologous protein. In some embodiments, the Vγ4+ cell is derived from a V4- cell and Vγ4 is the heterologous protein. The Vγ4- cell may be a mammalian cell of any suitable cell type, including an immune cell (e.g., an innate lymphoid cell, a monocyte, a macrophage, a dendritic cell, a neutrophil, an eosinophil, a basophil, a mast cell, or a lymphocyte, such as a T cell, a B cell, or an NK cell). In some embodiments, the immune cell is a lymphocyte. In some embodiments, the lymphocyte is a T cell (e.g., a CD8 T cell or a CD4 T cell) or an NK cell. In some embodiments, the T cell is a γδ T cell. In some embodiments, the γδ T cell is a Vδ2 cell (e.g., a Vγ9δ2 cell). In some embodiments, the Vy4+ cell is derived from a human cell. In some embodiments, the Vy4+ cell is derived from an induced pluripotent stem cell. In some embodiments, Vγ4 is an endogenously expressed protein (e.g., as in the case of an endogenous Vγ4δ1+ cell) and the heterologous protein expressed by the Vy4+ cell is a protein other than Vγ4.

In some embodiments, the Vy4+ cell expressing a heterologous protein is for use in the manufacture of a medicament. In some embodiments, the Vy4+ cell expressing a heterologous protein, or the medicament thereof, is for use in treating inflammation in the gut of a subject (e.g., IBD, for example ulcerative colitis and/or Crohn's disease) or cancer (e.g., colorectal cancer, colon cancer, rectal cancer, anal cancer, hereditary nonpolyposis colorectal cancer (HNPCC), familial adenomatous polyposis (FAP), small intestine cancer (e.g., adenocarcinoma, sarcoma, gastrointestinal carcinoid tumours, lymphoma, or gastrointestinal stromal tumours). For example, the Vy4+ cell expressing a heterologous protein, or the medicament thereof, can be used in a method of treating inflammation in the gut of a subject having a decreased expression level of BTNL3 and/or BTNL8, relative to a reference expression level (e.g., as a consequence of being heterozygous for a mutation in a polynucleotide encoding BTNL3, BTNL8, and/or HNF4A, e.g., a gene encoding BTNL3, BTNL8, and/or HNF4A). The reference expression level can be a wild-type expression level. In another example, Vy4+ cell expressing a heterologous protein, or the medicament thereof, can be used in a method of increasing a number or frequency of the Vy4+ cells in the gut of a subject (e.g., as a consequence of being heterozygous for a mutation in a polynucleotide encoding BTNL3, BTNL8, and/or HNF4A, e.g., a gene encoding BTNL3, BTNL8, and/or HNF4A).

In a second aspect, the invention provides a composition containing a population of Vy4+ cells according to the first aspect. The population may contain 10⁶-10¹⁰ cells (e.g., from 1 × 10⁶ cells to 5 × 10⁶ cells, from 5 × 10⁶ cells to 1 × 10⁷ cells, from 1 × 10⁷ cells to 5 × 10⁷ cells, from 5 × 10⁷ cells to 1 × 10⁸ cells, from 1 × 10⁸ cells to 5 × 10⁸ cells, from 5 × 10⁸ cells to 1 × 10⁹ cells, from 1 × 10⁹ cells to 5 × 10⁹ cells, from 5 × 10⁹ cells to 1 × 10¹⁰ cells, e.g., about 1 × 10⁶ cells, about 1.5 × 10⁶ cells, about 2 × 10⁶ cells, about 3 × 10⁶ cells, about 5 × 10⁶ cells, about 1 × 10⁷ cells, about 1.5 × 10⁷ cells, about 2 × 10⁷ cells, about 3 × 10⁷ cells, about 5 × 10⁷ cells, about 1 × 10⁸ cells, about 2 × 10⁸ cells, about 3 × 10⁸ cells, about 5 × 10⁸ cells, about 1 × 10⁹ cells, about 2 × 10⁹ cells, about 3 × 10⁹ cells, about 5 × 10⁹ cells, or about 1 × 10¹⁰ cells). In some embodiments, 10-50% of the population is a population of Vγ4+ T cells (e.g., from 10% to 20%, from 20% to 30%, from 30% to 40%, or from 40% to 50%, e.g., about 10%, about 15%, about 20%, about 25%, about 30%, about 35%, about 40%, about 45%, or about 50%) of the population is a population of Vy4+ T cells. In some embodiments, the number of Vy4+ T cells in the composition is from 1 × 10⁵ to 5 × 10⁹ (e.g., from 1 × 10⁵ cells to 5 × 10⁵ cells, from 5 × 10⁵ cells to 1 × 10⁶, from 1 × 10⁶ cells to 5 × 10⁶ cells, from 5 × 10⁶ cells to 1 × 10⁷ cells, from 1 × 10⁷ cells to 5 × 10⁷ cells, from 5 × 10⁷ cells to 1 × 10⁸ cells, from 1 × 10⁸ cells to 5 × 10⁸ cells, from 5 × 10⁸ cells to 1 × 10⁹ cells, or from 1 × 10⁹ cells to 5 × 10⁹ cells, e.g., about 1 × 10⁵ cells, about 1.5 × 10⁵ cells, about 2 × 10⁵ cells, about 3 × 10⁵ cells, about 5 × 10⁵ cells, about 1 × 10⁶ cells, about 1.5 × 10⁶ cells, about 2 × 10⁶ cells, about 3 × 10⁶ cells, about 5 × 10⁶ cells, about 1 x 10⁷ cells, about 1.5 x 10⁷ cells, about 2 x 10⁷ cells, about 3 x 10⁷ cells, about 5 x 10⁷ cells, about 1 × 10⁸ cells, about 2 × 10⁸ cells, about 3 × 10⁸ cells, about 5 × 10⁸ cells, about 1 × 10⁹ cells, about 2 × 10⁹ cells, about 3 × 10⁹ cells, or about 5 × 10⁹ cells). In some embodiments, the Vy4+ cells express a heterologous protein (e.g., Vγ4 or a protein other than Vγ4). For example, the cells in the population may be Vy4+ cells of the first aspect. The composition may further comprise one or more additional components, for example a pharmaceutically acceptable excipient or diluent.

In a third aspect, the invention provides a vector containing a polynucleotide sequence encoding a Vγ4 protein and a polynucleotide sequence encoding a Vδ1 protein (e.g., a polycistronic polynucleotide encoding a Vγ4 protein and a Vδ1 protein). In some embodiments, the vector further includes a polynucleotide sequence encoding a CD3 protein. In some embodiments, the vector containing one or more polynucleotides encoding a Vγ4 protein and a Vδ1 protein is for use in transducing a Vγ4δ1 receptor in a T cell (e.g., a non-Vy4+ cell, e.g., an αβ T cell (e.g., a CD8 T cell, a CD4 T cell, or a regulatory T cell), or a non-Vy4+ γδ cell, such as a Vδ2 cell). In some embodiments, the vector containing one or more polynucleotides encoding a Vγ4 protein, a Vδ1 protein, and a CD3 protein is for use in transducing a non-T cell (e.g., a monocyte, a macrophage, a dendritic cell, an NK cell, or a B cell) to express a Vδ1γ4 receptor. In some embodiments, the polynucleotide is DNA. In other embodiments, the polynucleotide is RNA. In some embodiments, the vector is a viral vector, such as a lentiviral vector, an adenoviral vector, or an adeno-associated viral (AAV) vector.

In a fourth aspect, the invention provides a vector containing a polynucleotide sequence encoding a Vγ4 protein and a polynucleotide sequence encoding a Vδ3 protein (e.g., a polycistronic polynucleotide encoding a Vγ4 protein and a Vδ3 protein). In some embodiments, the vector further includes a polynucleotide sequence encoding a CD3 protein. In some embodiments, the vector containing one or more polynucleotides encoding a Vγ4 protein and a Vδ3 protein is for use in transducing a Vγ4δ3 receptor in a T cell (e.g., a non-Vγ4+ cell, e.g., an αβ T cell (e.g., a CD8 T cell, a CD4 T cell, or a regulatory T cell), or a non-Vy4+ γδ cell, such as a Vδ2 cell). In some embodiments, the vector containing one or more polynucleotides encoding a Vγ4 protein, a Vδ3 protein, and a CD3 protein is for use in transducing a non-T cell (e.g., a monocyte, a macrophage, a dendritic cell, an NK cell, or a B cell) to express a Vγ4δ3 receptor. In some embodiments, the polynucleotide is DNA. In other embodiments, the polynucleotide is RNA. In some embodiments, the vector is a viral vector, such as a lentiviral vector, an adenoviral vector, or an adeno-associated viral (AAV) vector.

In some embodiments, the vector containing a polynucleotide sequence encoding a Vγ4 protein, a Vδ1 protein or a Vδ3 protein, and/or a CD3 protein is for use in the manufacture of a population of Vy4+ T cells. In some embodiments, the population of Vy4+ T cells is for treating inflammation in the gut of a subject having a decreased expression level of BTNL3 and/or BTNL8, relative to a reference expression level (e.g., as a consequence of being heterozygous for a mutation in a polynucleotide encoding BTNL3, BTNL8, and/or HNF4A, e.g., a gene encoding BTNL3, BTNL8, and/or HNF4A).

In some embodiments, the number of Vy4+ T cells in the population manufactured using the vector containing a polynucleotide sequence encoding a Vγ4 protein, a Vδ1 protein or a Vδ3 protein, and/or a CD3 protein is from 1 × 10⁵ to 5 × 10⁹ (e.g., from 1 × 10⁵ cells to 5 × 10⁵ cells, from 5 × 10⁵ cells to 1 × 10⁶, from 1 × 10⁶ cells to 5 × 10⁶ cells, from 5 × 10⁶ cells to 1 × 10⁷ cells, from 1 × 10⁷ cells to 5 × 10⁷ cells, from 5 × 10⁷ cells to 1 × 10⁸ cells, from 1 × 10⁸ cells to 5 × 10⁸ cells, from 5 × 10⁸ cells to 1 × 10⁹ cells, or from 1 × 10⁹ cells to 5 ×10⁹ cells, e.g., about 1 × 10⁵ cells, about 1.5 × 10⁵ cells, about 2 × 10⁵ cells, about 3 ×10⁵ cells, about 5 × 10⁵ cells, about 1 × 10⁶ cells, about 1.5 × 10⁶ cells, about 2 × 10⁶ cells, about 3 × 10⁶ cells, about 5 × 10⁶ cells, about 1 × 10⁷ cells, about 1.5 × 10⁷ cells, about 2 × 10⁷ cells, about 3 × 10⁷ cells, about 5 × 10⁷ cells, about 1 × 10⁸ cells, about 2 × 10⁸ cells, about 3 × 10⁸ cells, about 5 × 10⁸ cells, about 1 × 10⁹ cells, about 2 × 10⁹ cells, about 3 × 10⁹ cells, or about 5 × 10⁹ cells).

In a fifth aspect, the invention provides a vector containing a polynucleotide encoding a BTNL3 protein and a polynucleotide encoding a BTNL8 protein (e.g., a polycistronic polynucleotide encoding a BTNL3 protein and a BTNL8 protein). In some embodiments, the vector containing one or more polynucleotides encoding a BTNL3 protein and a BTNL8 protein is for use in transducing a gut epithelial cell (e.g., a gut epithelial cell having a mutation in a polynucleotide encoding BTNL3, BTNL8, and/or HNF4A, e.g., a gut epithelial cell having a heterozygous mutation in a polynucleotide encoding BTNL3, BTNL8, and/or HNF4A or a homozygous mutation in a polynucleotide encoding BTNL3, BTNL8, and/or HNF4A). In some embodiments, the vector further includes an HNF4A promoter. In some embodiments, the vector further includes a polynucleotide encoding an HNF4A protein, which can be useful when a subject is heterozygous or homozygous for a mutation in HNF4A. In some embodiments, the polynucleotide is DNA. In other embodiments, the polynucleotide is RNA. In some embodiments, the vector is a viral vector, such as a lentiviral vector, an adenoviral vector, or an adeno-associated viral (AAV) vector.

In some embodiments, the vector containing a polynucleotide encoding a BTNL3 protein and a polynucleotide encoding a BTNL8 protein is for use in the manufacture of a medicament. In some embodiments, the vector containing a polynucleotide encoding a BTNL3 protein and a polynucleotide encoding a BTNL8 protein is for use in treating inflammation in the gut of a subject (e.g., IBD) or cancer of the gut. For example, the vector containing a polynucleotide encoding a BTNL3 protein and a polynucleotide encoding a BTNL8 protein can be administered to a subject having a decreased expression level of BTNL3 and/or BTNL8, relative to a reference expression level (e.g., as a consequence of being homozygous for a mutation in a polynucleotide encoding BTNL3 or BTNL8 or heterozygous for a mutation in a polynucleotide encoding BTNL3, BTNL8, and/or HNF4A, e.g., a gene encoding BTNL3, BTNL8, and/or HNF4A). The reference expression level can be a wild-type expression level. In another example, the vector containing a polynucleotide encoding a BTNL3 protein and a polynucleotide encoding a BTNL8 protein can be used in a method of increasing a number or frequency of Vγ4+ cells in the gut of a subject (e.g., as a consequence of being homozygous for a mutation in a polynucleotide encoding BTNL3 or BTNL8 or heterozygous for a mutation in a polynucleotide encoding BTNL3, BTNL8, and/or HNF4A, e.g., a gene encoding BTNL3, BTNL8, and/or HNF4A).

In a sixth aspect, the invention provides a cell transduced with the vector of any of third, fourth or fifth aspects or embodiments thereof.

In a seventh aspect, the invention provides a composition including the vector of any of the third, fourth or fifth aspects or embodiments thereof. In some embodiments, the composition is used in a method of increasing the number or frequency of Vγ4+ cells in the gut of a subject. In some embodiments, the composition is used in a method of treating inflammation in the gut of a subject or in a method of treating cancer in the gut of a subject. In some embodiments, the inflammation in the gut of the subject is associated with inflammatory bowel disease (IBD).

In an eighth aspect, the invention provides a method of treating inflammation in the gut of a subject having a decreased expression level of BTNL3 and/or BTNL8, relative to a reference expression level (e.g., a wild-type expression level), by administering to the subject a polynucleotide encoding a BTNL3 protein and a polynucleotide encoding a BTNL8 protein, a cell according to the first aspect, a composition according to the second aspect, a vector according to the third to fifth aspects, a cell according to the sixth aspect or a composition according to the seventh aspect.

In some embodiments, the decreased expression level of BTNL3 and/or BTNL8 is the result of a mutation in a polynucleotide sequence encoding BTNL3 and/or BTNL8. In some embodiments, the mutation is a deletion variant. In some embodiments, the mutation is characterized by reduced or ablated trafficking of BLTN3 and/or BTNL8 to a cell surface. In some embodiments, the mutation is characterized by expression of a BTNL8*3 fusion protein. In other embodiments, the mutation is characterized by one or more variant SNPs in the BTNL-3 and/or BTNL-8 genes, for example the L3B30.2cl genotype. In some embodiments, the mutation is a heterozygous mutation. In some embodiments, the mutation is a homozygous mutation. In some embodiments, the mutation is a single nucleotide polymorphism (SNP). In some embodiments, the mutation is an SNP in a BTNL3 intron. In some embodiments, the polynucleotide encoding a BTNL3 protein and the polynucleotide encoding a BTNL8 protein are on the same expression cassette.

In some embodiments, the polynucleotide encoding a BTNL3 protein and the polynucleotide encoding a BTNL8 protein are encoded by a vector. In some embodiments, the vector is a viral vector (e.g., a lentiviral vector, an adenoviral vector, or an adeno-associated viral (AAV) vector).

In a ninth aspect, the invention provides a method of treating inflammation in the gut of a subject having a decreased expression level of BTNL3 and/or BTNL8, relative to a reference expression level (e.g., a wild-type expression level), by administering to the subject a polynucleotide encoding an HNF4A protein. In some embodiments, the subject has a decreased expression level of HNF4A, relative to a reference population. In some embodiments, the subject has mutation in HNF4A which is associated with decreased expression of BTNL3 and/or BTNL8. In some embodiments, the polynucleotide further includes an HNF4A promoter. In some embodiments, the polynucleotide encoding an HNF4A protein is encoded by a vector, e.g., a viral vector. In some embodiments, the viral vector is a lentiviral vector, an adenoviral vector, or an adeno-associated viral (AAV) vector. In some embodiments, the vector further includes an HNF4A promoter.

In a tenth aspect, the invention provides a method of increasing the number or frequency of Vγ4+ cells in the gut of a subject by administering a population of Vγ4+ cells to the subject, wherein the administered population of Vγ4+ cells has been screened for expression of Vγ4. In some embodiments, the screening for expression of Vγ4 includes determining whether or not Vγ4 is expressed or the degree of Vγ4 expression (e.g., by a percentage of cells that express Vγ4, a mean expression density of Vγ4 (e.g., by mean fluorescence intensity), or any combination thereof). In some embodiments, the population of Vγ4+ T cells administered to the subject is from 1 × 10⁵ to 5 × 10⁹ (e.g., from 1 × 10⁵ cells to 5 × 10⁵ cells, from 5 × 10⁵ cells to 1 × 10⁶, from 1 × 10⁶ cells to 5 × 10⁶ cells, from 5 × 10⁶ cells to 1 × 10⁷ cells, from 1 × 10⁷ cells to 5 × 10⁷ cells, from 5 × 10⁷ cells to 1 × 10⁸ cells, from 1 × 10⁸ cells to 5 × 10⁸ cells, from 5 × 10⁸ cells to 1 × 10⁹ cells, or from 1 × 10⁹ cells to 5 × 10⁹ cells, e.g., about 1 × 10⁵ cells, about 1.5 × 10⁵ cells, about 2 × 10⁵ cells, about 3 × 10⁵ cells, about 5 × 10⁵ cells, about 1 × 10⁶ cells, about 1.5 × 10⁶ cells, about 2 × 10⁶ cells, about 3 × 10⁶ cells, about 5 × 10⁶ cells, about 1 × 10⁷ cells, about 1.5 × 10⁷ cells, about 2 × 10⁷ cells, about 3 × 10⁷ cells, about 5 × 10⁷ cells, about 1 × 10⁸ cells, about 2 × 10⁸ cells, about 3 × 10⁸ cells, about 5 × 10⁸ cells, about 1 × 10⁹ cells, about 2 × 10⁹ cells, about 3 × 10⁹ cells, or about 5 × 10⁹ cells). In some embodiments, the population of Vγ4+ T cells administered to the subject is within a mixed population of cells, including non-Vγ4+ cells. For example, the Vy4+ in the population may account for 10-50% of the total cell population. The population of Vγ4+ T cells may, for example be cells according to the first or sixth aspects of the invention.

In an eleventh aspect, the invention provides a method of increasing the number or frequency of Vγ4+ cells in the gut of a subject by administering a population of Vγ4+ cells to the subject, wherein the subject has a mutation (e.g., a heterozygous mutation) in a polynucleotide sequence encoding BTNL3 and BTNL8. In some embodiments, the population of Vγ4+ T cells administered to the subject is from 1 × 10⁵ to 5 × 10⁹ (e.g., from 1 × 10⁵ cells to 5 × 10⁵ cells, from 5 × 10⁵ cells to 1 × 10⁶, from 1 × 10⁶ cells to 5 ×10⁶ cells, from 5 × 10⁶ cells to 1 × 10⁷ cells, from 1 × 10⁷ cells to 5 × 10⁷ cells, from 5 × 10⁷ cells to 1 × 10⁸ cells, from 1 × 10⁸ cells to 5 × 10⁸ cells, from 5 × 10⁸ cells to 1 × 10⁹ cells, or from 1 × 10⁹ cells to 5 × 10⁹ cells, e.g., about 1 × 10⁵ cells, about 1.5 × 10⁵ cells, about 2 × 10⁵ cells, about 3 × 10⁵ cells, about 5 × 10⁵ cells, about 1 × 10⁶ cells, about 1.5 × 10⁶ cells, about 2 × 10⁶ cells, about 3 × 10⁶ cells, about 5 × 10⁶ cells, about 1 × 10⁷ cells, about 1.5 × 10⁷ cells, about 2 × 10⁷ cells, about 3 × 10⁷ cells, about 5 × 10⁷ cells, about 1 × 10⁸ cells, about 2 × 10⁸ cells, about 3 × 10⁸ cells, about 5 × 10⁸ cells, about 1 × 10⁹ cells, about 2 × 10⁹ cells, about 3 × 10⁹ cells, or about 5 × 10⁹ cells). In some embodiments, the population of Vγ4+ T cells administered to the subject is within a mixed population of cells, including non-Vy4+ cells. For example, the Vy4+ in the population may account for 10-50% of the total cell population. The Vy4+ T cells may, for example be cells according to the first or sixth aspects of the invention.

In a twelfth aspect, the invention provides a method of increasing the number or frequency of Vy4+ cells in the gut of a subject by administering a population of Vy4+ cells to the subject, wherein the subject has inflammation of the gut, such as IBD, or cancer of the gut. In some embodiments, the population of Vy4+ T cells administered to the subject is from 1 × 10⁵ to 5 × 10⁹ (e.g., from 1 × 10⁵ cells to 5 × 10⁵ cells, from 5 × 10⁵ cells to 1 × 10⁶, from 1 × 10⁶ cells to 5 × 10⁶ cells, from 5 × 10⁶ cells to 1 × 10⁷ cells, from 1 × 10⁷ cells to 5 × 10⁷ cells, from 5 × 10⁷ cells to 1 × 10⁸ cells, from 1 × 10⁸ cells to 5 × 10⁸ cells, from 5 × 10⁸ cells to 1 × 10⁹ cells, or from 1 × 10⁹ cells to 5 × 10⁹ cells, e.g., about 1 × 10⁵ cells, about 1.5 × 10⁵ cells, about 2 × 10⁵ cells, about 3 × 10⁵ cells, about 5 × 10⁵ cells, about 1 × 10⁶ cells, about 1.5 × 10⁶ cells, about 2 × 10⁶ cells, about 3 × 10⁶ cells, about 5 × 10⁶ cells, about 1 × 10⁷ cells, about 1.5 × 10⁷ cells, about 2 × 10⁷ cells, about 3 × 10⁷ cells, about 5 × 10⁷ cells, about 1 × 10⁸ cells, about 2 × 10⁸ cells, about 3 × 10⁸ cells, about 5 × 10⁸ cells, about 1 × 10⁹ cells, about 2 × 10⁹ cells, about 3 × 10⁹ cells, or about 5 × 10⁹ cells). In some embodiments, the population of Vγ4+ T cells administered to the subject is within a mixed population of cells, including non-Vy4+ cells. For example, the Vy4+ in the population may account for 10-50% of the total cell population. The population of Vy4+ T cells may, for example be cells according to the first or sixth aspects of the invention.

In some embodiments, the Vy4+ cells administered to the subject to treat inflammation in the gut of a subject, or to increase the number or frequency of Vy4+ cells in the gut of a subject, expresses a heterologous protein. In some embodiments, the Vy4+ cells administered to the subject are derived from Vγ4- cells and Vγ4 is the heterologous protein. The Vγ4- cells may be any suitable cell type, including an immune cell (e.g., monocytes, macrophages, dendritic cells, neutrophils, eosinophils, basophils, mast cells, lymphocytes (e.g., T cells, B cells, NK cells, or a combination thereof), or a combination thereof. In some embodiments, the immune cells include lymphocytes. In some embodiments, the lymphocytes include T cells (e.g., CD8 T cells, CD4 T cells, or regulatory T cells) or NK cells. In some embodiments, the T cells include γδ T cells. In some embodiments, the γδ T cells include Vδ2 cells (e.g., Vγ9δ2 cells).

In some embodiments of any of the first to the twelfth aspects, the Vy4+ cells are derived from human cells. In some embodiments, Vγ4 is an endogenously expressed protein (e.g., as in the case of endogenous Vγ4δ1+ cells) and the heterologous protein expressed by the Vy4+ cell is a protein other than Vγ4.express a heterologous protein. In some embodiments, Vγ4 is an endogenously expressed protein (e.g., as in the case of an endogenous Vδ1γ4+ cell or a Vγ4δ3+) and the heterologous protein expressed by the Vγ4+ cell is a protein other than Vγ4.

In some embodiments of any of the first to the twelfth aspects, the Vy4+ cells administered to the subject endogenously express Vγ4. In some embodiments, the Vy4+ cells administered to the subject have not been modified to express a heterologous protein.

In some embodiments, the population of Vγ4+ cells administered to the subject increases the number of Vγ4+ cells in a population of γδ T cells in the gut of the subject to a number effective to alleviate one or more symptoms of inflammation in the gut or cancer of the gut. In some embodiments, the inflammation in the gut is associated with IBD.

In some embodiments, the method further includes administering one or more additional therapeutic agents to the subject.

In a thirteenth aspect, the invention provides a population of Vy4+ cells for use in a method according to any one of the tenth, eleventh or twelfth aspects. The population of Vy4+ cells may for example be Vy4+ cells of the first aspect.

In a fourteenth aspect, the invention provides a method of identifying a mutation in a polynucleotide sequence encoding BTNL3 and BTNL8 by (a) comparing a level of a polynucleotide sequence associated with a deletion variant in a polynucleotide encoding BTNL3 and BTNL8 in a sample of the subject with a reference level of the polynucleotide sequence associated with the deletion variant, wherein an increased level of the polynucleotide sequence associated with a deletion variant in a polynucleotide encoding BTNL3 and BTNL8 in the sample of the subject, relative to the reference level, indicates the presence of the mutation in a polynucleotide sequence encoding BTNL3 and BTNL8; or (b) comparing a level of a polynucleotide sequence encoding BTNL3 and BTNL8 in a sample of the subject with a reference level of the polynucleotide sequence encoding BTNL3 and BTNL8, wherein a decreased level of the polynucleotide encoding BTNL3 and BTNL8 in the sample of the subject, relative to the reference level, indicates the presence of the mutation in a polynucleotide sequence encoding BTNL3 and BTNL8.

In some embodiments, the reference level is of a sample having wild-type BTNL3 and BTNL8 genes.

In some embodiments, the mutation is a deletion variant. In some embodiments, the mutation is characterized by reduced or ablated trafficking of BLTN3 and/or BTNL8 to a cell surface, e.g., relative to a reference sample, e.g., a wild-type sample.

In some embodiments, the mutation is characterized by expression of a BTNL8*3 fusion protein.

In some embodiments, the mutation is a single nucleotide polymorphism (SNP). In some embodiments, the mutation is an SNP in a BTNL3 intron.

In a fifteenth aspect, the invention provides a method of identifying a subject as susceptible to inflammation in the gut, the method comprising:
providing a sample of cells from the subject, and
determining the presence of a mutation in a polynucleotide sequence encoding HNF4A, BTNL3 and/or BTNL8 in said cells, wherein the presence of said mutation is indicative that the subject is susceptible to inflammation in the gut.

In some embodiments, a method of identifying a subject as likely to develop or at risk of developing inflammation in the gut (e.g., IBD) may comprise by (a) determining whether the subject has a mutation in a polynucleotide sequence encoding BTNL3 and BTNL8; and (b) based on the presence of the mutation, identifying the subject as likely to develop IBD.

In some embodiments, step (a) includes identifying a mutation in a polynucleotide sequence encoding BTNL3 and BTNL8 by (i) comparing a level of a polynucleotide sequence associated with a deletion variant in a polynucleotide encoding BTNL3 and BTNL8 in a sample of the subject with a reference level of the polynucleotide sequence associated with the deletion variant, wherein an increased level of the polynucleotide sequence associated with a deletion variant in a polynucleotide encoding BTNL3 and BTNL8 in the sample of the subject, relative to the reference level, indicates the presence of the mutation in a polynucleotide sequence encoding BTNL3 and BTNL8; or (i) comparing a level of a polynucleotide sequence encoding BTNL3 and BTNL8 in a sample of the subject with a reference level of the polynucleotide sequence encoding BTNL3 and BTNL8, wherein a decreased level of the polynucleotide encoding BTNL3 and BTNL8 in the sample of the subject, relative to the reference level, indicates the presence of the mutation in a polynucleotide sequence encoding BTNL3 and BTNL8. In some embodiments, the reference level is of a sample having wild-type BTNL3 and BTNL8 genes. In some embodiments, the mutation is a deletion variant. In some embodiments, the mutation is characterized by reduced or ablated trafficking of BLTN3 and/or BTNL8 to a cell surface. In some embodiments, the mutation is characterized by expression of a BTNL8*3 fusion protein. In some embodiments, the mutation is a single nucleotide polymorphism (SNP). In some embodiments, the mutation is an SNP in a BTNL3 intron.

In some embodiments, the method further includes (c) providing a recommendation to the subject to pursue gene therapy or selecting the individual for gene therapy, wherein the gene therapy is for induction of BTNL3 and/or BTNL8 expression. For example, the subject may have decreased expression of BTNL3 and/or BTNL8, e.g., as a result of a heterozygous mutation in one or more genes encoding BTNL3, BTNL8, and HNF4A. Alternatively, the subject may have no functional expression of BTNL3 and/or BTNL8, e.g., as a result of a homozygous mutation in one or more genes encoding BTNL3, BTNL8, and HNF4A.

In some embodiments, the gene therapy involves administering to the subject a polynucleotide encoding a BTNL3 protein and a polynucleotide encoding a BTNL8 protein. In some embodiments, the polynucleotide encoding a BTNL3 protein and the polynucleotide encoding a BTNL8 protein are on the same expression cassette. In some embodiments, the polynucleotide encoding a BTNL3 protein and the polynucleotide encoding a BTNL8 protein are encoded by a viral vector. In some embodiments, the viral vector is a lentiviral vector, an adenoviral vector, or an adeno-associated viral (AAV) vector.

In other embodiments, the gene therapy involves administering to the subject a polynucleotide encoding HNF4A protein. In some embodiments, the polynucleotide encoding an HNF4A protein is encoded by a viral vector. In some embodiments, the viral vector is a lentiviral vector, an adenoviral vector, or an AAV vector.

In some embodiments, the method further includes (c) providing a recommendation to the subject to pursue adoptive cell therapy or selecting the subject for adoptive cell therapy. For example, the subject may be recommended or selected for treatment using a method of any one of the tenth, eleventh or twelfth aspects. The adoptive cell therapy may for example involve administering a population of Vγ4+ cells to the subject, wherein the subject has a mutation (e.g., a heterozygous mutation) in a polynucleotide sequence encoding BTNL3 and BTNL8. In some embodiments, the population of Vγ4+ T cells administered to the subject is from 1 × 10⁵ to 5 × 10⁹ (e.g., from 1 × 10⁵ cells to 5 × 10⁵ cells, from 5 × 10⁵ cells to 1 × 10⁶, from 1 × 10⁶ cells to 5 × 10⁶ cells, from 5 × 10⁶ cells to 1 × 10⁷ cells, from 1 × 10⁷ cells to 5 × 10⁷ cells, from 5 × 10⁷ cells to 1 × 10⁸ cells, from 1 × 10⁸ cells to 5 × 10⁸ cells, from 5 × 10⁸ cells to 1 × 10⁹ cells, or from 1 × 10⁹ cells to 5 × 10⁹ cells, e.g., about 1 × 10⁵ cells, about 1.5 × 10⁵ cells, about 2 × 10⁵ cells, about 3 × 10⁵ cells, about 5 × 10⁵ cells, about 1 × 10⁶ cells, about 1.5 × 10⁶ cells, about 2 × 10⁶ cells, about 3 × 10⁶ cells, about 5 × 10⁶ cells, about 1 × 10⁷ cells, about 1.5 × 10⁷ cells, about 2 × 10⁷ cells, about 3 × 10⁷ cells, about 5 × 10⁷ cells, about 1 × 10⁸ cells, about 2 × 10⁸ cells, about 3 × 10⁸ cells, about 5 × 10⁸ cells, about 1 × 10⁹ cells, about 2 × 10⁹ cells, about 3 × 10⁹ cells, or about 5 × 10⁹ cells). In some embodiments, the population of Vγ4+ T cells administered to the subject is within a mixed population of cells, including non-Vγ4+ cells. For example, the Vγ4+ in the population may account for 10-50% of the total cell population.

Any embodiment disclosed in this application may be combined with any other disclosed embodiment, within each of the first to the fourteenth aspects of the invention.

### BRIEF DESCRIPTION OF THE DRAWINGS

Embodiments and experiments illustrating the principles of the invention will now be discussed with reference to the accompanying figures in which:

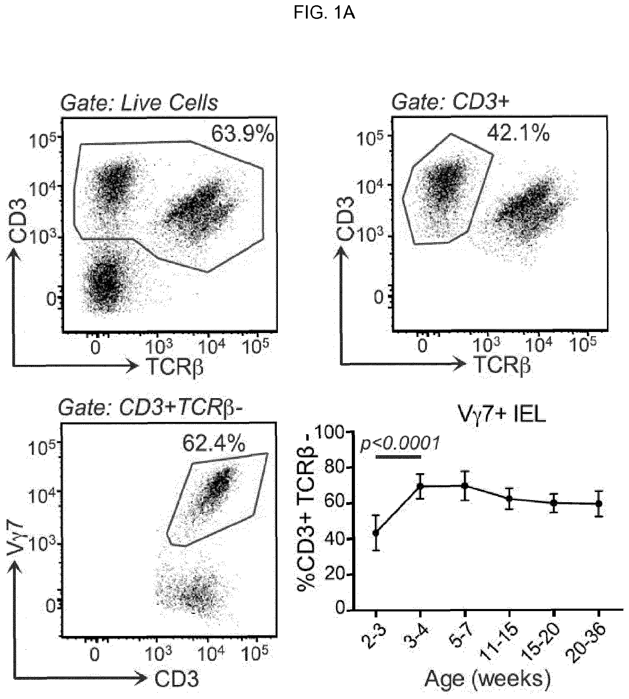

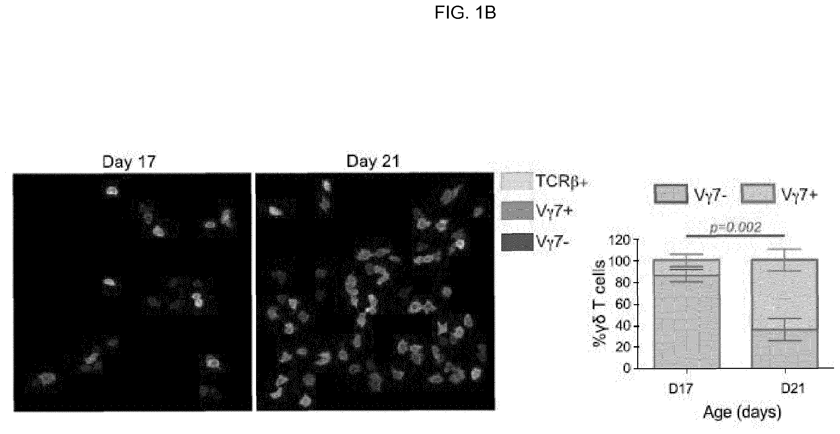

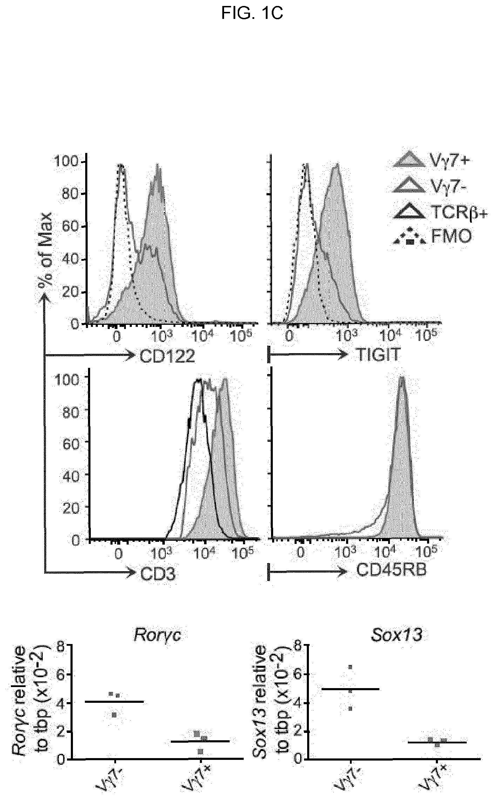

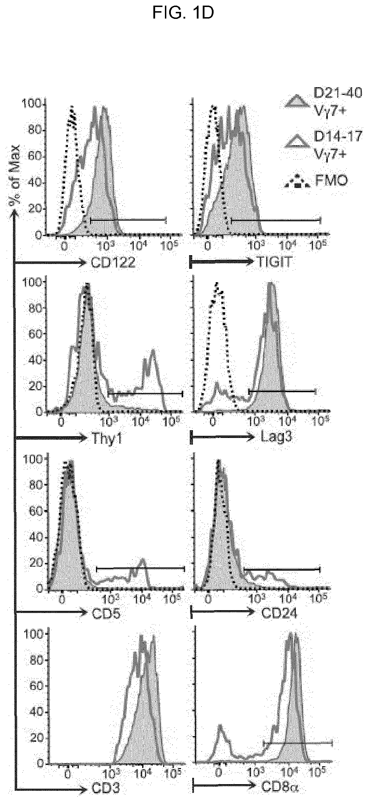

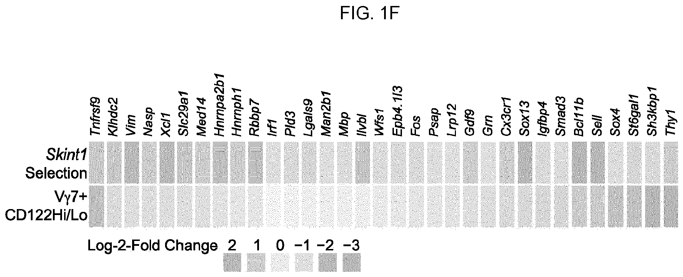

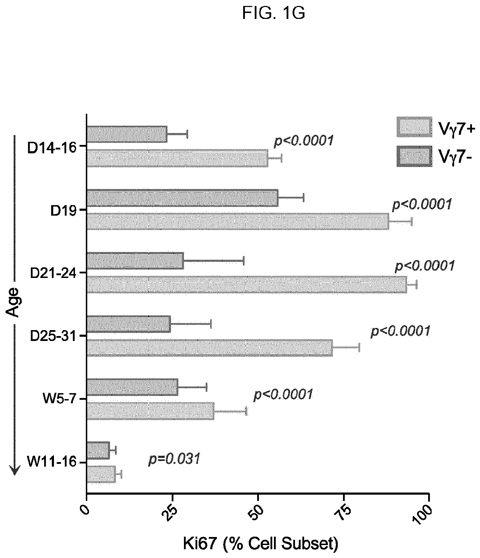

FIGS. 1A-1G are a series of graphs describing the development and maturation of murine intraepithelial lymphocytes (IELs). Small intestinal Vy7+ IELs developed by 2-3 weeks of age and remained stable for at least 9 months (FIG. 1A). By day 21, most IELs were Vγ7+ in whole mount small intestine (SI) confocal microscopy (FIG. 1B). Vγ7+ IELs 21-to 40-day-old mice had high surface expression of CD122, TIGIT, CD3 (TCR), and CD45RB, and low expression of Roryc and Sox13 (FIG. 1C). In contrast, Vγ7- and αβ IELs had low surface levels of CD122 and TIGIT, Vγ7- IELs expressed higher levels of Roryc and Sox13 (FIG. 1C). Surface levels of various markers in Vy7+ IELs were increased at days 21-40 compared to days 14-17 (CD122, TIGIT, Lag3, CD8α), with immature Vy7+ IELs having a more similar phenotype to mature Vγ7- IELs (FIGS. 1D-1E). Differentially expressed genes between Vγ7+CD122^{HI} and Vγ7+CD122^{LO} IELs from day 14-17 mice were similar to differentially expressed genes between Skint1-selected and non-selected Vy5+ dendritic epidermal T cell (DETC) progenitors (FIG. 1F). Vy7+ IELs also had greater Ki67 expression than Vγ7- IELs, indicative of cell cycling (FIG. 1G). D, day; W, week. All error bars represent mean ± SD.

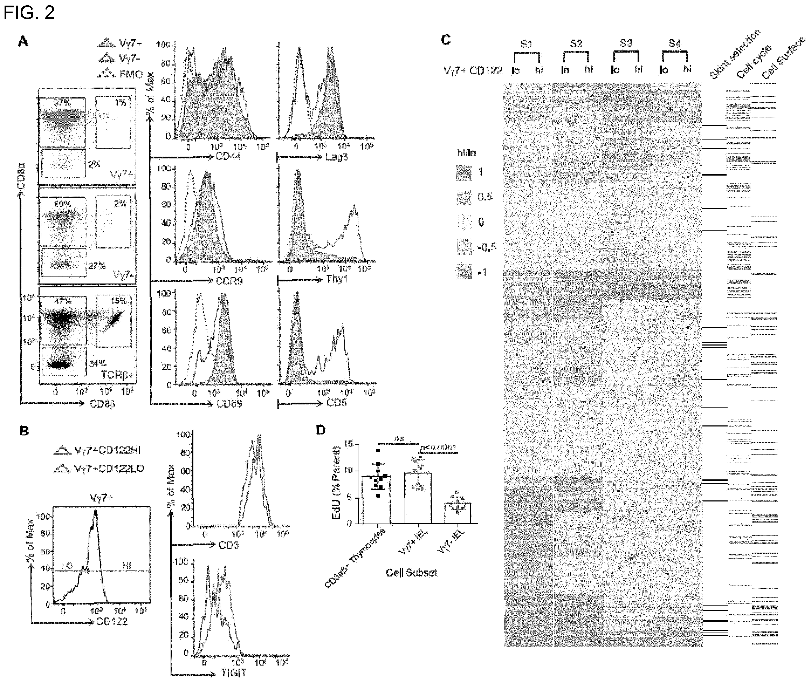

FIGS. 2A-2D are a series of graphs showing phenotypic differences between CD122^{HI} Vγ7+ IELs and other IEL subsets. Vγ7+, Vγ7-, and TCRβ+ IELs express different cell surface markers. Vy7+ expressed higher surface levels of Lag3, CD69, and CD8αα+, and lower levels of Thy1 and CD5 (FIG. 2A). WT Vγ7+CD122^{HI} IELs had a different cell surface phenotype than Vγ7+CD122^{LO} IELs (FIG. 2B). Vy7+CD122 ^{HI} and Vy7+CD122 ^{LO} IELs from D14-D17 WT mice showed differential gene expression (log-2-FoldChange) of many cell surface proteins. Many of the same genes were also regulated by Skint1-selection of DETC progenitors (FIG. 2C). Vy7+ IELs incorporated significantly more EdU than Vy7+ IELs, indicative of increased cell division (FIG. 2D). All error bars represent mean ± SD.

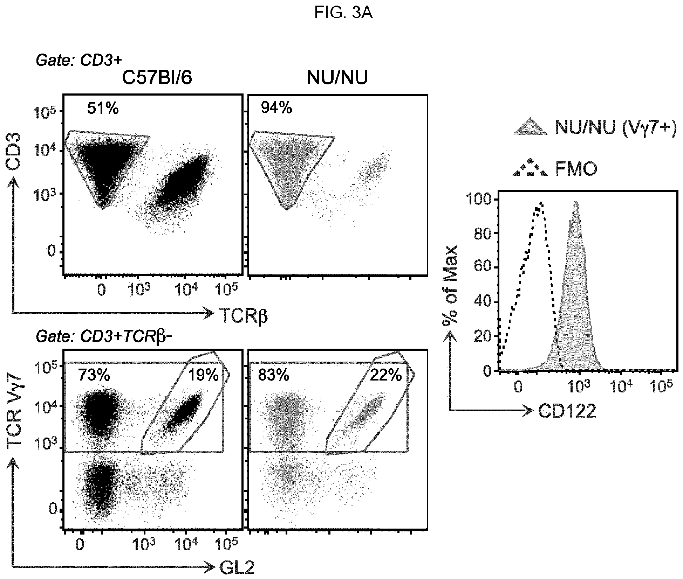

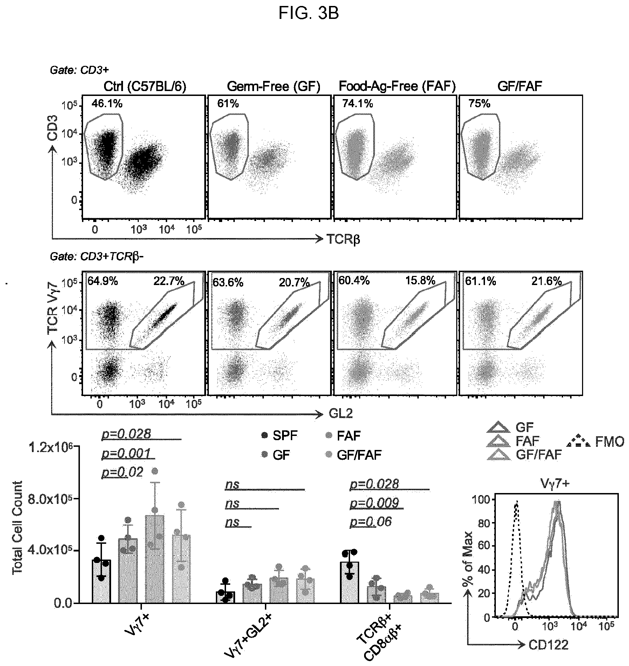

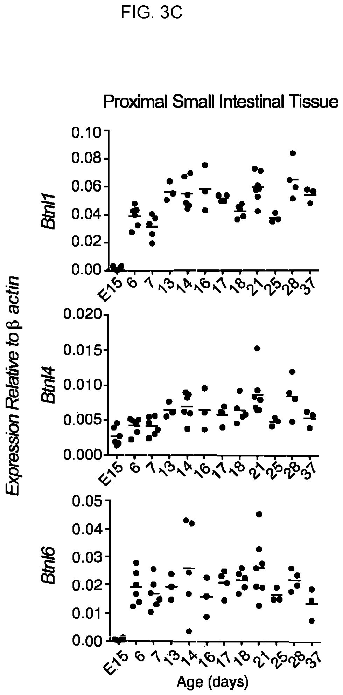

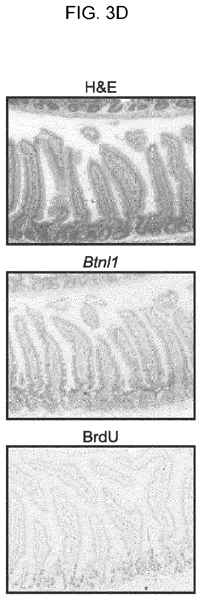

FIGS 3A-3E are a series of graphs demonstrating that endogenous intestinal elements shape IELs. IELs were present in athymic NU/NU mice, and antibody GL2 detected Vδ4 (TRDV2-2-encoded) chains in IELs (FIG. 3A). Vy7+ IELs were similar in CD122 and TCR surface levels between mice in conventional housing and mice raised in germ-free conditions, fed a protein-antigen-free diet, or both, indicating that Vy7+ IELs are likely regulated by endogenous rather than environmental factors (FIG. 3B). Btnl1, Btnl4, and Btnl6 were expressed in proximal SI, with levels stabilizing approximately 14 days after birth (FIG. 3C). Histological analysis showed Btnl1, Btnl4, and Btnl6 were expressed in post-mitotic vilus enterocytes that are interspersed with IELs (FIGS. 3D-3E). All error bars represent mean ± SD.

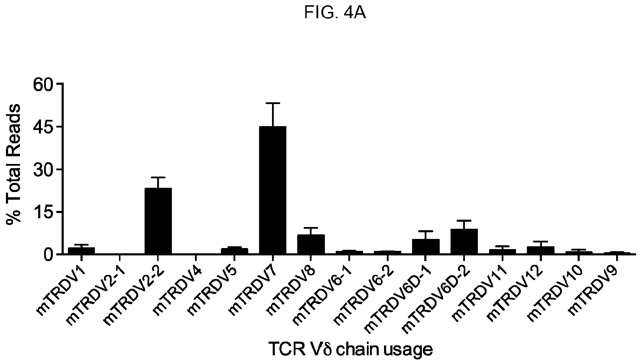

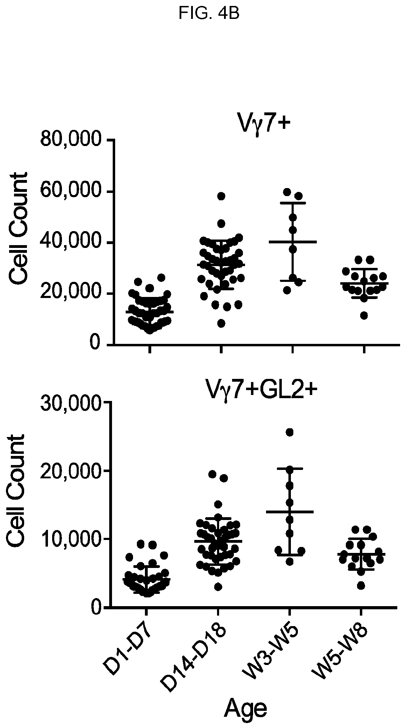

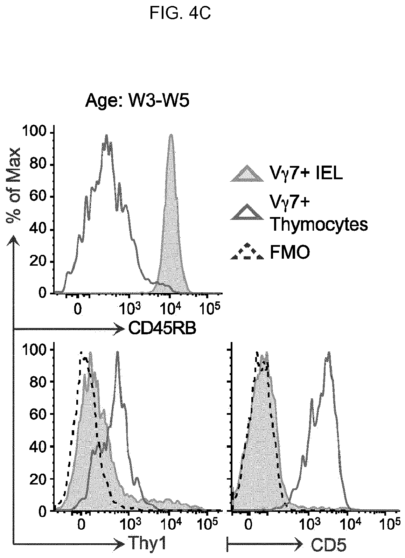

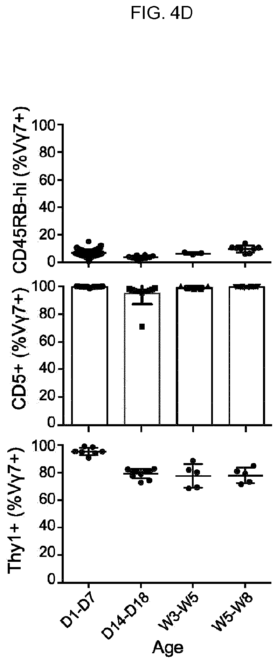

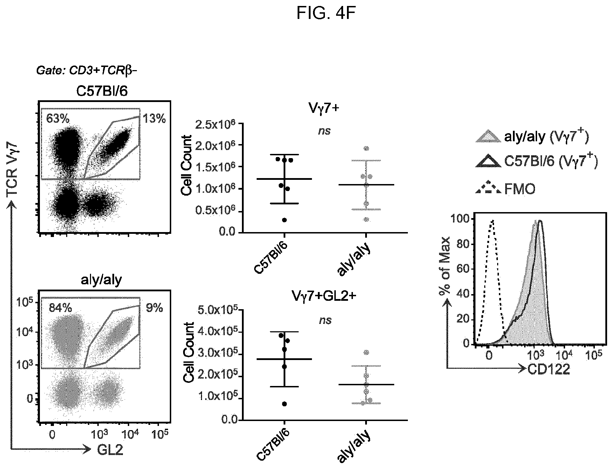

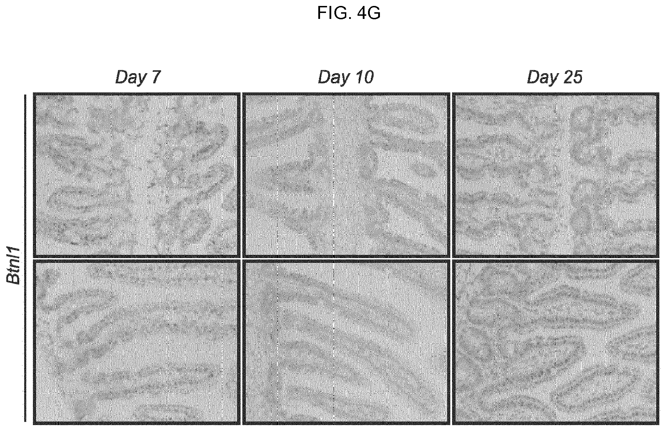

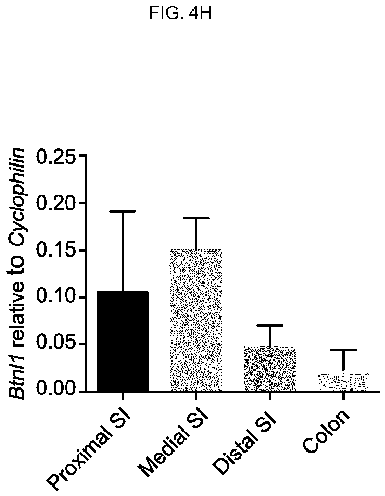

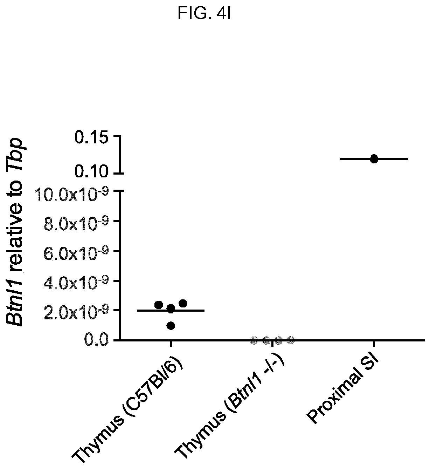

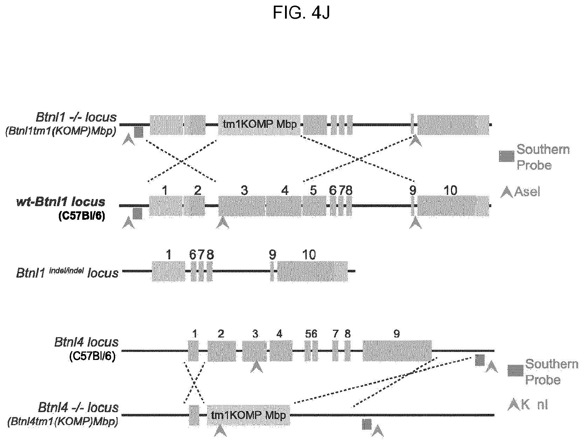

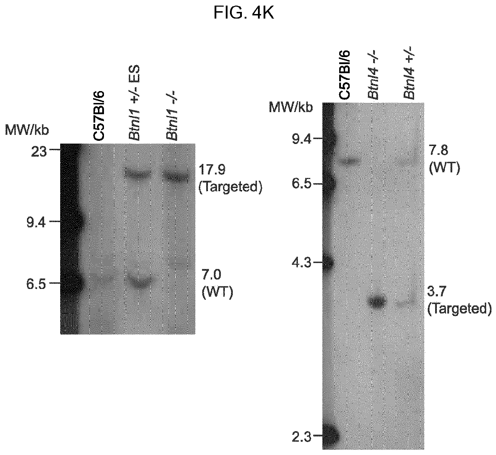

FIGS. 4A-4K are a series of graphs and schematics characterizing the local intestinal development of CD122^{HI} Vγ7+ IELs. Deep sequencing of TCR Vδ chain usage in WT Vy7+ IEL sorted from W7-10 C57BI/6 (WT) mice showed approximately 25% TRVD2-2 (FIG. 4A). Vy7+ and Vy7+GL2+ thymocytes were not observed in large numbers in the first 8 weeks of life (FIG. 4B). Vy7+ thymocytes express cell surface markers that indicate a lack of maturation compared to Vγ7+ IELs (CD5+, Thy1+, and CD122, CD45RB, and TCR low) (FIGS. 4C-4E). Mice lacking lymph nodes and Peyer's patches (aly/aly) developed Vγ7+ and Vγ7+GL2+ IELs (FIG. 4F). Btnl1 was expressed in post-mitotic villus enterocytes that are interspersed with IELs (FIG. 4G) and Btnl1 expression was highest in the proximal and medial SI (FIG. 4H), and expressed much more highly in the proximal SI than the thymus (FIG. 4I). Schematic showing targeted loci in Btnl1-/- and Btnl1-/- mice (FIG. 4J). Southern blots were used to confirm that Btnl1 and Btnl4 genomic DNA sequences were not present in knockout mice (FIG. 4K). All error bars represent mean ± SD.

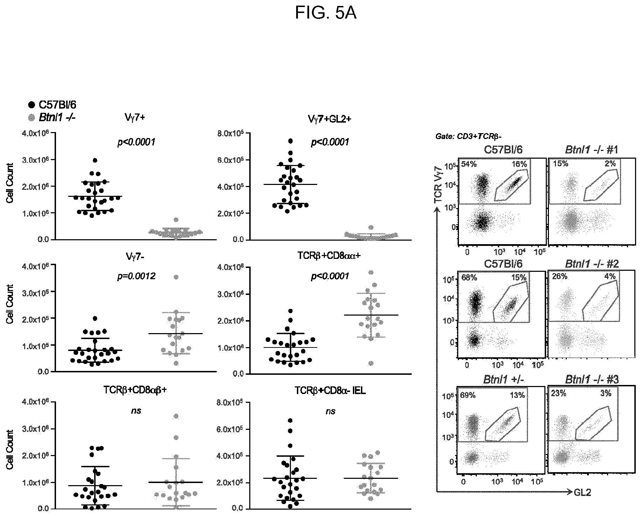

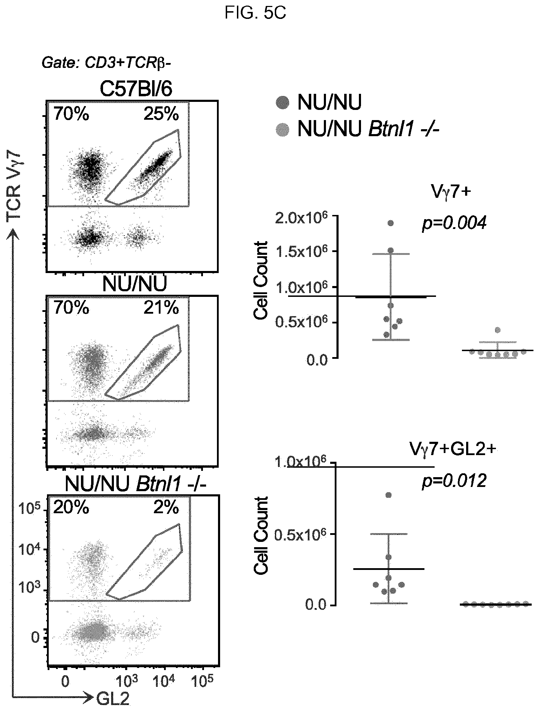

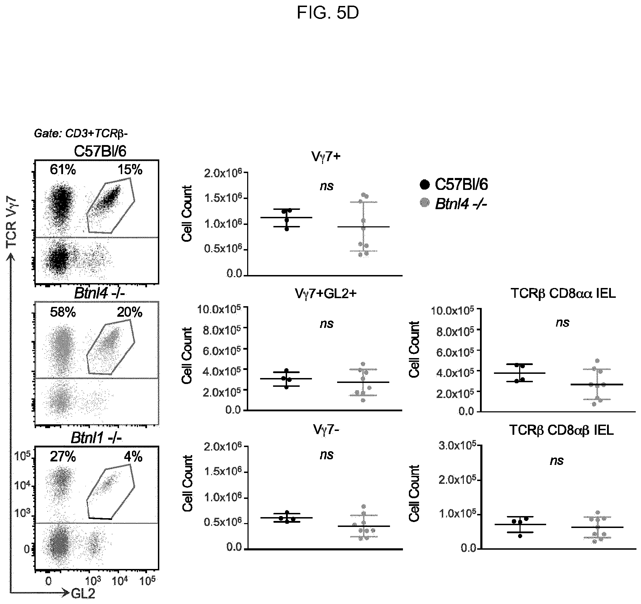

FIGS. 5A-5D are a series of graphs demonstrating that gut IEL composition depends on Btnl1. Vy7+ and Vy7+GL2+ IELs were reduced in Btnl1-/- mice, while other IEL cell types were unaffected (FIGS. 5A-5B). The decrease in Vγ7+ and Vγ7+GL2+ IELs was also observed in Btnl-/- NU/NU mice, indicating that Btnl acts extrathymically (FIG. 5C). Vγ7+ and Vγ7+GL2+ IELs were not reduced in Btnl4-/- mice, indicating that Vγ7+ and Vγ7+GL2+ IELs are specifically dependent on Btnl1 (FIG. 5D). All error bars represent mean ± SD.

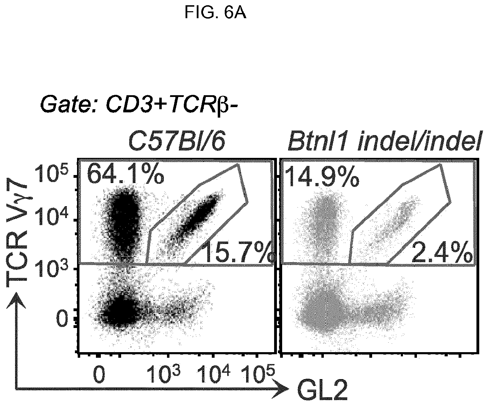

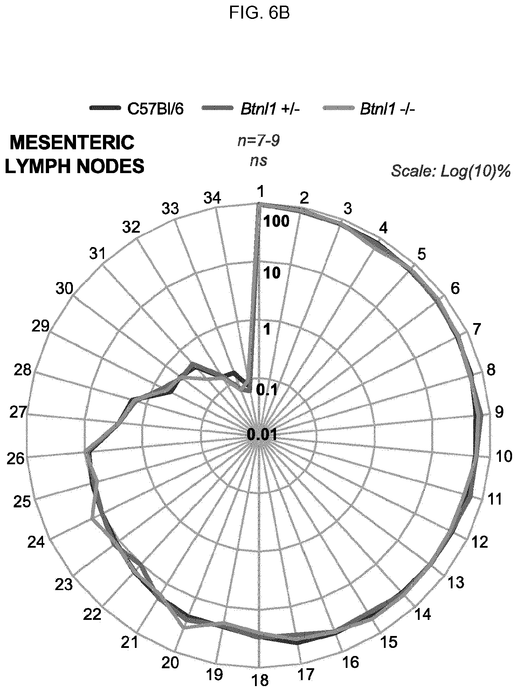

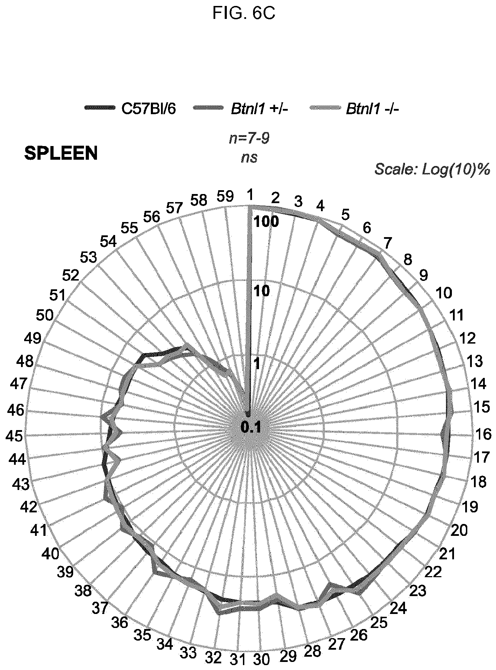

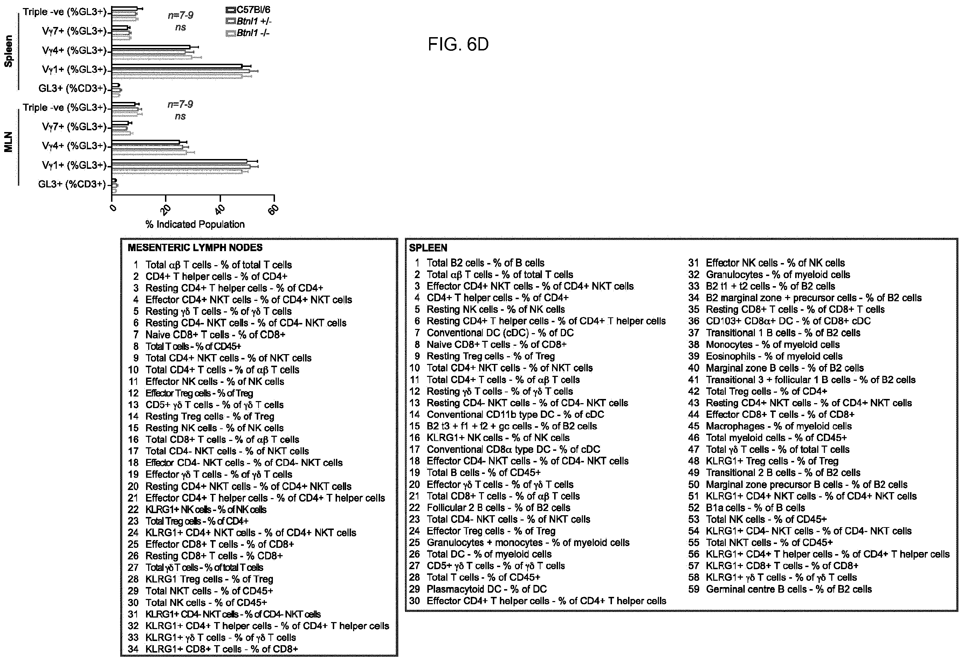

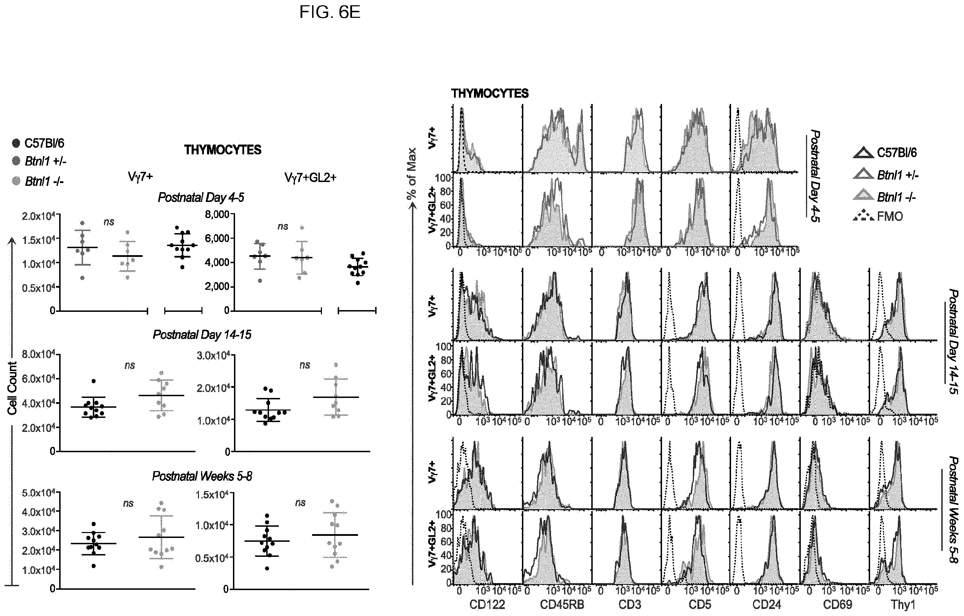

FIGS. 6A-6E are a series of graphs and tables demonstrating that Btnl1 has no detectable effect on the systemic T, B, and myeloid cell compartments. Vy7+ IELs were reduced in Btnl1indel/indel mice compared to WT mice (FIG. 6A). Mesenteric Lymph Node (MLN) and Splenic immune compartments of WT, Btnl1+/-, and Btnl1-/- were analysed by flow cytometry and were found to be comparable among all 3 genotypes (FIGS. 6B-6C). TCRVγ chain usage in MLN and splenic lymphocytes harvested from WT,Btnl1+/- and Btnl1-/- mice was assessed by flow cytometry and found to be comparable among the 3 genotypes. (FIG. 6D). Vy7+ and Vy7+GL2+ thymocytes from WT or Btnl1+/- and Btnl1-/- mice were assayed by flow cytometry to enumerate total cell counts (left) and cell surface phenotype (right) at three time points, which were similar between the three genotypes (FIG. 6E). All error bars represent mean ± SD.

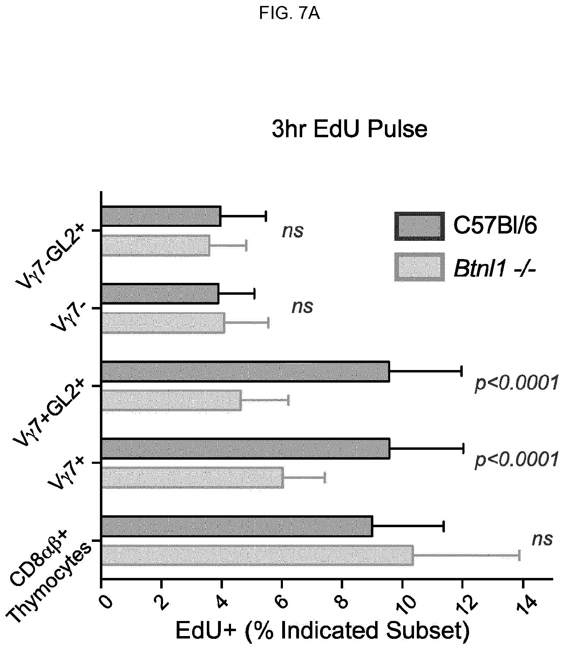

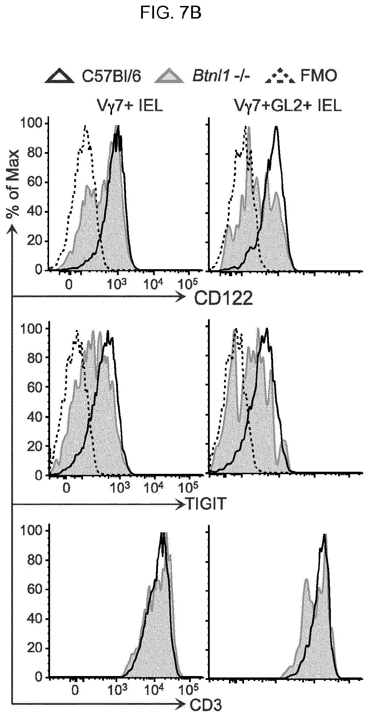

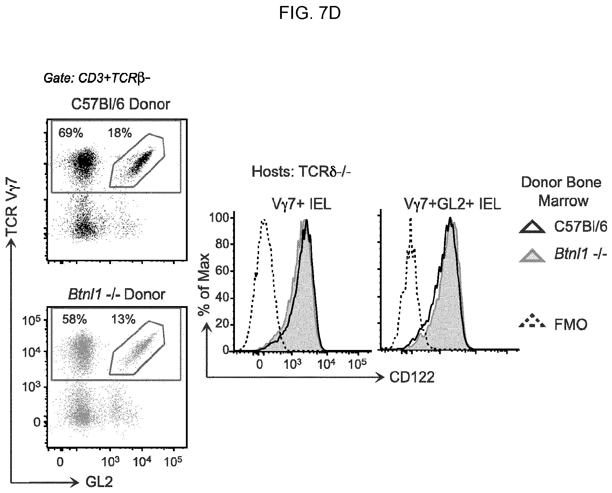

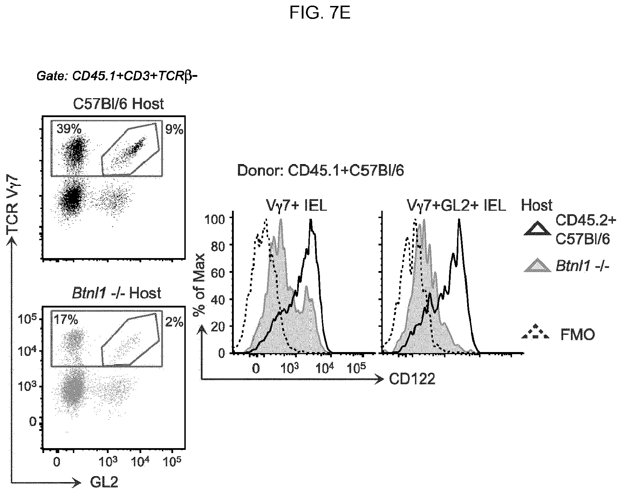

FIGS. 7A-7E are a series of graphs showing that Btnl1 drives selective expansion and maturation of gut IEL. Significantly fewer Vy7+ IELs incorporated EdU in week 5 Btnl1-/- mice compared to WT (FIG. 7A). Vy7+ and Vy7+GL2+ IELs in Btnl1-/- mice also retained an immature phenotype, having lower levels of CD122 and TIGIT, and higher levels of Thy1, than mature WT Vy7+ and Vy7+GL2+ IELs (FIGS. 7B- 7C). Bone marrow (BM) transfer into irradiated TCRδ-/- mice demonstrated that BM from either WT or Btnl1-/- mice was equally effective in IEL reconstruction (FIG. 7D). However, reconstitution of irradiated Btnl1-/- Vy7+ IELs was much less effective than reconstitution of irradiated CD45.2+C57BI/6 Vy7+ IELs with CD45.2+C57BI/6 BM was used (FIG. 7E). All error bars represent mean ± SD.

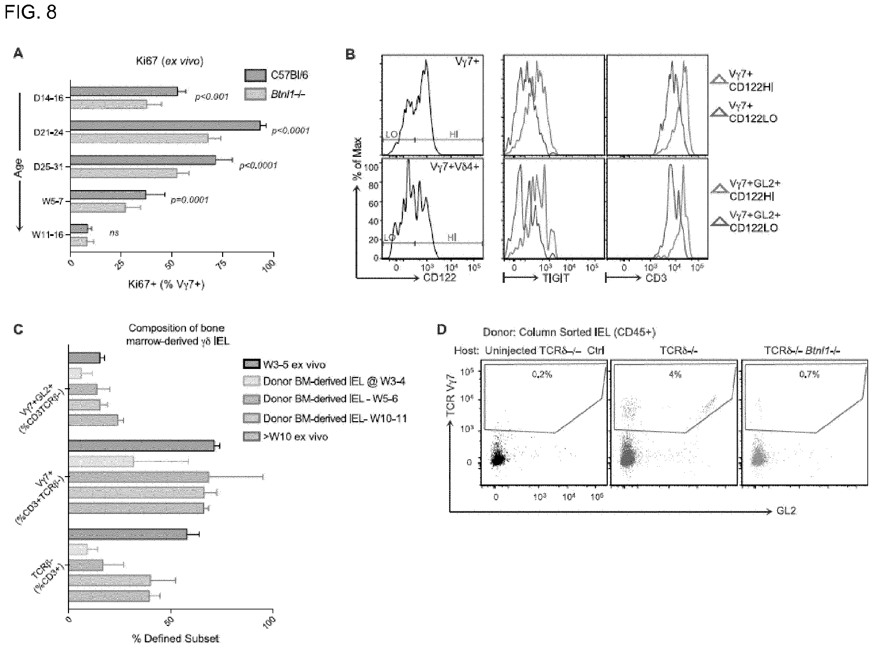

FIGS. 8A-8D are a series of graphs that show the impact of Btnl1 on intestinal engraftment, expansion, and retention of CD122^{HI} Vy7+ IELs. Significantly fewer Vy7+ IELs expressed Ki67 in Btnl1-/- mice than in WT mice, indicating reduced cell cycling (FIG. 8A). The btnl1-/- Vγ7+CD122^{HI} and Vγ7+GL2+CD122^{HI} IELs shown in FIG. 7B have a different cell surface phenotype from the Vγ7+CD122^{LO} and Vγ7+GL2+CD122^{LO} IELs displayed in FIG. 7B (FIG. 8B). Irradiated TCRδ KO mice reconstituted with WT BM were analysed for γδ IEL composition at the indicated time-points after BM transfer to establish a time course (FIG. 8C). IEL isolated from WT W4-5 mice were column-purified using CD45 microbeads and adoptively transferred intraenously into W6 TCRδ-/- or TCRδ-/-Btnl1-/- hosts. γδ T cell composition was assayed 2-3 weeks later by flow cytometry and showed that reconstitution was impaired in TCRδ-/-Btnl1-/- hosts (FIG. 8D). All error bars represent mean ± SD.

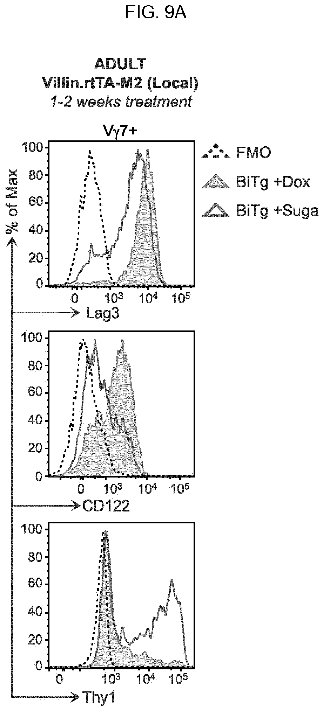

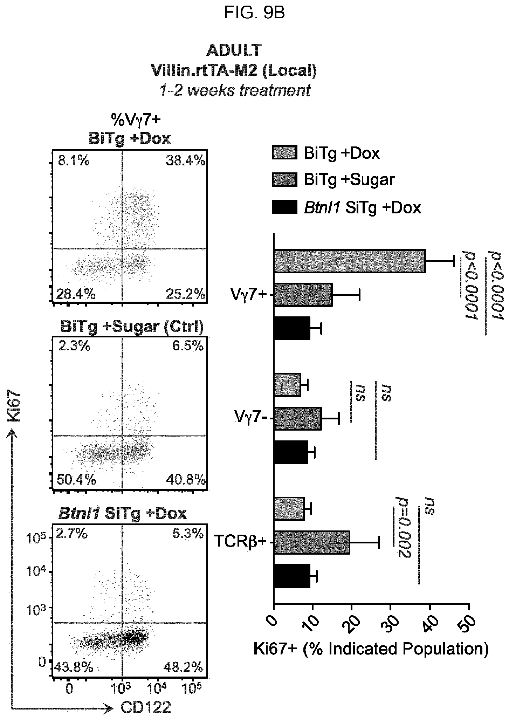

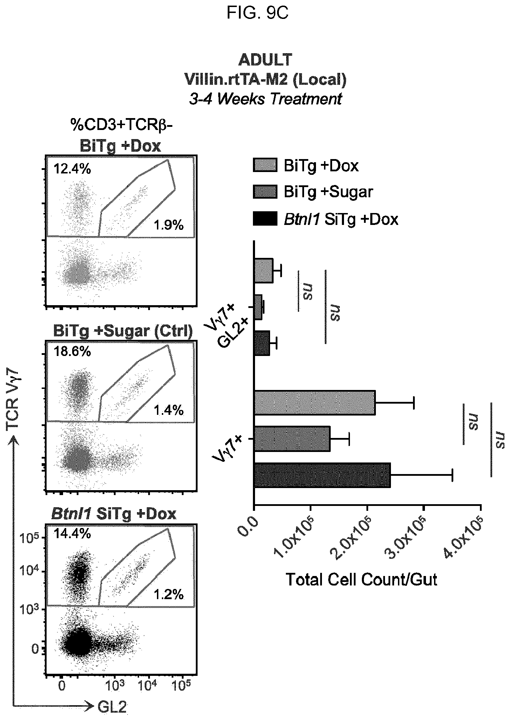

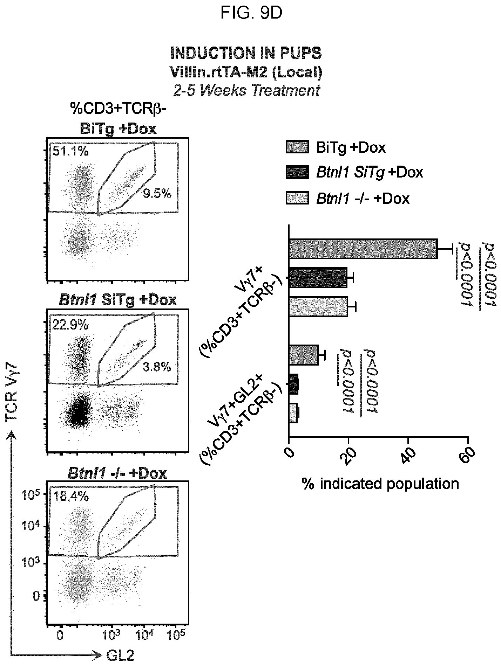

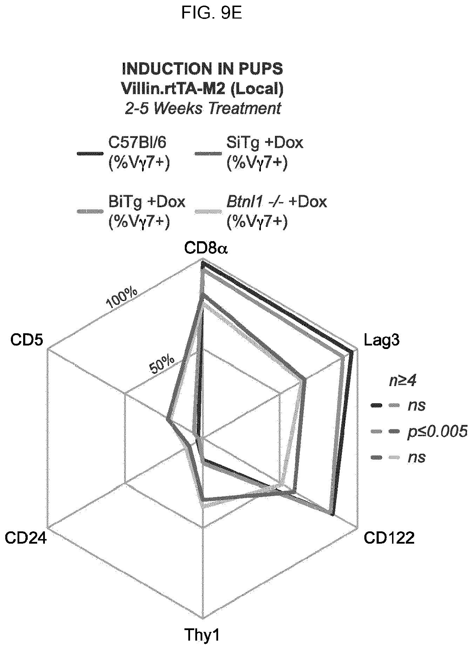

FIGS. 9A-9E are a series of graphs showing that villin-specific Btnl1 induction rescues Vy7+ IEL in vivo. Vy7+ IELs in bitransgenic (BiTg) adult mice expressing Dox-inducible Btnl1 and rtTA under the control of the vilin promoter showed a shift in the expression of surface markers toward a more mature Vy7+ phenotype when mice were administered Dox (1 mg/ml, 2% sucrose) (FIG. 9A). Dox-treated adult BiTg mice also showed an increase in Ki67, which was not observed in single transgenic (SiTg) Dox- treated mice that did not carry rtTA or mice that were not administered Dox (administered 2% sucrose) (FIG. 9B). Dox-treated adult BiTg mice did not show a change in Vy7+ cell numbers (FIG. 9C). Dox- treatment of BiTg pups increased the percentage of Vy7+ IELs (FIG. 9D) and altered the expression of cell surface markers such that Vy7+ cells from BiTg pups exposed to Dox phenocopied WT Vy7+ cells (FIG. 9E). All error bars represent mean ± SD.

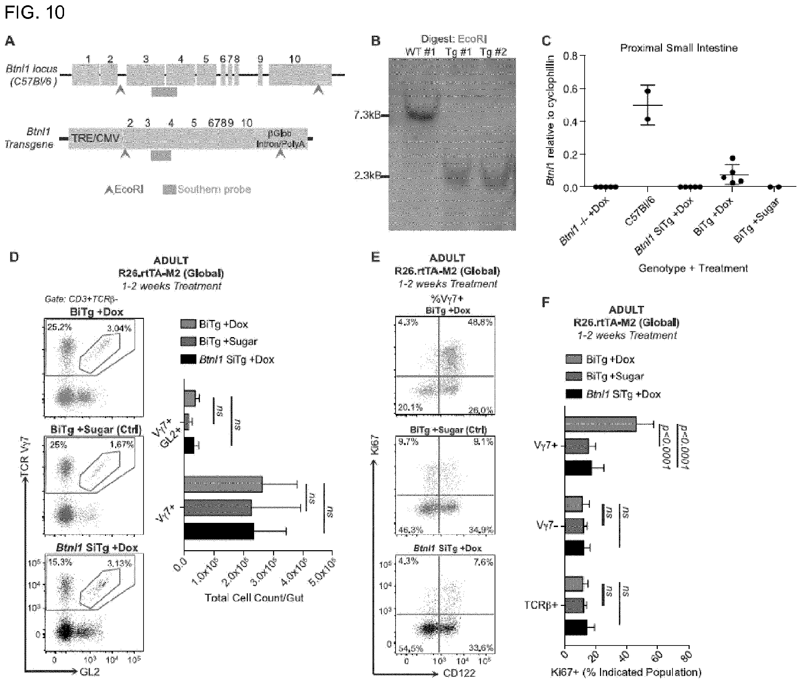

FIGS. 10A-10F are a series of schematics and graphs characterizing inducible Btnl1 transgene expression and its impact in adult mice. Schematic representation of the WT Btnl1 locus (top) and TRE- Btnl1 transgene construct (bottom). Light bars: untranslated region; darker bars: translated region; (TRE) upstream-tetracycline response element/CMV promoter and downstream-β-globulin/polyA (FIG. 10A). Southern blot to detect transgene insertion. Genomic DNA was digested with EcoR1 as indicated (arrowheads) and a probe (bar below schematic in FIG. 10A) targeting the indicated region (Exon3/4 boundary in ORF) was generated to detect the WT and targeted allele (FIG. 10B). W7-13 (adult) mice of indicated genotypes on a Btnl1-/- background were administered doxycycline water (1 mg/ml Dox, 2% sucrose) or ctrl water (2% sucrose) for 1-2 weeks. Gene expression assessed by qRTPCR in proximal small intestine of adult mice following the indicated treatment demonstrated that btnl1 expression was increased in BiTg mice treated with Dox (FIG. 10C). γδIEL composition (left) and absolute cell counts (right) were assessed by flow cytometry in adult mice following the indicated treatment and treatment was found not to affect Vy7+ cell proportions or cell numbers (FIG. 10D). Ki67 and cell surface CD122 expression were analysed in Vγ7+ IEL from adult mice following the indicated treatment and both were found to be increased in Dox-treated BiTg mice (FIG. 10E). Ki67 expression was significantly higher in Vy7+ IELs than in Vγ7- and TCRβ+ IELs from Dox-treated BiTg adult mice (FIG. 10F). All error bars represent mean ± SD.

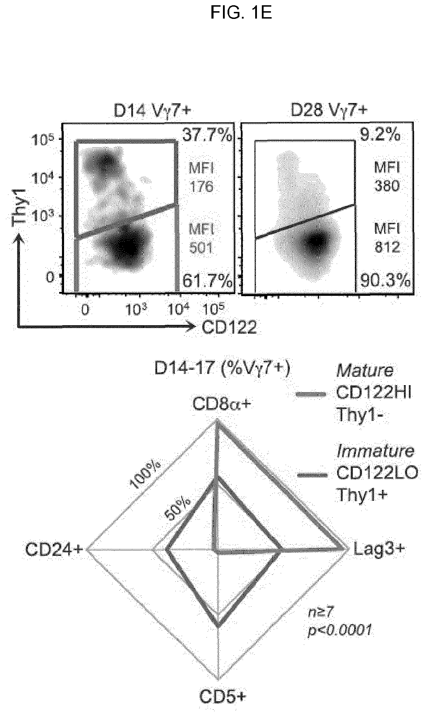

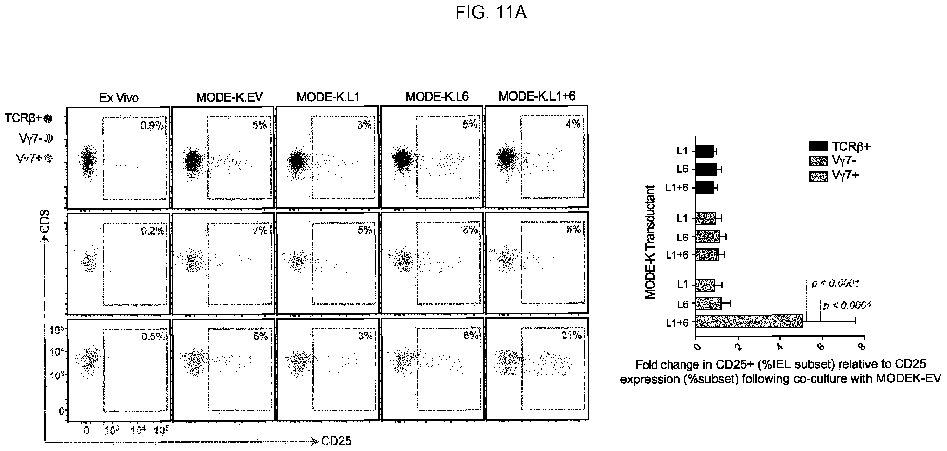

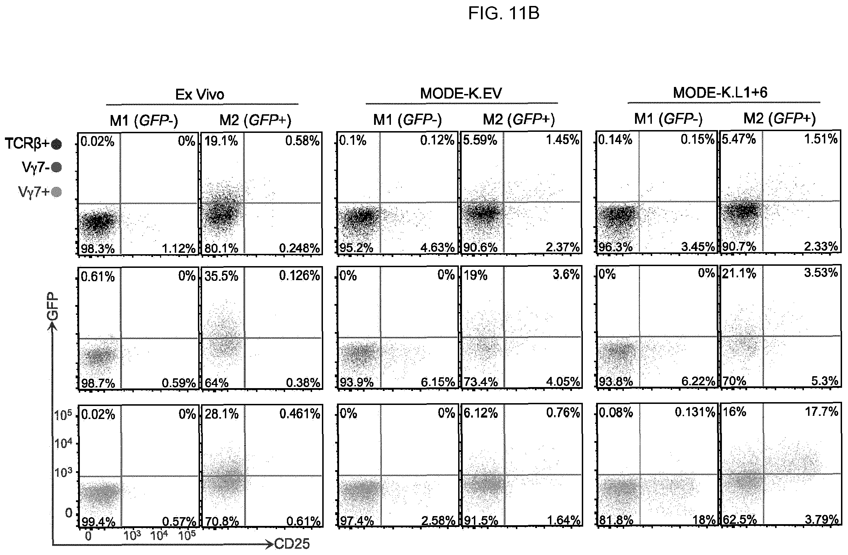

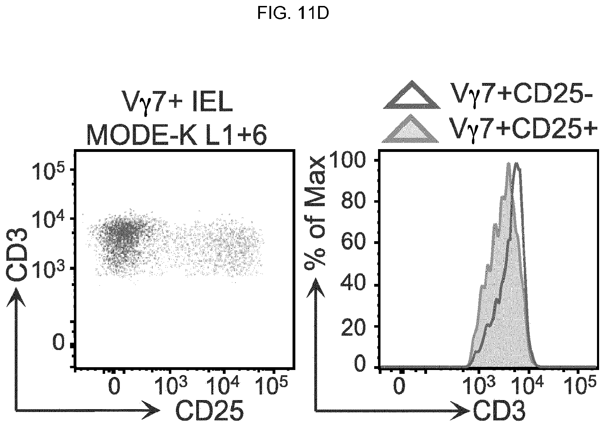

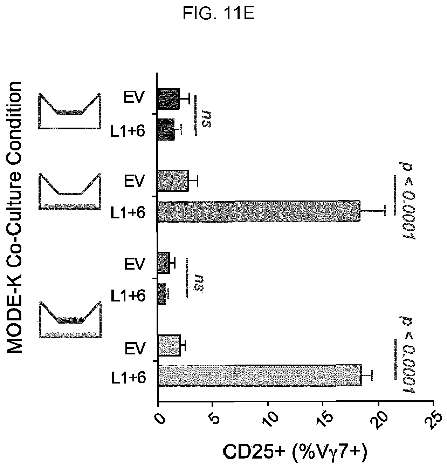

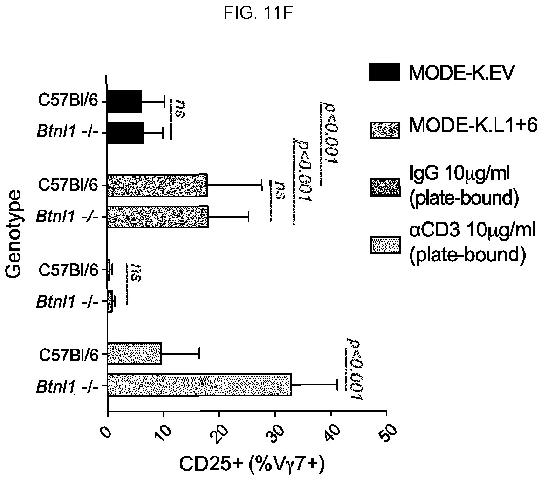

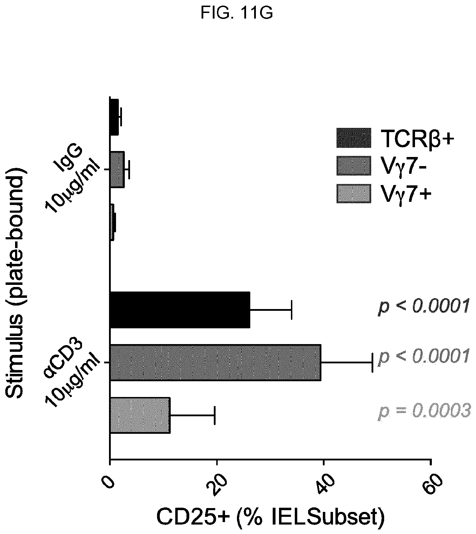

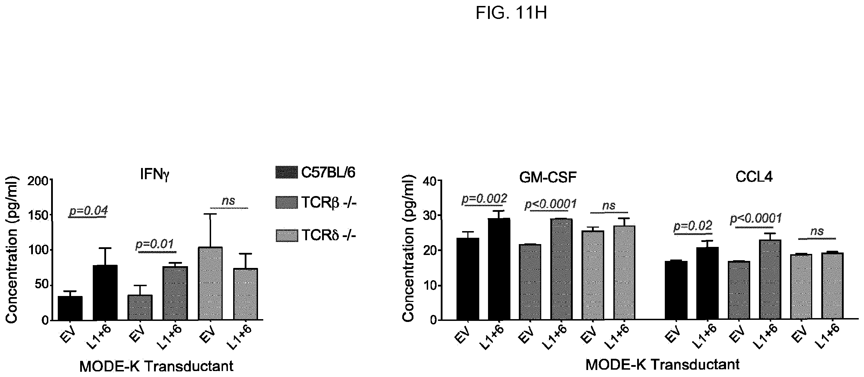

FIGS. 11A-11H are a series of graphs showing that gut Vy7+ IELs respond to Btnl proteins. IELs exposed to L1+6 MODE-K cells (cells transfected with Btnl1 and Btnl6) for 12 hrs showed upregulation of CD25, a readout for TCR stimulation, primarily in Vy7+ IELs (FIG. 11A). Co-culture of L1+6 cells for 12 hrs with IELs from Nurr77.gfp mice led to an increase in GFP in Vy7+ IELs, another indicator of TCR activation (FIG. 11B). CD25 activation also leads to CD122 downregulation, and a population of CD25+GFP+CD122-arose among Vy7+ IELs co-cultured with L1+6 MODE-K cells, but not among Vy7+ IELs co-cultured with MODE-K cells transfected with empty vector (EV) (FIG. 1 1C). Vγ7+CD25+ IELs co-cultured with L1+6 MODE-K cells also showed a slight downregulation of TCR (CD3) (FIG. 11D). Only IELs in contact with L1+6 cells showed in increase in CD25 (FIG. 11E). Vy7+ cells from Btnl1-/- mice could also respond to L1+6 by increasing CD25 surface expression (FIG. 11F), and IELs that had not been experienced Btnl1 selection in vivo (IELs from Btnl1-/- mice, and TCRαβ+ and Vy7+ IELs) responded to anti-CD3 by increasing surface CD25 expression (FIG. 11G). Increases in IEL effector cytokines IFNy, CCL4, and GM-CSF were observed in the supernatants of IEL co-cultures with L1+6 cells from WT and TCRβ-/- mice, but not TCRδ-/- mice (FIG. 11H). All error bars represent mean ± SD.

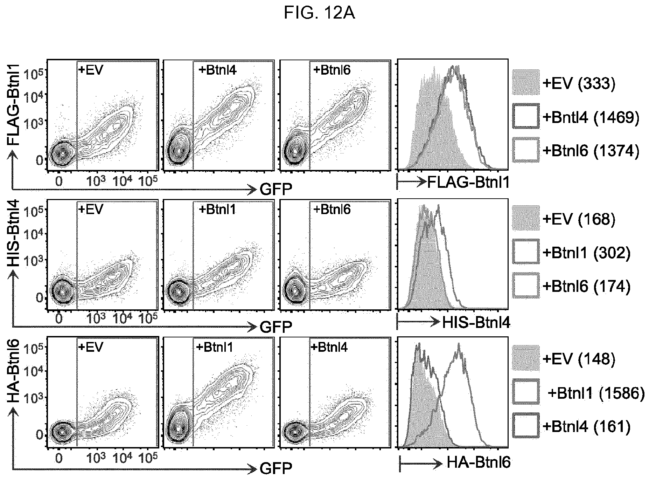

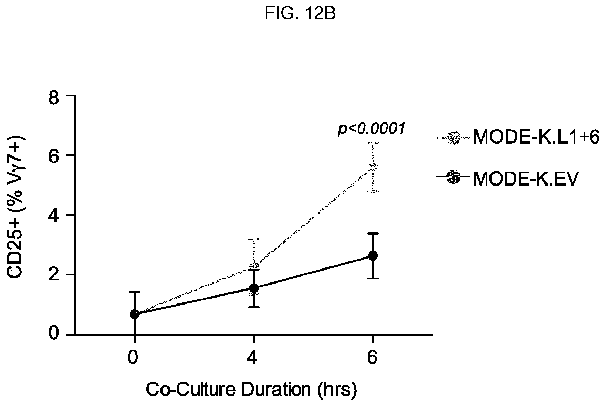

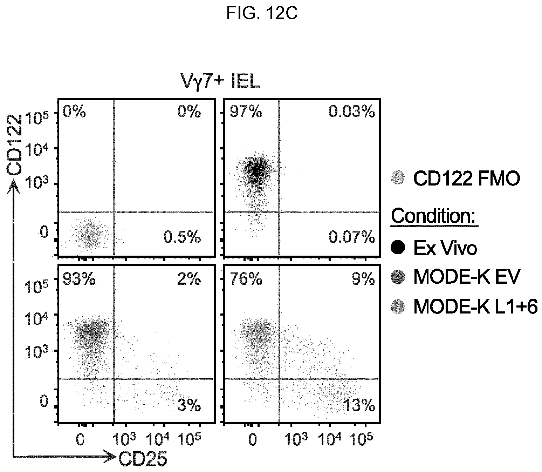

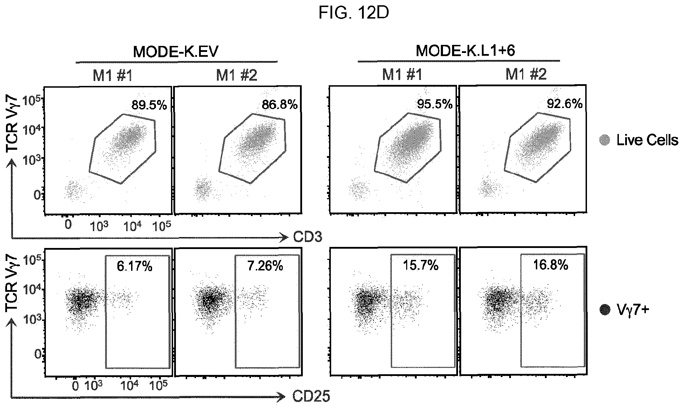

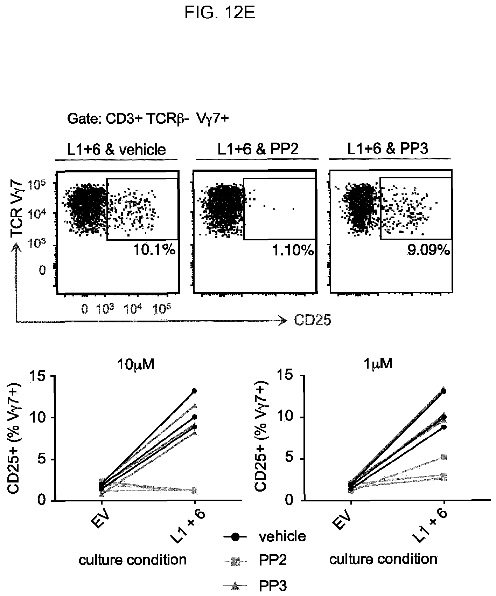

FIGS. 12A-12E are a series of graphs showing the impact of co-expression of Btnl1 and Btnl6 on Vy7+ IELs. Cell surface expression of FLAG-Btnl1, HIS-Btnl4 or HA-Btnl6 co-transfected in MODE-K cells. Histogram overlays show the expression of each BTNL after gating on GFP+ cells (numbers in brackets indicate geometric mean fluorescence intensity, gMFI), and demonstrate that co-expression of Btnl4 or Btnl6 with Btnl1 increases surface expression (FIG. 12A). Primary small intestinal IEL cultured for the indicated times with MODE-K cells transduced with constructs expressing an empty vector (EV) versus Btnl1+Btnl6 (L1+6) showed that Vγ7+ IELs increased CD25 surface expression when co-cultured with MODE-K cells expressing L1+6 (FIG. 12B). Representative plots of cell surface CD122 and CD25 expression on Vy7+ cells after the indicated overnight culture conditions demonstrated that co-culture with MODE-K cells leads to an increase in CD25 and a decrease in CD122 (FIG. 12C). Cell surface CD25 expression was increased in positively FACS-sorted Vy7+ IEL after overnight co-culture with MODE-K cells expressing L1+6 compared to EV (FIG. 12D). Cell surface CD25 expression in primary Vy7+ IEL after overnight co-culture with the indicated MODE-K transductants in the presence PP2, PP3 or vehicle shows that CD25 upregulation was inhibited by PP2 (FIG. 12E). All error bars represent mean ± SD.

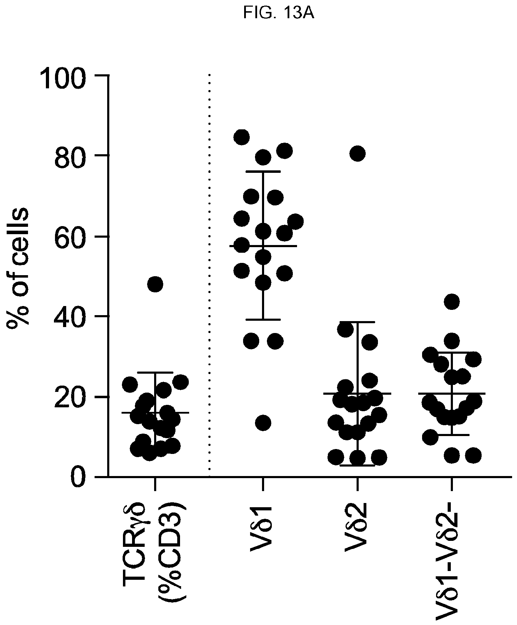

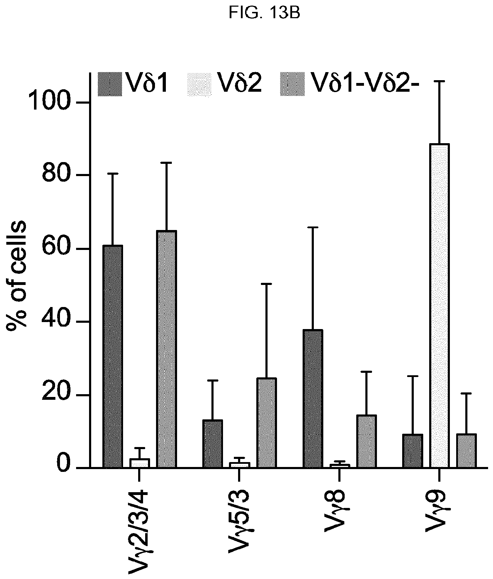

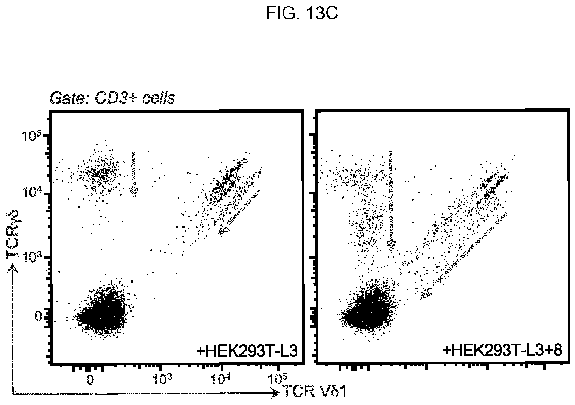

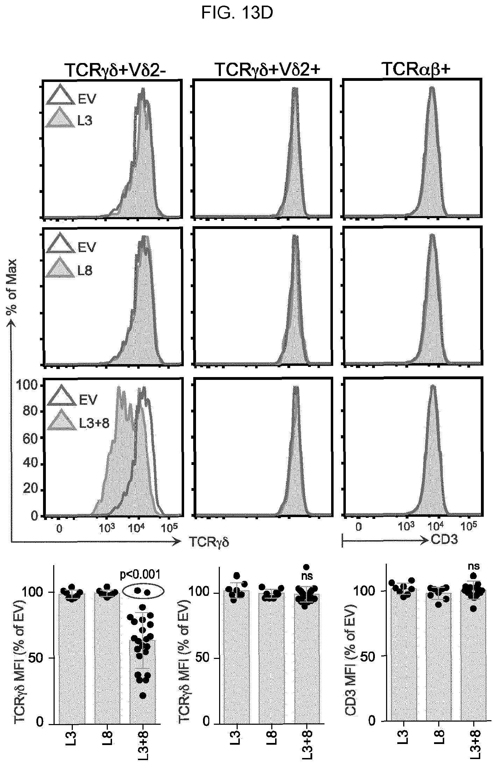

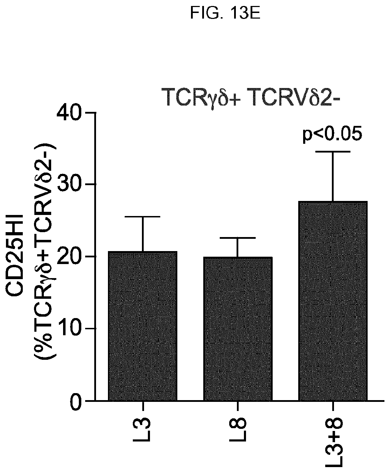

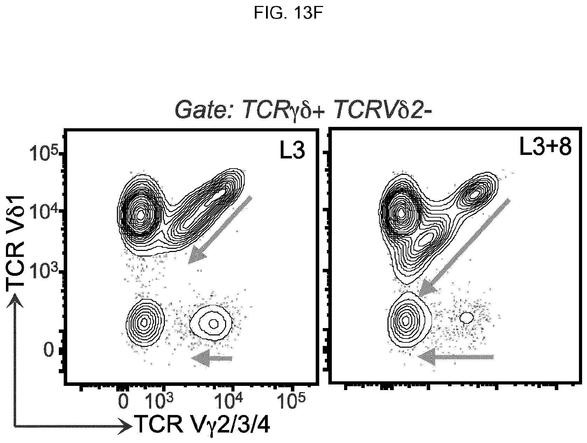

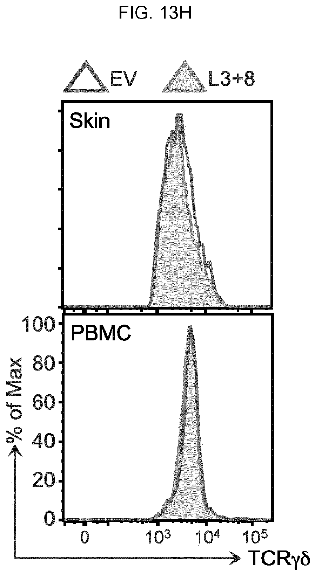

FIGS. 13A-13H are a series of graphs demonstrating the regulation of human gut Vy4+ cells by BTNL3 and BTNL8. Human γδ cells isolated from ascending colon were enriched in Vδ1+ cells, but Vδ2+ and Vδ1-Vδ2- cells were also present (FIG. 13A). Human gut γδ T cells with particular Vδ profiles reacted with different Vγ antibodies, indicating the presence of subsets of cells (FIG. 13B). Surface TCRγδ/Vδ1 expression on human gut lymphocytes after was reduced after 12-hr co-culture with BTNL3 plus BTNL8 (L3+8)-transduced HEK293T cells compared to BTNL3 (L3)-transduced HEK293T cells. Arrows denote shifts in TCR staining (FIG. 13C). TCR downregulation was only observed in Vδ2- cells, and only in response to co-culture with L3+8 cells (FIG. 13D). L3+8 cells also induced CD25 upregulation (FIG. 13E). Vδ2- cells that showed TCR downregulation after co-culture with L3+8 cells were detected with antibodies to Vy2/3/4, but not with antibodies to Vγ8, Vy5/3, or Vγ9 (FIGS. 13F-13G). L3+8 did not induce TCR downregulation in γδ T cells from skin or peripheral blood mononuclear cells (PBMC) (FIG. 13H). All error bars represent mean ± SD.

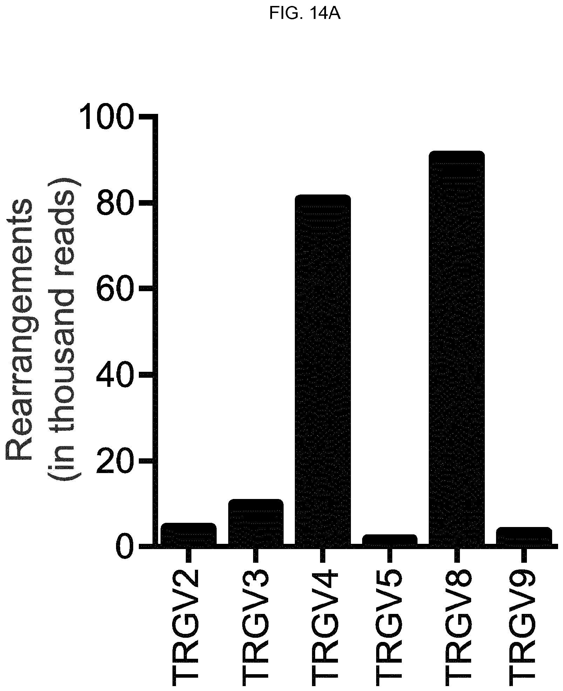

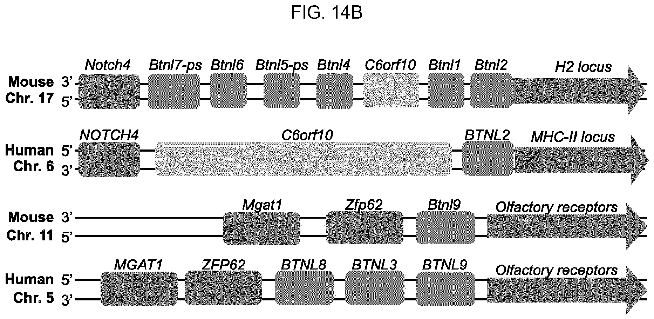

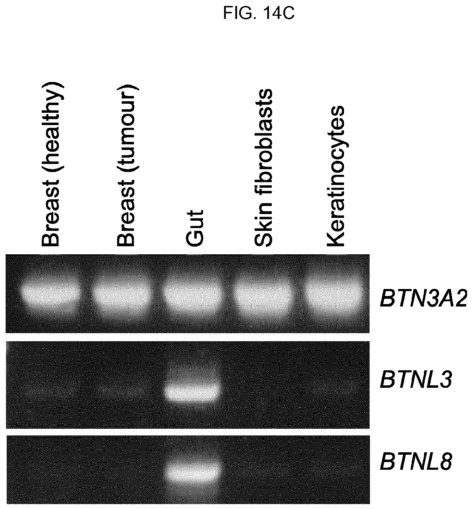

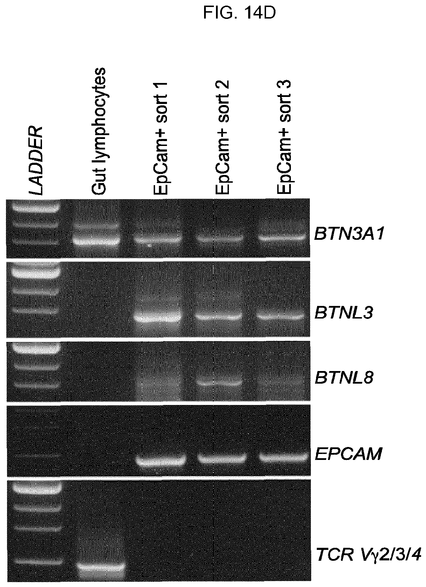

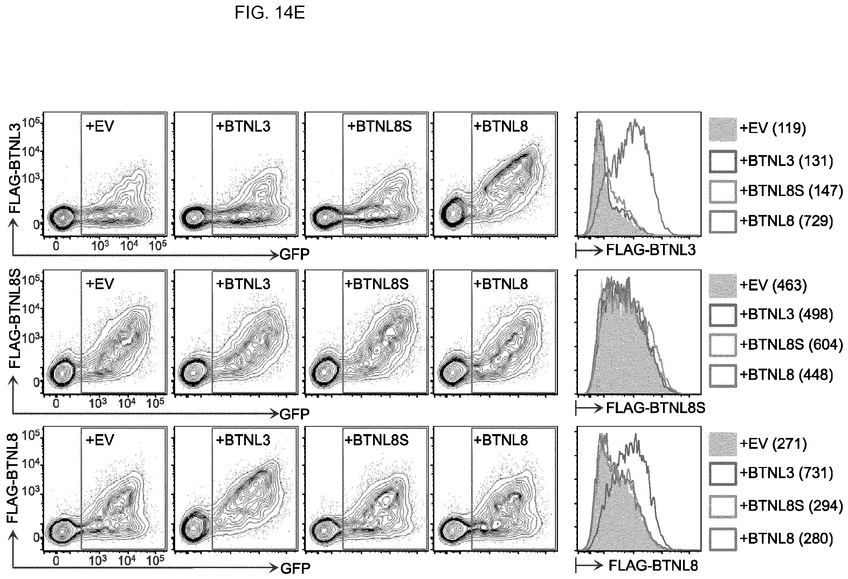

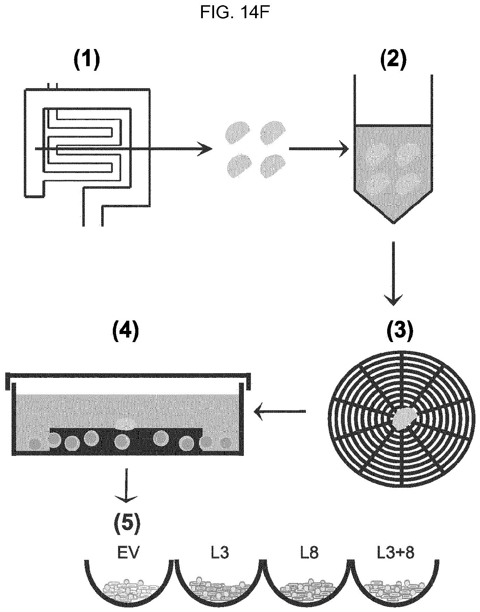

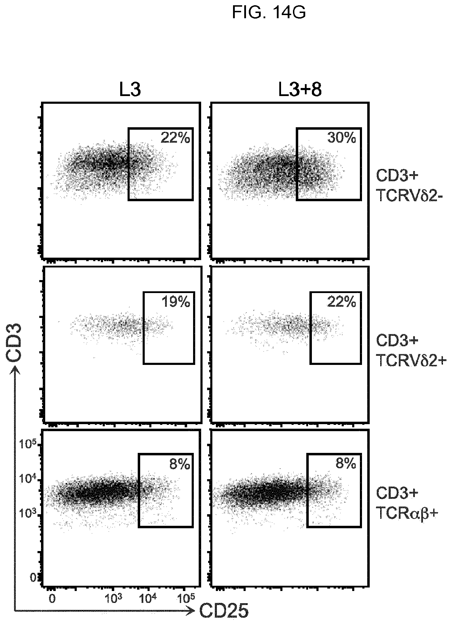

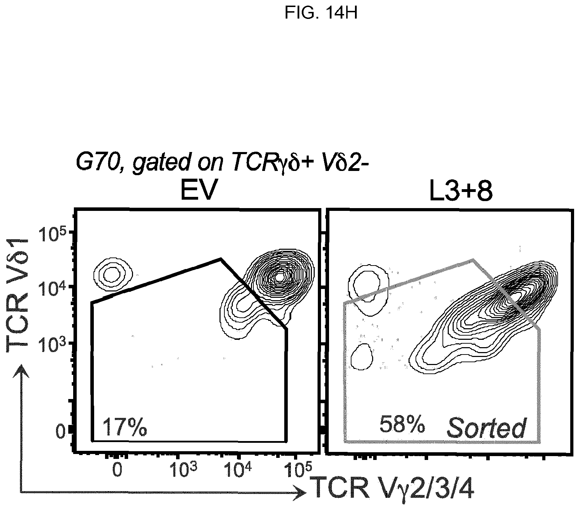

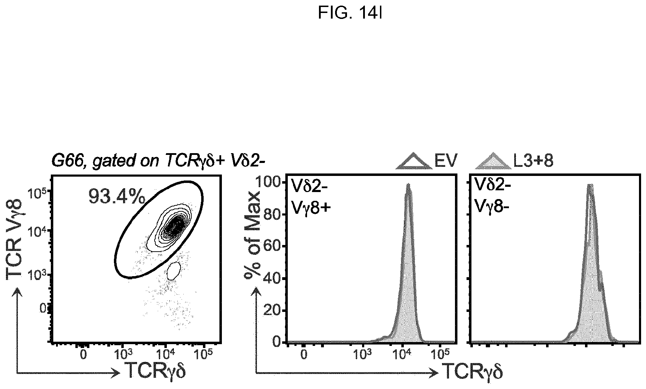

FIGS. 14A-14I are a series of graphs, gels and schematics characterizing human intestinal γδ cells and the selective impact of BTNL3 and BTNL8 co-expression on γδ cells. FACS-sorted γδ T cells harvested from human intestinal tissue were analysed by deep sequencing for TCR Vγ chain usage and found to primarily express Vγ4 and Vγ8 (FIG. 14A). Schematic illustrating the murine and human Btnl2/BTNL2 and Btnl9/BTNL9 loci, adapted from the NCBI gene viewer (FIG. 14B). (C) Conventional RT-PCR analysis of BTN3A2, BTNL3 and BTNL8 expression in the indicated tissues showed that BTNL3 and BTNL8 were specifically expressed in the gut (FIG. 14C). Conventional RT-PCR analysis of BTN3A1, BTNL3, BTNL8, EPCAM and TCR Vγ2/3/4 expression in the indicated samples showed that BTNL3 and BTNL8 were expressed in EpCAM+ epithelial cells (FIG. 14D). Cell surface expression of FLAG-BTNL3, FLAG-BTNL8S or FLAG-BTNL8 co-transfected in HEK293 cells with the indicated constructs. Histogram overlays show the expression of each BTNL after gating on GFP+ cells (numbers in brackets indicate geometric mean fluorescence intensity, gMFI), and demonstrate that co-expression of BTNL3 and BTNL8 increases the expression of both proteins (FIG. 14E). Schematic illustrating the method of human intestinal tissue-resident lymphocytes isolation and co-culture with HEK293 transductants. (1) Endoscopic biopsies recovered from ascending colon of healthy donors. (2) Washed in complete media supplemented with antibiotic. (3) One biopsy applied to each matrix. (4) Culture for 5-7 days in complete medium supplemented with antibiotics, IL-2 and IL-15. (5) Co-culture with HEK293 cell lines transduced with EV, L3, L8 or L3+8 (FIG. 14F). Cell surface CD25 expression was increased in subsets of human gut-derived lymphocytes after co-culture with L3+8-transduced HEK293 cells compared to EV-transduced cells (FIG. 14G). Gating parameters for sorting of Btnl3+8-responsive human gut-derived lymphocytes. A greater amount of Vγ4 expression was observed when L3+8 responsive cells were sorted (FIG. 14H). TCRVγ chain usage (left) and cell surface TCRγδ expression (right) in gut-derived γδ T cells (isolated from a donor unresponsive to BTNL3+8) after co-culture with EV versus L3+8-transduced HEK293 cells indicated that the unresponsive donor had a γδ T cell population dominated by Vy8+ cells (FIG. 14I).

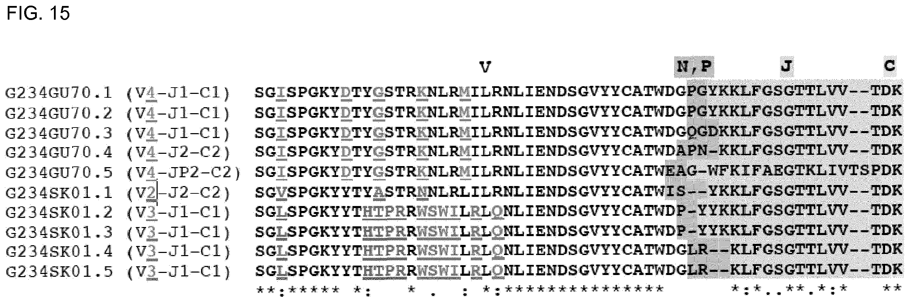

FIG. 15 is a sequence alignment showing the sequence of TCRγ chains of intestine and skin cells. The top four sequences are from L3+8-responsive gut lymphocytes with downregulated TCRs that were flow cytometry sorted from one donor. These cells expressed Vγ4. The bottom four sequences are TCRγ transcripts from skin-derived TCRγδ+ cells (G234SK01), which were biased toward Vγ3.

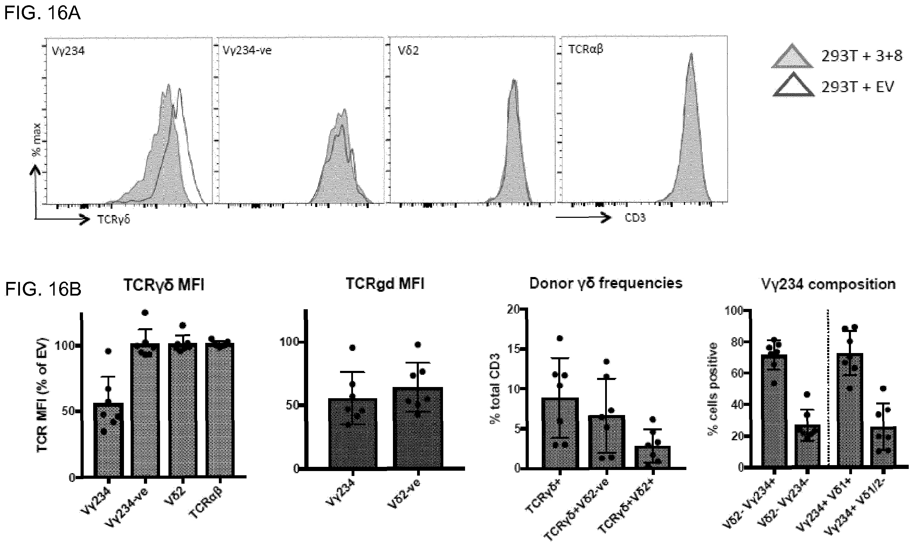

FIG. 16A is a series of flow cytometry histograms showing responses of human gut γδ cells following overnight co-cultures with 293 cells transduced with empty vector (EV) or vector expressing BTNL3 and BTNL8 (red histograms). FIG. 16B is a series of graphs. The first panel on the left shows quantitation of TCRγδ expression, measured as in FIG. 16A, for seven donors. The second panel from the left shows TCRγδ expression in TCRVγ2/3/4 cells, compared to all TCRV9- cells. The third panel from the left shows γδ cell frequency as a percentage of CD3 cells. The fourth (right-most) panel shows percentage of each cell type that express TCRVy2/3.

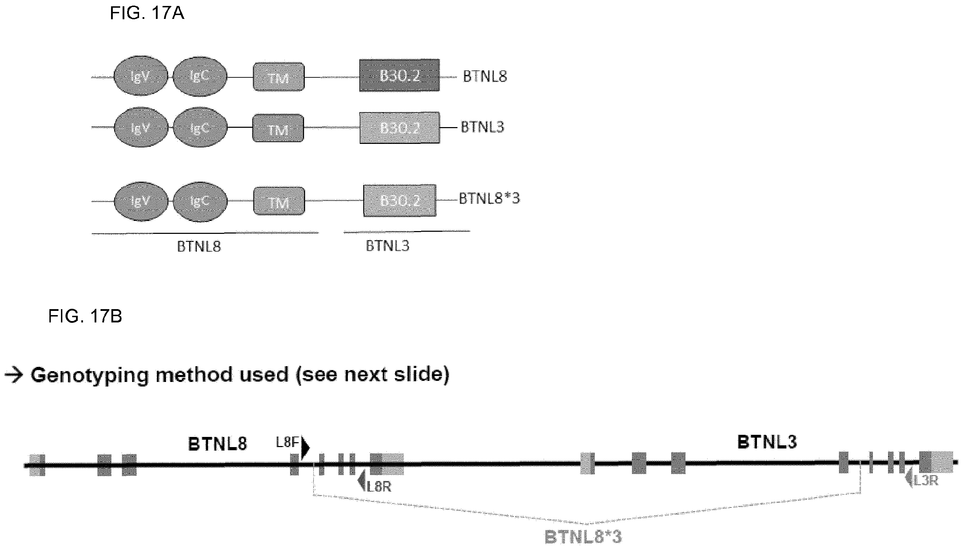

FIG. 17A shows schematic protein-coding structures of human BTNL3, BTNL8, and BTNL8*3, which is a fusion protein encoded by the haplotype in which BTNL8 ectodomains and the transmembrane (TM) domain are fused to the intracellular domains of BTNL3, as indicated. (FIG. 17A). FIG. 17B shows the PCR strategy used to detect the common haplotype encoding BTNL8 and BTNL3 from the rarer haplotype encoding BTNL8*3. There is a common forward primer (black) and genotype-specific reverse primers (blue, red). The product of each PCR reaction is approximately 1.3kb.

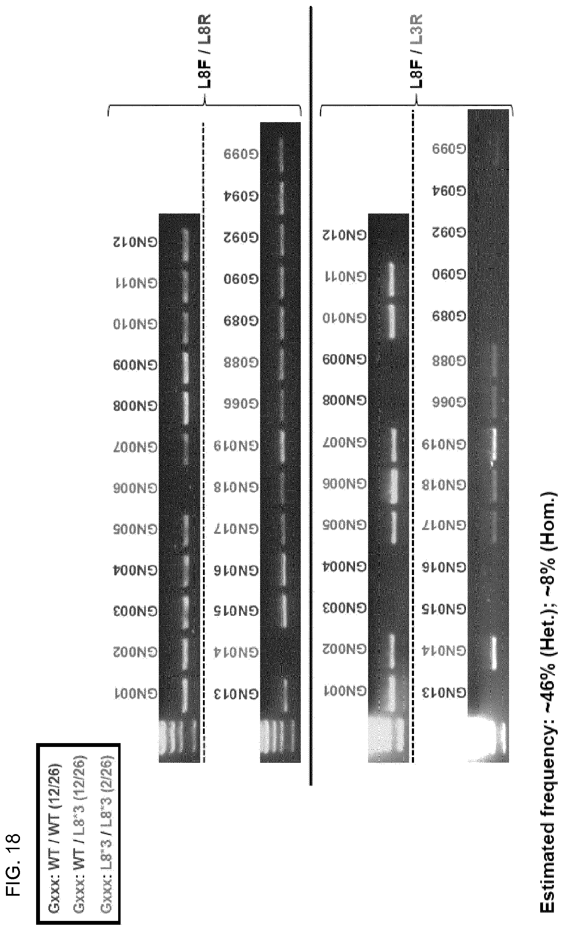

FIG. 18 is a series of images showing BTNL8*3 genotyping results. Gel electrophoresed PCR products of individual donors designated above each lane, aligned with size markers that in ascending order, are approximately 1 kb, 1.5 kb, 2 kb, and 3 kb. Donor designations are color-coded according to the genotype that the PCR analysis identifies, summarized in the top left box. Top left: red = homozygous for BTNL8*3; purple = heterozygous for BTNL8*3 and for an allele encoding BTNL8 and BTNL3 [designated WT]; blue = two alleles, each encoding BTNL8 and BTNL3.

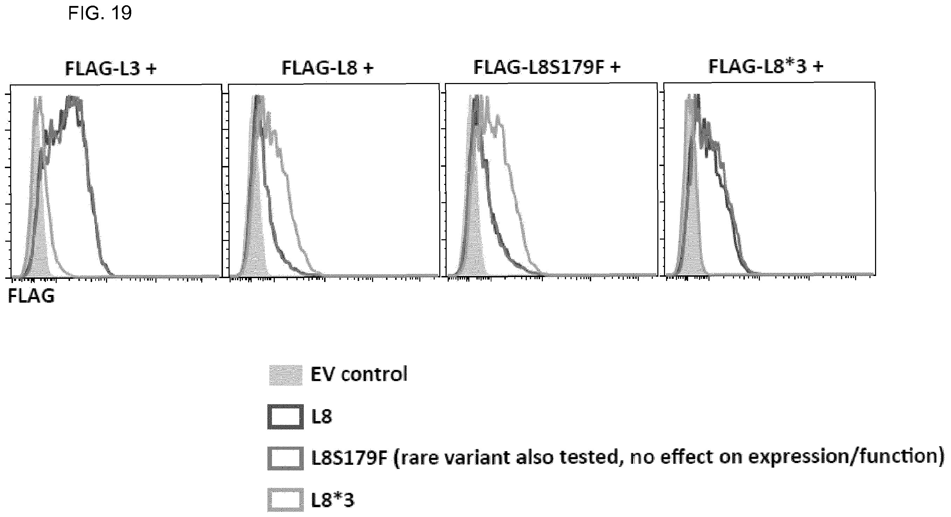

FIG. 19 is a series of graphs showing a cell biological analysis of BTNL proteins. Flow cytometry of surface protein expression by 293 cells of FLAG-tagged proteins (designated above each plot) that were introduced into 293 cells by gene transfer, together with the un-tagged constructs listed below the panels, color-coded to match the respective histograms.

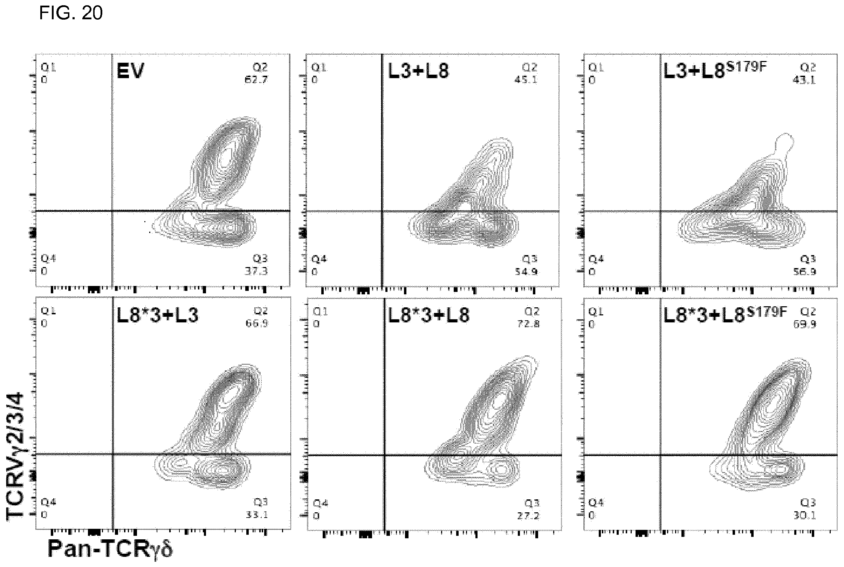

FIG. 20 is a series of flow cytometry plots showing a functional analysis of BTNL proteins. Flow cytometry data for TCRγδ expression (x-axis) and TCRVγ2/3/4 expression (y-axis) following co-culture with 293 cells into which empty vector (EV) had been introduced, or the BTNL genes denoted in each panel.

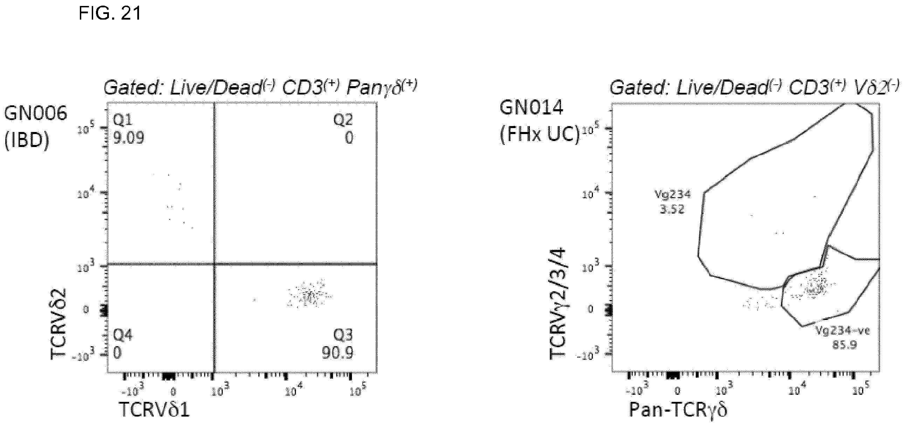

FIG. 21 is a set of flow cytometry plots showing gut γδ T cell compartments in to BTNL8*3 homozygotes. The left panel shows a γδ T cell population of patient GN006, who was diagnosed with IBD, and the right panel shows a γδ T cell population of patient GN014, who has a family history of ulcerative colitis.

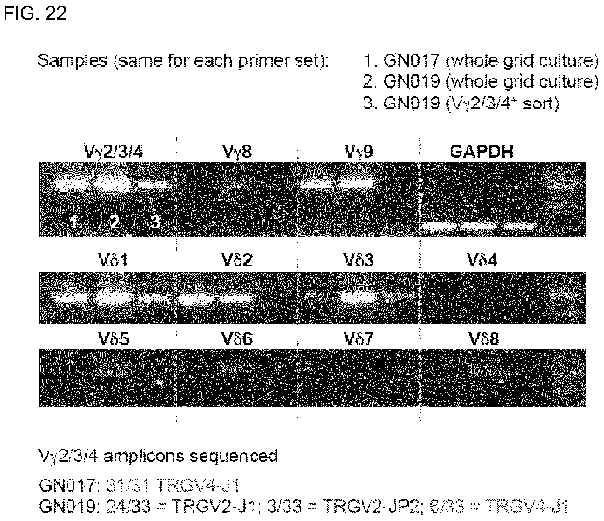

FIG. 22 is a series of immunoblots showing indicated TCR expression in BTNL8*3 heterozygotes. Band 1 (left) is derived from whole grid-cultured cells from patient GN017, band 2 (center) is derived from whole grid-cultured cells from patient GN019, and band 3 (right) is derived from Vy2/3/4+ sorted cells from patient GN019.

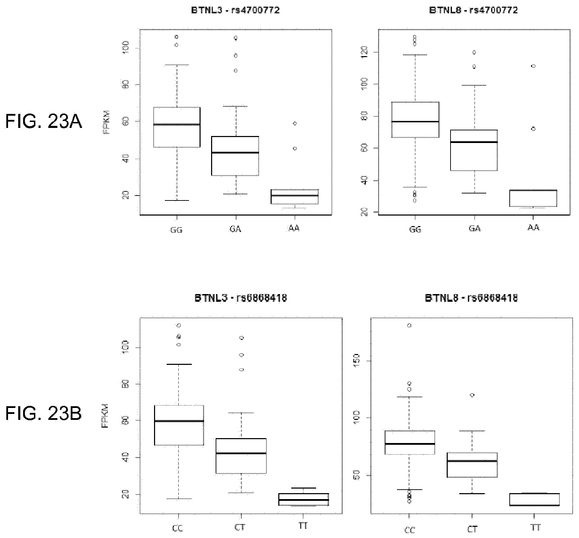

FIGS. 23A-23B are a series of graphs showing the effect of SNPs on BTNL3 and BTNL8 cell surface levels. FIG. 23A is a set of whisker plots showing BTNL3 (left) and BTNL8 (right) expression in subject having the rs4700772 haplotype as a homozygote (AA; n=9), or as a heterozygote (GA; n=36),compared to the more common allele (GG; n=75). FIG. 23B is a set of whisker plots showing BTNL3 (left) and BTNL8 (right) expression in subjects with the rs6868418 haplotype as a homozygote (TT; n=6), or as a heterozygote (CT; n=30), compared to the more common allele (CC; n=69). Expression levels were derived from normalized RNASeq data from individual colonic biopsy samples.

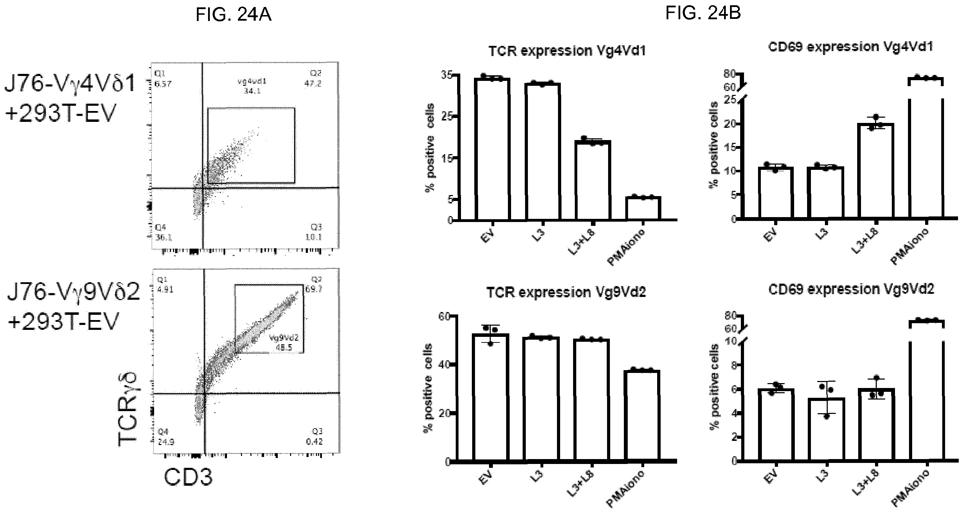

FIGS. 24A-24B are a series of graphs showing BTNL responsiveness conferred by the γδ TCR. The lefthand panels show flow cytometry plots of J76 human T cells expressing transduced recombinant TCRs of Vγ4Vδ1 (top) or Vγ9Vδ2 (bottom), as assessed by antibody-mediated detection of CD3 (x-axis) or TCRγδ (y-axis). The right-hand panels show quantitation of triplicate measures of TCR expression and of CD69 expression for cells shown in the left hand panel, following overnight exposure to the conditions indicated: 293 cells transduced with empty vector; 293 cells transduced with BTNL3; 293 cells transduced with BTNL3 and BTNL8. PMAiono = PMA/ionomycin.

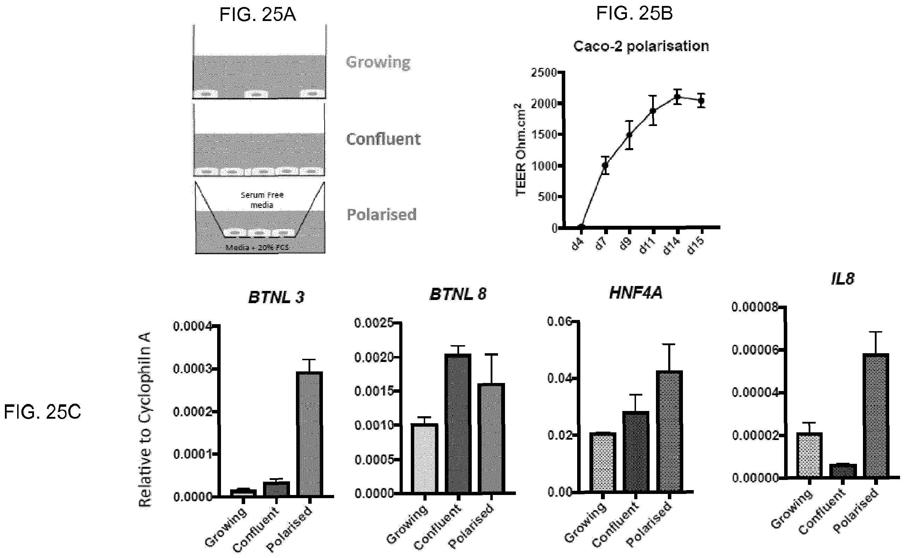

FIG. 25A is a schematic showing various growth conditions of human epithelial cells (Caco2), as used in the present study. FIG. 25B is a graph showing trans-epithelial electrical resistance (TEER) measurements, wherein the x-axis denotes days of culture. FIG. 25C is a series of graphs showing quantitative RT-PCR of expression of the denoted genes by Caco2 cells grown under the conditions shown in FIG. 25A.

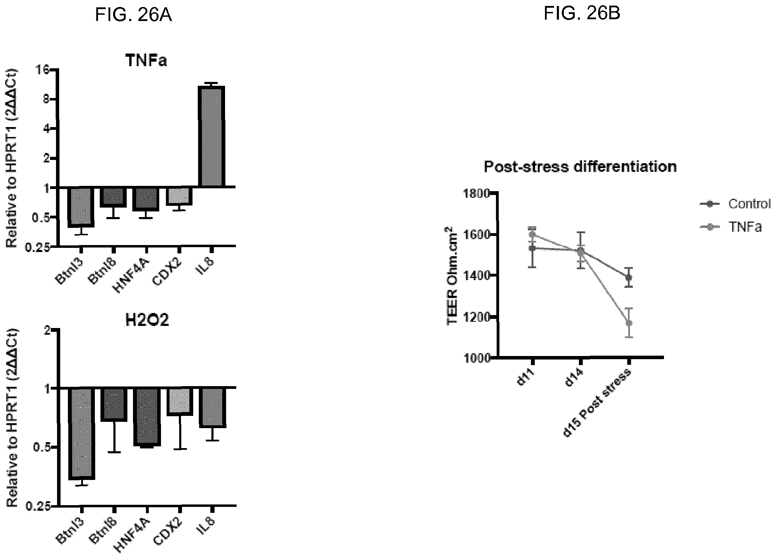

FIGS. 26A-26B are a set of graphs showing the effect of stress on BTNL3 and BTNL8, HNF4A, CDX2, and IL-8 expression.

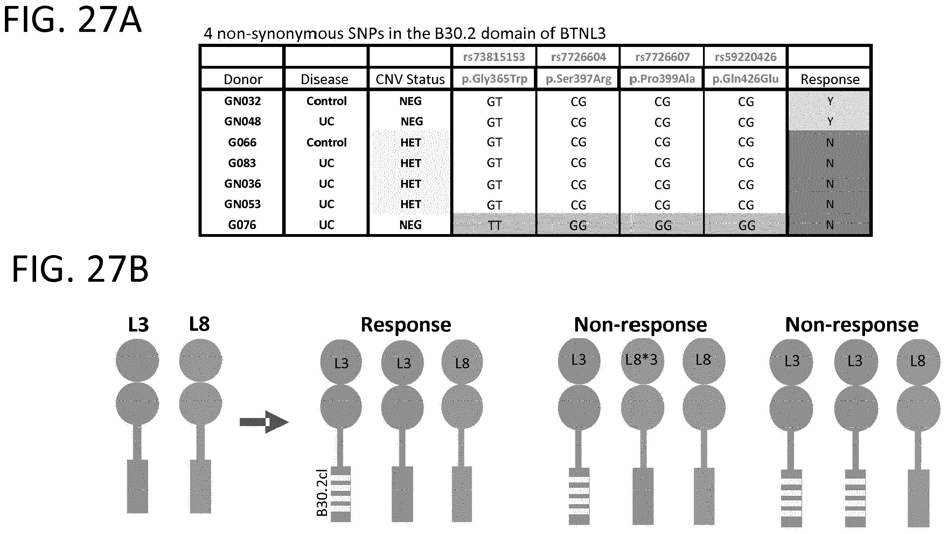

FIGS. 27A-27B show additional BTNL polymorphisms that are likely to affect function. FIG. 27A is a panel of non-synonymous SNPs in the B30.2 domain of BTNL3 (L3B30.2cl). FIG. 27B. illustrates the association of the BTNL3*8 and L3B30.2cl genotypes with responsiveness. Copy number variant (CNV) status: NEG = negative L8*3 allele. HET = heterozygous for L8*3. UC=ulcerative colitis. "rs" number indicates single nucleotide polymorphism. Response defined as >25% TCR downregulation compared with EV after co-culture with full-length L3+L8.

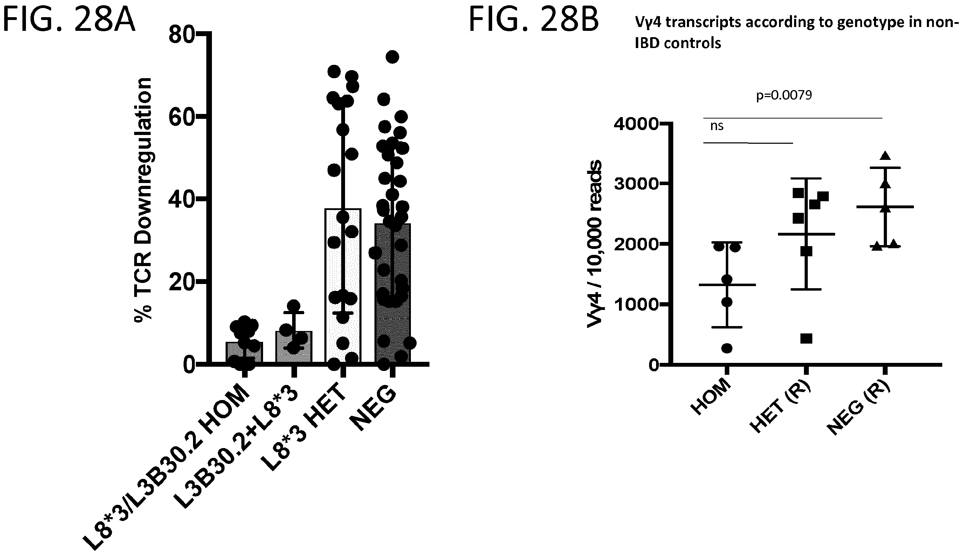

FIGS. 28A-28B demonstrate that the carriage of two or more BTNL polymorphisms is associated with the disruption of the Vγ4-BTNL axis and associated with a non-response endophenotype. FIG. 28A: % TCR downregulation compared to EV after co-culture with HEK 293T expressing L3+L8 of donors according to donor genotype. HOM (donors homozygous for L8*3/L3B30.2cl) (n=10), L3B30.2cl+L8*3 (Compound heterozygotes) (n=4), L8*3 HET (n=21), NEG (n=31). FIG. 28B: TCR Vg chain usage analysed by TCR sequencing of whole colonic mRNA. Vg4 displayed as number of reads per 10,000 of total Vg-chain reads and displayed according to genotype. HOM = donors with genotype homozygous for L8*3. HET (R) = Donors homozygous for L8*3 who make expected TCR down-regulation responses (>25%) in response to L3+L8 in an in vitro BTNL response assay. NEG (R) = Donors negative for the L8*3 allele who make expected TCR down-regulation responses (>25%) in response to L3+L8 in an in vitro BTNL response assay. (Analysed by one-way ANOVA).

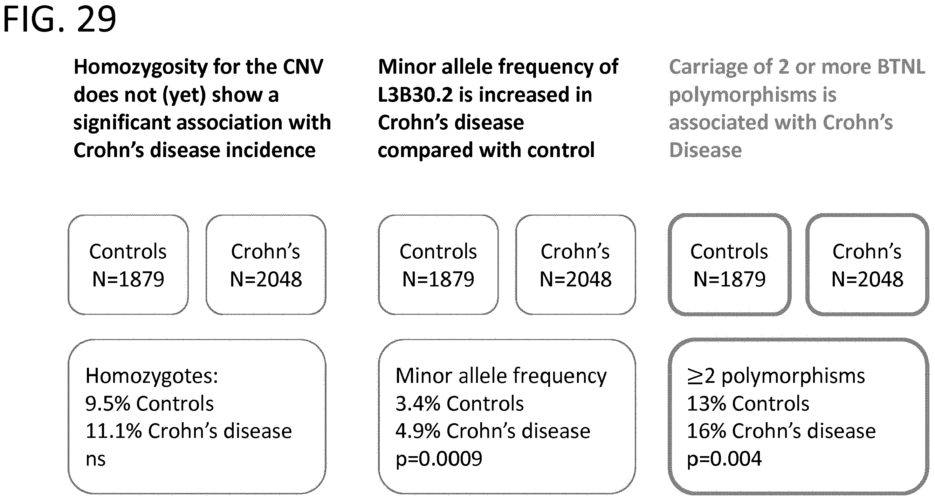

FIG. 29 shows the association of BTNL polymorphisms in a case control study genotyped by TaqMan SNP assay for the relevant polymorphism rs72494581 which is a proxy for the L8*3 CNV and rs59220426 which is one of the 4 missense polymorphisms in the L3B30.2 domain. These are analysed by Chisquared test. Genotypes are combined in the last column demonstrating that carriage of ≥2 polymorphism is greater in the disease group. Carriage of ≥2 identified BTNL polymorphisms yielded an Odds Ratio of disease 1.38 1.1-1.6 p<0.004.

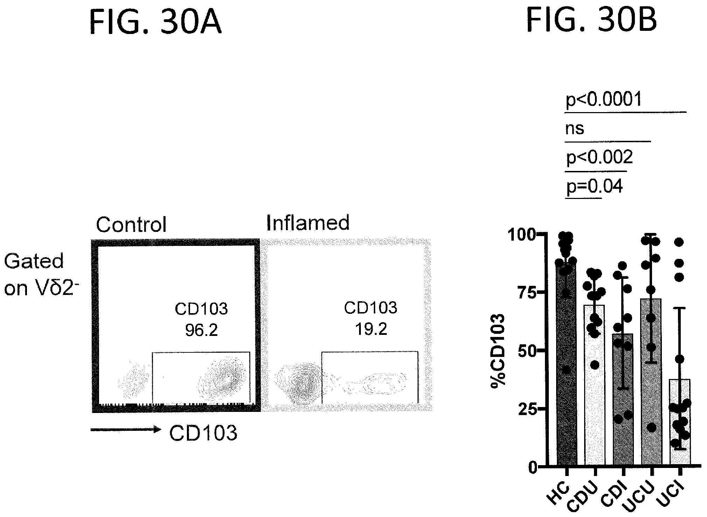

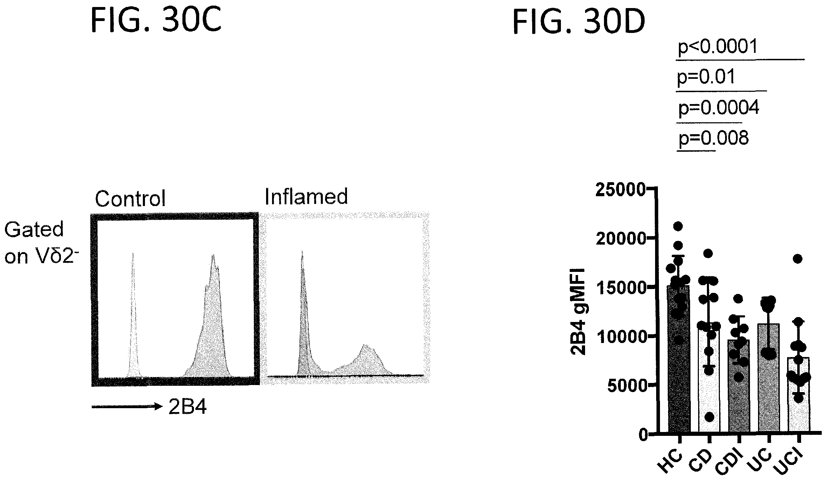

FIGS. 30A-30D shows the typical colonic γδ surface phenotype is dysregulated in IBD. FIG.30A: Representative flow cytometry contour plots demonstrating CD103 expression of TCRγδ+Vδ2- cells obtained from the colonic mucosa of a control donor and donor with active UC (UCI) after isolation by short term explant culture (Isotype control in grey). FIG. 30B: Summary data of expression levels of TCRγδ+Vδ2- cells according to disease subtype (n=5-14) (Analysed by one way ANOVA). FIG. 30C: Representative flow cytometry contour plots demonstrating CD103 expression of TCRγδ⁺Vδ2⁻ cells obtained from the colonic mucosa of a control donor and donor with active UC (UCI) after isolation by short term explant culture (Isotype control in grey). FIG. 30D: Summary data of expression levels of TCRγδ+Vδ2-cells according to disease subtype (n=5-14) (Analysed by one way ANOVA). HC: healthy control; CDU: Crohn's disease uninflamed; CDI: Crohn's disease inflamed; UCU: ulcerative colitis uninflamed; UCI: ulcerative colitis inflamed.

### Detailed Description

Aspects and embodiments of the present invention will now be discussed with reference to the accompanying figures. Further aspects and embodiments will be apparent to those skilled in the art. All documents and database accession numbers mentioned in this text are incorporated herein by reference for all purposes.

The present invention is based, in part, on the discovery that mutations in the polynucleotide region containing the genes encoding BTNL3 and BTNL8 can result in a fusion of the two proteins that is unable to traffic to the cell surface. In humans homozygous for such mutations, few, if any Vy4+ T cells, occupy the gut. In heterozygous patients, the presence of Vγ4+ T cells is diminished. The lack of Vy4+ T cells in these patients is associated (e.g., correlated) with altered risk for inflammatory bowel disease (IBD). Thus, by restoring expression of BTNL3 and BTNL8, or of transcription factors facilitating expression thereof (e.g., by gene therapy), Vy4+ T cells can be recruited to restore homeostasis in the gut and regulate inflammation. Similarly, administration of Vγ4+ T cells (e.g., Vy4+ T cells expressing heterologous proteins or Vγ4- cells expressing Vγ4) can support strategies for IBD treatment. Indeed, the inventors have shown that heterologous expression of Vγ4 on a cell was shown to be sufficient to bind and respond to BTNL3+BTNL8.

It is to be understood that aspects and embodiments of the invention described herein include "comprising," "consisting," and "consisting essentially of" aspects and embodiments. As used herein, the singular form "a," "an," and "the" includes plural references unless indicated otherwise.

The term "about" as used herein refers to the usual error range for the respective value readily known to the skilled person in this technical field. Reference to "about" a value or parameter herein includes (and describes) embodiments that are directed to that value or parameter per se. In some instances, "about" encompass variations of +20%, in some instances +10%, in some instances +5%, in some instances +1%, or in some instances +0.1% from the specified value, as such variations are appropriate to perform the disclosed methods. As used herein, the term "subject" refers to any single animal, more preferably a mammal (e.g., humans and non-human animals as non-human primates) for which treatment is considered or desired. In particular embodiments, the subject herein is a human. The subject may be a "cancer patient," i.e., one who is suffering from cancer, or at risk for suffering from cancer, or suffering from one or more symptoms of cancer.

The term "butyrophilin 3" or "BTNL3" as used herein, refers to any functional BTNL3 protein from any primate source (e.g., humans), unless otherwise indicated. Functional BTNL3 is expressed on the cell surface and associates with BTNL8 to retain Vγ4+ cells in tissues, such as gut. The amino acid sequence of an exemplary human BTNL3 is set forth in SEQ ID NO: 1 and corresponds to accession number AAQ88751.1. The nucleic acid sequence of an exemplary gene encoding human BTNL3 is shown in SEQ ID NO: 2 and corresponds to accession number NM_197975.2. Murine butyrophilin 1 or BTNL1 is an ortholog for human BTNL3. The amino acid sequence of an exemplary murine BTNL1 is set forth in SEQ ID NO: 3 and corresponds to accession number NP_001 104564.1. The nucleic acid sequence of an exemplary gene encoding murine BTNL1 is shown in SEQ ID NO: 4 and corresponds to accession number NM_001111094.1. A BTNL3 or BTNL1 protein may for example comprise a reference amino acid sequence as set out above or a variant thereof.

The term "butyrophilin 8" or "BTNL8" as used herein, refers to any functional BTNL8 protein from any primate source (e.g., humans), unless otherwise indicated. Functional BTNL8 is expressed on the cell surface and associates with BTNL3 to retain Vγ4+ cells in tissues, such as gut. The amino acid sequence of an exemplary human BTNL8 is set forth in SEQ ID NO: 5 and corresponds to accession number AAI19697.1. The nucleic acid sequence of an exemplary gene encoding human BTNL8 is shown in SEQ ID NO: 6 and corresponds to accession number NM_001040462.2. BTNL8S is an exemplary splice variant of BTNL8. The amino acid sequence of an exemplary human BTNL8S is set forth in SEQ ID NO: 7 and corresponds to accession number NP_079126.1. The nucleic acid sequence of an exemplary gene encoding human BTNL8S is shown in SEQ ID NO: 8 and corresponds to accession number NM_024850.2. A BTNL8 or BTNL8S protein may for example comprise a reference amino acid sequence as set out above or a variant thereof.

As used herein, the term "BTNL8*3 fusion protein" refers to a protein comprising BTNL8 (or a portion thereof) and BTNL3 (or a portion thereof). An exemplary BTNL8*3 fusion protein results from a ~56-kb deletion polymorphism (chr5:180375027-180430596 in hg19) as reported in Aigner et al., BMC Genetics (2013), 14:16.

As used herein, the term L3B30.2cl refers to a gene encoding BTNL-3 containing four SNPs in the L3B30.2 domain as shown in FIG. 27 or the protein encoded thereby.

The term "hepatocyte nuclear factor 4-alpha" or "HNF4A" as used herein, refers to any functional HNF4A protein from any primate source (e.g., humans) unless otherwise indicated. Functional HNF4A is a transcription factor expressed in the intestine. The amino acid sequence of an exemplary human HNF4A is set forth in SEQ ID NO: 9 and corresponds to accession number NP_001274113.1. The nucleic acid sequence of an exemplary gene encoding human HNF4A is shown in SEQ ID NO: 10 and corresponds to accession number P41235 (Gene ID: 3172). A nucleic acid sequence of an exemplary gene encoding a mouse orthologue of Hnf4a is shown in SEQ ID NO: 11, which encodes a protein having the sequence shown in SEQ ID NO: 12. A HNF4A protein may for example comprise a reference amino acid sequence as set out above or a variant thereof.

The term "CD103" or "cluster of differentiation 103" (Gene ID 3682) as used herein, refers to any functional CD103 protein from any primate source (e.g., humans) unless otherwise indicated. The amino acid sequence of an exemplary or reference human CD103 may comprise the amino acid sequence of database accession number NP_002199.3. The nucleic acid sequence of an exemplary or reference nucleotide sequence encoding human CD103 may comprise the sequence of database accession number NM_002208.4. A CD103 protein may for example comprise a reference amino acid sequence as set out above or a variant thereof.

The term "2B4" (also called CD244; Gene ID 51744) as used herein, refers to any functional 2B4 protein from any primate source (e.g., humans) unless otherwise indicated. The amino acid sequence of an exemplary or reference human 2B4 may comprise the amino acid sequence of database accession number NP_001160135.1. The nucleic acid sequence of an exemplary or reference nucleotide sequence encoding human 2B4 may comprise the sequence of database accession number NM_001166663.1. A 2B4 protein may for example comprise a reference amino acid sequence as set out above or a variant thereof.

The term "CD3" or "cluster of differentiation 3" as used herein, refers to any functional CD3 protein from any primate source (e.g., humans) unless otherwise indicated. The term CD3 may include CD3G (Gene ID 917), CD3E (Gene ID 916) and/or CD3E (Gene ID 915). In some embodiments, expression of CD3 as described herein may include expression of any one, two or all three of CD3G, CD3E and CD3D. An amino acid sequence of an exemplary or reference human CD3 may comprise the amino acid sequence of database accession number NP_000064.1 (CD3G), NP_000724.1 (CD3E) or NP_000723.1 (CD3D). The nucleic acid sequence of an exemplary or reference nucleotide sequence encoding human CD3 may comprise the sequence of database accession number NM_000073.2 (CD3G), NM_000733.3 (CD3E), or NM_000732.4 (CD3D). A CD3 protein may for example comprise a reference amino acid sequence as set out above or a variant thereof.

As used herein, a "wild-type" or WT protein or polynucleotide refers to the reference sequence from which mutated variants are derived. In general, the wild-type sequence for a given protein is the sequence that is most common in nature. Similarly, a wild-type gene sequence is the sequence for that gene which is most commonly found in nature.

A protein described herein that is a variant of a reference sequence, such as a BTNL8, BTNL3 or HNF4A sequence described above, may have 1 or more amino acid residues altered relative to the reference sequence. For example, 50 or fewer amino acid residues may be altered relative to the reference sequence, preferably 45 or fewer, 40 or fewer, 30 or fewer, 20 or fewer, 15 or fewer, 10 or fewer, 5 or fewer or 3 or fewer, 2 or 1. For example, a variant described herein may comprise the sequence of a reference sequence with 50 or fewer, 45 or fewer, 40 or fewer, 30 or fewer, 20 or fewer, 15 or fewer, 10 or fewer, 5 or fewer, 3 or fewer, 2 or 1 amino acid residues mutated. For example, a chimeric protein described herein may comprise an amino acid sequence with 50 or fewer, 45 or fewer, 40 or fewer, 30 or fewer, 20 or fewer, 15 or fewer, 10 or fewer, 5 or fewer, 3 or fewer, 2 or 1 amino acid residue altered relative to any one of SEQ ID NOs: 1, 3, 5, 7, or 9.

An amino acid residue in the reference sequence may be altered or mutated by insertion, deletion or substitution, preferably substitution for a different amino acid residue. Such alterations may be caused by one or more of addition, insertion, deletion or substitution of one or more nucleotides in the encoding nucleic acid.

A protein as described herein that is a variant of a reference sequence, such as a BTNL8, BTNL3 or HNF4A sequence described above, may share at least 50% sequence identity with the reference amino acid sequence, at least 55%, at least 60%, at least 65%, at least 70%, at least about 80%, at least 90%, at least 95%, at least 98% or at least 99% sequence identity. For example, a variant of a protein described herein may comprise an amino acid sequence that has at least 50% sequence identity with the reference amino acid sequence, at least 55%, at least 60%, at least 65%, at least 70%, at least about 80%, at least 90%, at least 95%, at least 98% or at least 99% sequence identity with the reference amino acid sequence, for example one or more of SEQ ID NOs: 1, 3, 5, 7, or 9.

Sequence identity is commonly defined with reference to the algorithm GAP (Wisconsin GCG package, Accelerys Inc, San Diego USA). GAP uses the Needleman and Wunsch algorithm to align two complete sequences that maximizes the number of matches and minimizes the number of gaps. Generally, default parameters are used, with a gap creation penalty = 12 and gap extension penalty = 4. Use of GAP may be preferred but other algorithms may be used, e.g. BLAST (which uses the method of Altschul et al. (1990) J. Mol. Biol. 215: 405-410), FASTA (which uses the method of Pearson and Lipman (1988) PNAS USA 85: 2444-2448), or the Smith-Waterman algorithm (Smith and Waterman (1981) J. Mol Biol. 147: 195-197), or the TBLASTN program, of Altschul et al. (1990) supra, generally employing default parameters. In particular, the psi-Blast algorithm may be used (Nucl. Acids Res. (1997) 25 3389-3402). Sequence identity and similarity may also be determined using Genomequest^{™} software (Gene-IT, Worcester MA USA).

Sequence comparisons are preferably made over the full-length of the relevant sequence described herein.

A "reference level," "reference expression level," or "reference sample," as used herein, refers to a level, an expression level, a sample, or a standard that is used for comparison purposes. For example, a reference sample can be obtained from a healthy individual (e.g., an individual expressing functionallevels of BTNL3 and/or BTNL8). A reference level can be the level of expression of one or more reference samples. For example, an average expression (e.g., a mean expression or median expression) among a plurality of individuals (e.g., healthy individuals, or individuals expressing functional levels of BTNL3 and/or BTNL8). In other instances, a reference level can be a predetermined threshold level, e.g., based on functional expression as otherwise determined, e.g., by empirical assays.

As used herein, a "Vγ4+ cell" or a "Vγ4+ T cell" refers to a Vγ4Vδ1 T cell (e.g., a CD3+ T cell that expresses Vδ1 and Vγ4) or a Vγ4Vδ3 T cell (e.g., a CD3+ T cell that expresses Vδ3 and Vγ4). A Vγ4+ cell can express Vδ1 or Vδ3, and/or Vγ4 as an endogenous protein or as a heterologous protein. In some instances, a Vγ4+ cell is a non-haematopoietic cell, as defined in WO 2017/072367.

As used herein, a cell or population of cells that has been "screened for" expression of a marker (e.g., Vγ4) refers to a cell or population of cells in which the marker has been explicitly identified and/or quantified ex vivo. In some embodiments, for example, a population of Vγ4+ cells that are administered to a subject will have been screened for expression of Vγ4 (e.g., after isolation and/or expansion from a donor and prior to administration to the subject) to confirm the presence of Vγ4+ cells, the total number of Vy4+ cells, a frequency of Vγ4+ cells within a given population of cells (e.g., a percentage of Vγ4+ cells among the total number of Vδ1+ cells, a percentage of Vy4+ cells among the total number of Vδ3+ cells, a percentage of Vγ4+ cells among the total number of γδ T cells, or a percentage of Vγ4+ cells among the total number of T cells), a mean or median Vγ4 expression level among a given population, or any measure of one or more such characteristics.

As used herein, a cell that is "derived from" a cell of a different phenotype refers to a cell that has been modified from an endogenous cell type. For example, a Vγ4+ cell that is derived from a Vγ4- cell describes a cell that was endogenously negative for Vγ4, but became Vγ4+ upon transduction with a gene encoding Vγ4.

A cell or population of cells that "expresses" a marker of interest is one in which mRNA encoding the protein, or the protein itself, including fragments thereof, is determined to be present in the cell or the population. Expression of a marker can be detected by various means. For example, in some embodiments, expression of a marker refers to a surface density of the marker on a cell. Mean fluorescence intensity (MFI), for example, as used as a readout of flow cytometry, is representative of the density of a marker on a population of cells. A person of skill in the art will understand that MFI values are dependent on staining parameters (e.g., concentration, duration, and temperature) and fluorochrome composition. However, MFI can be quantitative when considered in the context of appropriate controls. For instance, a population of cells can be said to express a marker if the MFI of an antibody to that marker is significantly higher than the MFI of an appropriate isotype control antibody on the same population of cells, stained under equivalent conditions. Additionally or alternatively, a population of cells can be said to express a marker on a cell-by-cell basis using a positive and negative gate according to conventional flow cytometry analytical methods (e.g., by setting the gate according to isotype or "fluorescence-minus-one" (FMO) controls). By this metric, a population can be said to "express" a marker if the number of cells detected positive for the marker is significantly higher than background (e.g., by gating on an isotype control).

As used herein, when a population's expression is stated as a percentage of positive cells and that percentage is compared to a corresponding percentage of positive cells of a reference population, the percentage difference is a percentage of the parent population of each respective population. For example, if a marker is expressed on 10% of the cells of population A, and the same marker is expressed on 1% of the cells of population B, then population A is said to have a 9% greater frequency of markerpositive cells than population B (i.e., 10%-1%, not 10%÷1%). When a frequency is multiplied through by the number of cells in the parent population, the difference in absolute number of cells is calculated. In the example given above, if there are 100 cells in population A, and 10 cells in population B, then population A has 100-fold the number of cells relative to population B, i.e., (10% × 100) - (1% × 10).

An expression level of a marker may be a nucleic acid expression level (e.g., a DNA expression level or an RNA expression level, e.g., an mRNA expression level). Any suitable method of determining a nucleic acid expression level may be used. In some embodiments, the nucleic acid expression level is determined using qPCR, rtPCR, RNA-seq, multiplex qPCR or RT-qPCR, microarray analysis, serial analysis of gene expression (SAGE), MassARRAY technique, in situ hybridization (e.g., FISH), or combinations thereof.

As used herein, "treatment" (and grammatical variations thereof such as "treat" or "treating") refers to clinical intervention, whether of a human or an animal (e.g. in veterinary applications), in which some desired therapeutic effect is achieved, for example, the inhibition or delay of the progress of the condition, and includes a reduction in the rate of progress, a halt in the rate of progress, amelioration of the condition, cure or remission (whether partial or total) of the condition, preventing, delaying, abating, or arresting one or more symptoms and/or signs of the condition, or prolonging survival of a subject or patient beyond that expected in the absence of treatment.

Treatment as a prophylactic measure (i.e. prophylaxis) is also included. For example, a patient, subject, or individual susceptible to or at risk of the occurrence or re-occurrence of inflammation may be treated as described herein. Such treatment may prevent or delay the occurrence or re-occurrence of inflammation in the patient, subject, or individual.

As used herein, "administering" is meant a method of giving a dosage of a therapeutic composition (e.g., a pharmaceutical composition) to a subject. The compositions utilized in the methods described herein can be administered, for example, intramuscularly, intravenously, intradermally, percutaneously, intraarterially, intraperitoneally, intralesionally, intracranially, intraarticularly, intraprostatically, intrapleurally, intratracheally, intrathecally, intranasally, intravaginally, intrarectally, topically, intratumourally, peritoneally, subcutaneously, subconjunctivally, intravesicularly, mucosally, intrapericardially, intraumbilically, intraocularly, intraorbitally, intravitreally (e.g., by intravitreal injection), by eye drop, orally, topically, transdermally, by inhalation, by injection, by implantation, by infusion, by continuous infusion, by localized perfusion bathing target cells directly, by catheter, by lavage, in cremes, or in lipid compositions. The compositions utilized in the methods described herein can also be administered systemically or locally. The method of administration can vary depending on various factors (e.g., the therapeutic agent or composition being administered and the severity of the condition, disease, or disorder being treated).

The term "pharmaceutical composition" refers to a preparation which is in such form as to permit the biological activity of one or more active ingredients contained therein to be effective, and which contains no additional components which are unacceptably toxic to a patient to which the formulation would be administered.

Cells obtained by any of the methods described herein (e.g., Vγ4+ T cells, e.g., Vγ4+ T cells expressing a heterologous protein) may be used as a medicament, for example, as an adoptive T cell therapy. The therapy may be autologous, or the therapy may be allogeneic. In instances involving transfer of Vγ4+ T cells, the Vγ4+ T cells may be substantially free of non-Vγ4+ T cells, such as Vγ9δ2 T cells or αβ T cells. For example, non-Vγ4+ T cells (e.g., Vγ9δ2 T cells or αβ T cells) may be depleted from the Vγ4+ T cell population prior to in vivo expansion, after in vivo expansion, or at any point during in vivo expansion using any suitable means known in the art (e.g., by negative selection, e.g., using magnetic beads). In other embodiments, the cells are not selected, and a mixed population of cells may be administered.

In some instances, the population of Vγ4+ T cells administered to the patient is part of a larger population including non-Vγ4+ T cells, such as Vγ9δ2 T cells and αβ T cells. Vγ4+ T cells can also be part of a population that includes αβ T cells, NK cells, B cells, and innate lymphoid cells (ILC). Vγ4+ T cells may account for 1 % to 99% of the total population of cells administered to the patient in a single dose or over the course of a regimen (e.g., 5% to 95%, 10% to 90%, 15% to 85%, 20% to 80%, 25% to 75%, 30% to 70%, 35% to 65%, 40% to 60%, or 45% to 55%, e.g., at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95%, e.g., no more than 5%, no more than 10%, no more than 15%, no more than 20%, no more than 25%, no more than 30%, no more than 35%, no more than 40%, no more than 45%, no more than 50%, no more than 55%, no more than 60%, no more than 65%, no more than 70%, no more than 75%, no more than 80%, no more than 85%, no more than 90%, or no more than 95%, e.g., about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, or about 95% of the total population of cells administered to the patient in a single dose or over the course of a regimen).

In some embodiments, a dose of expanded cells (e.g., Vy4+ T cells, e.g., Vy4+ T cells expressing a heterologous protein) comprises about 1 × 10⁶, 1.1 × 10⁶, 2 × 10⁶, 3.6 × 10⁶, 5 × 10⁶, 1 × 10⁷, 1.8 × 10⁷, 2 × 10⁷, 5 × 10⁷, 1 × 10⁸, 2 × 10⁸, or 5 × 10⁸ cells/kg. In some embodiments, a dose of cells (e.g., Vy4+ T cells, e.g., Vy4+ T cells expressing a heterologous protein) comprises at least about 1 × 10⁶, 1.1 × 10⁶, 2 × 10⁶, 3.6 × 10⁶, 5 × 10⁶, 1 × 10⁷, 1.8 × 10⁷, 2 × 10⁷, 5 × 10⁷, 1 × 10⁸, 2 × 10⁸, or 5 × 10⁸ cells/kg. In some embodiments, a dose of expanded cells (e.g., Vy4+ T cells, e.g., Vy4+ T cells expressing a heterologous protein) comprises up to about 1 × 10⁶, 1.1 × 10⁶, 2 × 10⁶, 3.6 × 10⁶, 5 × 10⁶, 1 × 10⁷, 1.8 × 10⁷, 2 × 10⁷, 5 × 10⁷, 1 × 10⁸, 2 × 10⁸, or 5 × 10⁸ cells/kg. In some embodiments, a dose of expanded cells (e.g., Vy4+ T cells, e.g., Vy4+ T cells expressing a heterologous protein) comprises about 1.1 × 10⁶- 1.8 × 10⁷ cells/kg. In some embodiments, a dose of expanded cells (e.g., Vy4+ T cells, e.g., Vy4+ T cells expressing a heterologous protein) comprises about 1 × 10⁷, 2 × 10⁷, 5 × 10⁷, 1 × 10⁸, 2 × 10⁸, 5 × 10⁸, 1 × 10⁹, 2 × 10⁹, or 5 × 10⁹ cells. In some embodiments, a dose of expanded cells (e.g., Vy4+ T cells, e.g., Vy4+ T cells expressing a heterologous protein) comprises at least about 1 × 10⁷, 2 × 10⁷, 5 × 10⁷, 1 × 10⁸, 2 × 10⁸, 5 × 10⁸, 1 × 10⁹, 2 × 10⁹, or 5 × 10⁹ cells. In some embodiments, a dose of expanded cells (e.g., Vy4+ T cells, e.g., Vy4+ T cells expressing a heterologous protein) comprises up to about 1 × 10⁷, 2 × 10⁷, 5 × 10⁷, 1 × 10⁸, 2 × 10⁸, 5 × 10⁸, 1 × 10⁹, 2 × 10⁹, or 5 × 10⁹ cells. In some embodiments, the subject is administered 10⁴ to 10⁶ cells (e.g., Vy4+ T cells, e.g., Vy4+ T cells expressing a heterologous protein) per kg body weight of the subject. In some embodiments, the subject receives an initial administration of a population of cells (e.g., Vy4+ T cells, e.g., Vy4+ T cells expressing a heterologous protein, e.g., an initial administration of 10⁴ to 10⁶ cells per kg body weight of the subject, e.g., 10⁴ to 10⁵ cells per kg body weight of the subject), and one or more (e.g., 2, 3, 4, or 5) subsequent administrations of cells (e.g., Vy4+ T cells, e.g., Vy4+ T cells expressing a heterologous protein, e.g., one or more subsequent administration of 10⁴ to 10⁶ expanded non-haematopoietic tissue-resident γδ T cells per kg body weight of the subject, e.g., 10⁴ to 10⁵ expanded cells per kg body weight of the subject). In some embodiments, the one or more subsequent administrations are administered less than 15 days, e.g., 14, 13, 12, 11, 10, 9, 8, 7, 6, 5, 4, 3, or 2 days after the previous administration, e.g., less than 4, 3, or 2 days after the previous administration. The treatment can be administered once, or, optionally, repeated one or more times, e.g., once weekly, once biweekly, once monthly, once bimonthly, three times annually, twice annually, or once annually. In some embodiments, the subject receives a total of about 10⁶ cells (e.g., Vγ4+ T cells, e.g., Vγ4+ T cells expressing a heterologous protein) per kg body weight of the subject over the course of at least three administrations of a population of cells, e.g., the subject receives an initial dose of 1 × 10⁵ cells, a second administration of 3 × 10⁵ cells, and a third administration of 6 × 10⁵ cells, and each administration is administered less than 4, 3, or 2 days after the previous administration.

Vγ4+ T cells useful as part of the present invention can be derived from any suitable cell source. For example, Vγ4+ cells for use as part of the compositions and methods of the invention can be derived from solid tissue (e.g., epithelial tissue, such as tissue of the gastrointestinal epithelium (e.g., from a biopsy of the colon, e.g., the ascending colon), or skin tissue) or a body fluid, such as blood. Cells derived from a tissue can be manipulated after isolation from a donor tissue, e.g., by separation (e.g., positive or negative selection from another population). In some instances, lymphocytes are separated from solid tissue using an organotypic cell culture, such as that described by Clark, et al (Clark, et al., Journal of Investigational Dermatology. 2006. 126(5):1059-70) and International Patent Application No. WO 2017/072367, each of which are incorporated herein by reference in its entirety. Cells can be expanded according to known methods, prior to and/or after a separation step. Cells for use in the present invention can be autologous or allogeneic.

In some embodiments, Vγ4+ T cells useful as part of the present invention can be derived from induced pluripotent stem cells (iPSCs). For example, the Vγ4+ T cells may be differentiated from iPSCs or may be descendants of such cells. Methods of generating T cells from iPSCs are well-known in the art (see for example WO2018/147801).

The invention includes generation and use of cells (e.g., Vγ4+ cells) that express a heterologous protein. For example, Vγ4+ cells can be endogenous Vγ4δ1 cells that are transduced with a gene encoding a heterologous protein. In other instances, Vγ4+ cells can be derived from Vγ4- cells and the Vγ4 is transduced as the heterologous protein. Suitable methods for the transduction of mammalian cells to express heterologous proteins, for example using viral vectors are well known in the art. In this case, the Vγ4- cell may be any suitable cell type, including an immune cell (e.g., a monocyte, a macrophage, a dendritic cell, a neutrophil, an eosinophil, a basophil, a mast cell, or a lymphocyte, such as a T cell, a B cell, an ILC, or an NK cell). For example, the immune cell can be a lymphocyte. In some embodiments, the lymphocyte is a T cell (e.g., a CD8 T cell, a CD4 T cell, or a regulatory T cell (e.g., a Foxp3+ Treg)) or an NK cell. For example, CD8 and/or CD4 T cells can be used for treatment of cancer of the gut. Alternatively, Tregs or other suppressive T cells can be used for treatment of inflammation in the gut.

In some preferred embodiments, the T cell is a γδ T cell (e.g., a Vδ2 cell, e.g., a Vγ9δ2 cell).

In some embodiments, one or more additional therapeutic agents can be administered to the subject. The additional therapeutic agent may be selected from the group consisting of an immunotherapeutic agent, a cytotoxic agent, a growth inhibitory agent, a radiation therapy agent, an anti-angiogenic agent, or a combination of two or more agents thereof. The additional therapeutic agent may be administered concurrently with, prior to, or after administration of the cells (e.g., Vy4+ T cells, e.g., Vy4+ T cells expressing a heterologous protein). The additional therapeutic agent may be an immunotherapeutic agent, which may act on a target within the subject's body (e.g., the subject's own immune system) and/or on the transferred cells. The administration of the compositions may be carried out in any convenient manner. The compositions described herein may be administered to a patient transarterially, subcutaneously, intradermally, intranodally, intramedullary, intramuscularly, by intravenous injection, intrarectally, intraperitoneally, by intradermal or subcutaneous injection, admixed with fecal transfer material (e.g., as part of a faecal transplant (e.g., by colonoscopy, enema, or orogastric tube), or orally.

Pharmaceutical compositions may include cells as described herein (e.g., Vy4+ T cells) in combination with one or more pharmaceutically or physiologically acceptable carrier, diluents, or excipients. Such compositions may include buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Cryopreservation solutions which may be used in the pharmaceutical compositions of the invention include, for example, DMSO. Compositions can be formulated, e.g., for intravenous administration.

Subjects that may be treated with the compositions (e.g., cells or vectors) and according to the methods described herein include subjects having or at risk of developing IBD. IBD includes disorders that involve chronic inflammation of the digestive tract, and types of IBD include ulcerative colitis (UC) and Crohn's disease. The compositions and methods described herein can also be used to treat subjects with cancer, such as subjects with colorectal cancer, colon cancer, rectal cancer, anal cancer, hereditary nonpolyposis colorectal cancer (HNPCC), familial adenomatous polyposis (FAP), small intestine cancer (e.g., adenocarcinoma, sarcoma, gastrointestinal carcinoid tumours, lymphoma, or gastrointestinal stromal tumours), and small bowel cancer. In some embodiments, the cancer is a gastrointestinal cancer, such as non-metastatic or metastatic colorectal cancer, pancreatic cancer, gastric cancer, or hepatocellular cancer. The compositions and methods described herein can be used to treat a subject with normal numbers of γδ T cells in the gut, reduced numbers of γδ T cells in the gut, reduced cell surface expression of BTNL3 and/or BTNL8, normal surface expression of BTNL3 and/or BTNL8, a mutation in BTNL3 and/or BTNL8, reduced expression of HNF4A, or normal expression of HNF4A. Compositions (e.g., cells or vectors) of the invention are administered in an amount sufficient to improve one or more of the symptoms of IBD or cancer. The compositions described herein can be administered in an amount sufficient to reduce or inhibit one or more of the following symptoms: diarrhoea, fever, fatigue, abdominal pain and cramping, reduced appetite, or unintended weight loss. The compositions described herein can be administered in an amount sufficient to treat the cancer or tumour, cause remission, reduce tumour growth, volume, metastasis, invasion, proliferation, or number, increase cancer cell death, increase time to recurrence, or improve survival. The compositions described herein can be administered in an amount sufficient to increase the number of γδ T cells in the gut, or, in the case of administration of a vector, to increase BTNL3 cell surface expression, increase BTNL8 cell surface expression, and/or increase HNF4A expression. The compositions described herein may reduce one or more IBD symptoms by 10%, 15%, 20%, 25%, 30%, 35%, 40%, 45%, 50%, 55%, 60%, 65%, 70%, 75%, 80%, 85%, 90%, 95%, or 100%. These effects may occur, for example, within 1 week, 2 weeks, 3 weeks, 4 weeks, 5 weeks, 6 weeks, 7 weeks, 8 weeks, 9 weeks, 10 weeks, 15 weeks, 20 weeks, 25 weeks, or more, following administration of the compositions described herein. The patient may be evaluated, for instance, 2 weeks, 1 month, 2 months, 3 months, 4 months, 5 months, 6 months or more following administration of the composition, depending on the route of administration used for treatment.

Any expression products provided by the present invention (e.g., BTNL3/8, Vγ4δ1 TCR, Vγ4δ3 TCR, or HNF4A) can be encoded on vectors known in the art or described herein. In some instances, BTNL3 and BTNL8 are delivered together (e.g., on the same vector) to facilitate dimerization on the cell surface. Similarly, Vδ1 and Vγ4 or Vδ3 and Vγ4 can be delivered together (e.g., on the same vector) to facilitate correct TCR assembly.

In addition to achieving high rates of transcription and translation, stable expression of an exogenous gene in a mammalian cell can be achieved by integration of the polynucleotide containing the gene into the nuclear genome of the mammalian cell. A variety of vectors for the delivery and integration of polynucleotides encoding exogenous proteins into the nuclear DNA of a mammalian cell have been developed. Examples of expression vectors are disclosed in, e.g., WO 1994/011026 and are incorporated herein by reference. Expression vectors for use in the compositions and methods described herein contain a polynucleotide sequence that encodes BTNL3, BTNL8, Vδ1, Vδ3, Vy4, or HNF4A, as well as, e.g., additional sequence elements used for the expression of these agents and/or the integration of these polynucleotide sequences into the genome of a mammalian cell. Certain vectors that can be used for the expression of BTNL3, BTNL8, Vδ1, Vδ3, Vγ4, or HNF4A include plasmids that contain regulatory sequences, such as promoter and enhancer regions, which direct gene transcription. Other useful vectors for expression of BTNL3, BTNL8, Vδ1, Vδ3, Vγ4, or HNF4A contain polynucleotide sequences that enhance the rate of translation of these genes or improve the stability or nuclear export of the mRNA that results from gene transcription. These sequence elements include, e.g., 5' and 3' untranslated regions, an internal ribosomal entry site (IRES), and polyadenylation signal site in order to direct efficient transcription of the gene carried on the expression vector. The expression vectors suitable for use with the compositions and methods described herein may also contain a polynucleotide encoding a marker for selection of cells that contain such a vector. Suitable marker include genes that encode resistance to antibiotics, such as ampicillin, chloramphenicol, kanamycin, or nourseothricin.

Expression vectors for use in the compositions and methods described herein may express BTNL3, BTNL8, Vδ1, Vδ3, Vγ4, or HNF4A from monocistronic or polycistronic expression cassettes. A monocistronic expression cassette contains a polynucleotide sequence that encodes a single gene. Pluripotent cells described herein can be transfected with multiple plasmids, for example, each containing a monocistronic expression cassette, or with a single plasmid containing more than one monocistronic expression cassette. Polycistronic expression cassettes can be used to simultaneously express two or more proteins from a single transcript. Polycistronic expression cassettes include bicistronic expression cassettes, which can be used to generate two proteins from a single transcript and may include IRES sequences to recruit ribosomes to initiate translation from a region of the mRNA other than the 5' cap.

Viral genomes provide a rich source of vectors that can be used for the efficient delivery of exogenous genes, such as BTNL 3/8 and Vγ4δ1 or Vγ4δ3 TCR, into a mammalian cell. Viral genomes are particularly useful vectors for gene delivery because the polynucleotides contained within such genomes are typically incorporated into the nuclear genome of a mammalian cell by generalized or specialized transduction. These processes occur as part of the natural viral replication cycle, and do not require added proteins or reagents in order to induce gene integration. Examples of viral vectors include a retrovirus (e.g., Retroviridae family viral vector), adenovirus (e.g., Ad5, Ad26, Ad34, Ad35, and Ad48), parvovirus (e.g., adeno-associated viruses), coronavirus, negative strand RNA viruses such as orthomyxovirus (e.g., influenza virus), rhabdovirus (e.g., rabies and vesicular stomatitis virus), paramyxovirus (e.g. measles and Sendai), positive strand RNA viruses, such as picornavirus and alphavirus, and double stranded DNA viruses including adenovirus, herpesvirus (e.g., Herpes Simplex virus types 1 and 2, Epstein-Barr virus, cytomegalovirus), and poxvirus (e.g., vaccinia, modified vaccinia Ankara (MVA), fowlpox and canarypox). Other viruses include Norwalk virus, togavirus, flavivirus, reoviruses, papovavirus, hepadnavirus, human papilloma virus, human foamy virus, and hepatitis virus, for example. Examples of retroviruses include: avian leukosis-sarcoma, avian C-type viruses, mammalian C-type, B-type viruses, D-type viruses, oncoretroviruses, HTLV-BLV group, lentivirus, alpharetrovirus, gammaretrovirus, spumavirus (Coffin, J. M., Retroviridae: The viruses and their replication, Virology, Third Edition (Lippincott-Raven, Philadelphia, 1996)). Other examples include murine leukemia viruses, murine sarcoma viruses, mouse mammary tumour virus, bovine leukemia virus, feline leukemia virus, feline sarcoma virus, avian leukemia virus, human T-cell leukemia virus, baboon endogenous virus, Gibbon ape leukemia virus, Mason Pfizer monkey virus, simian immunodeficiency virus, simian sarcoma virus, Rous sarcoma virus and lentiviruses. Other examples of vectors are described, for example, in McVey et al. (US 5,801,030), the teachings of which are incorporated herein by reference.

Nucleic acids of the compositions and methods described herein may be incorporated into rAAV vectors and/or virions in order to facilitate their introduction into a cell. AAV vectors can be used in the central nervous system, and appropriate promoters and serotypes are discussed in Pignataro et al., J Neural Transm (2017), epub ahead of print, the disclosure of which is incorporated herein by reference as it pertains to promoters and AAV serotypes useful in CNS gene therapy. rAAV vectors useful in the compositions and methods described herein are recombinant nucleic acid constructs that include (1) a heterologous sequence to be expressed and (2) viral sequences that facilitate integration and expression of the heterologous genes. The viral sequences may include those sequences of AAV that are required in cis for replication and packaging (e.g., functional ITRs) of the DNA into a virion. Such rAAV vectors may also contain marker or reporter genes. Useful rAAV vectors have one or more of the AAV WT genes deleted in whole or in part, but retain functional flanking ITR sequences. The AAV ITRs may be of any serotype suitable for a particular application. Methods for using rAAV vectors are described, for example, in Tal et al., J. Biomed. Sci. 7:279 (2000), and Monahan and Samulski, Gene Delivery 7:24 (2000), the disclosures of each of which are incorporated herein by reference as they pertain to AAV vectors for gene delivery.

The nucleic acids and vectors described herein can be incorporated into a rAAV virion in order to facilitate introduction of the nucleic acid or vector into a cell. The capsid proteins of AAV compose the exterior, non-nucleic acid portion of the virion and are encoded by the AAV cap gene. The cap gene encodes three viral coat proteins, VP1, VP2 and VP3, which are required for virion assembly. The construction of rAAV virions has been described, for instance, in US 5,173,414; US 5,139,941; US 5,863,541; US 5,869,305; US 6,057,152; and US 6,376,237; as well as in Rabinowitz et al., J. Virol. 76:791 (2002) and Bowles et al., J. Virol. 77:423 (2003), the disclosures of each of which are incorporated herein by reference as they pertain to AAV vectors for gene delivery.

rAAV virions useful in conjunction with the compositions and methods described herein include those derived from a variety of AAV serotypes including AAV 1, 2, 3, 4, 5, 6, 7, 8, 9, 10 and rh74. For targeting cells located in or delivered to the central nervous system, AAV2, AAV9, and AAV10 may be particularly useful. Construction and use of AAV vectors and AAV proteins of different serotypes are described, for instance, in Chao et al., Mol. Ther. 2:619 (2000); Davidson et al., Proc. Natl. Acad. Sci. USA 97:3428 (2000); Xiao et al., J. Virol. 72:2224 (1998); Halbert et al., J. Virol. 74:1524 (2000); Halbert et al., J. Virol. 75:6615 (2001); and Auricchio et al., Hum. Molec. Genet. 10:3075 (2001), the disclosures of each of which are incorporated herein by reference as they pertain to AAV vectors for gene delivery. Also useful in conjunction with the compositions and methods described herein are pseudotyped rAAV vectors. Pseudotyped vectors include AAV vectors of a given serotype pseudotyped with a capsid gene derived from a serotype other than the given serotype (e.g., AAV1, AAV2, AAV3, AAV4, AAV5, AAV6, AAV7, AAV8, AAV9, and AAV10, among others etc.). Techniques involving the construction and use of pseudotyped rAAV virions are known in the art and are described, for instance, in Duan et al., J. Virol. 75:7662 (2001); Halbert et al., J. Virol. 74:1524 (2000); Zolotukhin et al., Methods, 28:158 (2002); and Auricchio et al., Hum. Molec. Genet. 10:3075 (2001).AAV virions that have mutations within the virion capsid may be used to infect particular cell types more effectively than non-mutated capsid virions. For example, suitable AAV mutants may have ligand insertion mutations for the facilitation of targeting AAV to specific cell types. The construction and characterization of AAV capsid mutants including insertion mutants, alanine screening mutants, and epitope tag mutants is described in Wu et al., J. Virol. 74:8635 (2000). Other rAAV virions that can be used in methods described herein include those capsid hybrids that are generated by molecular breeding of viruses as well as by exon shuffling. See, e.g., Soong et al., Nat. Genet., 25:436 (2000) and Kolman and Stemmer, Nat. Biotechnol. 19:423 (2001).

The delivery vector used in the methods and compositions described herein may be a retroviral vector. One type of retroviral vector that may be used in the methods and compositions described herein is a lentiviral vector. Lentiviral vectors (LVs), a subset of retroviruses, transduce a wide range of dividing and non-dividing cell types with high efficiency, conferring stable, long-term expression of the transgene. An overview optimization strategies for lentiviral vectors is provided in Delenda, The Journal of Gene Medicine 6: S125 (2004), the disclosure of which is incorporated herein by reference. The use of lentivirus-based gene transfer techniques relies on the in vitro production of recombinant lentiviral particles carrying a highly deleted viral genome in which the transgene of interest is accommodated. In particular, the recombinant lentivirus are recovered through the in trans co-expression in a permissive cell line of (1) the packaging constructs, i.e., a vector expressing the Gag-Pol precursors together with Rev (alternatively expressed in trans); (2) a vector expressing an envelope receptor, generally of an heterologous nature; and (3) the transfer vector, consisting in the viral cDNA deprived of all open reading frames, but maintaining the sequences required for replication, encapsidation, and expression, in which the sequences to be expressed are inserted. A lentiviral vector used in the methods and compositions described herein may include one or more of a 5'-Long terminal repeat (LTR), HIV signal sequence, HIV Psi signal 5'-splice site (SD), delta-GAG element, Rev Responsive Element (RRE), 3'-splice site (SA), Elongation factor (EF) 1-alpha promoter and 3'-self inactivating LTR (SIN-LTR). The lentiviral vector optionally includes a central polypurine tract (cPPT) and a woodchuck hepatitis virus post-transcriptional regulatory element (WPRE), as described in US 6,136,597, the disclosure of which is incorporated herein by reference as it pertains to WPRE. The lentiviral vector may further include a pHR' backbone, which may include for example as provided below.

The Lentigen lentiviral vector described in Lu et al., Journal of Gene Medicine 6:963 (2004) may be used to express the DNA molecules and/or transduce cells. A lentiviral vector used in the methods and compositions described herein may include a 5'-Long terminal repeat (LTR), HIV signal sequence, HIV Psi signal 5'-splice site (SD), delta-GAG element, Rev Responsive Element (RRE), 3'-splice site (SA), Elongation factor (EF) 1-alpha promoter and 3'-self inactivating LTR (SIN-LTR). It will be readily apparent to one skilled in the art that optionally one or more of these regions is substituted with another region performing a similar function. Transgene expression is driven by a promoter sequence. Optionally, the lentiviral vector includes a CMV promoter. The promoter may also be Elongation factor (EF) 1-alpha promoter or PGK promoter. A person skilled in the art will be familiar with a number of promoters that will be suitable in the vector constructs described herein.

Enhancer elements can be used to increase expression of modified DNA molecules or increase the lentiviral integration efficiency. The lentiviral vector used in the methods and compositions described herein may further include a nef sequence. The lentiviral vector used in the methods and compositions described herein may further include a cPPT sequence which enhances vector integration. The cPPT acts as a second origin of the (+)-strand DNA synthesis and introduces a partial strand overlap in the middle of its native HIV genome. The introduction of the cPPT sequence in the transfer vector backbone strongly increased the nuclear transport and the total amount of genome integrated into the DNA of target cells. The lentiviral vector used in the methods and compositions described herein may further include a Woodchuck Posttranscriptional Regulatory Element (WPRE). The WPRE acts at the transcriptional level, by promoting nuclear export of transcripts and/or by increasing the efficiency of polyadenylation of the nascent transcript, thus increasing the total amount of mRNA in the cells. The addition of the WPRE to lentiviral vector results in a substantial improvement in the level of transgene expression from several different promoters, both in vitro and in vivo. The lentiviral vector used in the methods and compositions described herein may include both a cPPT sequence and WPRE sequence. The vector may also include an internal ribosome entry site (IRES) sequence that permits the expression of multiple polypeptides from a single promoter. In addition to IRES sequences, other elements known in the art which permit expression of multiple polypeptides are useful.

The vector used in the methods and compositions described herein may, be a clinical grade vector.

Viral regulatory elements are components of delivery vehicles used to introduce nucleic acid molecules into a host cell. Viral regulatory elements are optionally retroviral regulatory elements. For example, the viral regulatory elements may be the LTR and gag sequences from HSC1 or MSCV. The retroviral regulatory elements may be from lentiviruses or they may be heterologous sequences identified from other genomic regions. One skilled in the art would also appreciate that as other viral regulatory elements are identified, these may be used with the nucleic acid molecules described herein.

Vectors of the invention may induce expression specifically in the gut through the use of gut- specific promoters (e.g., constitutively active gut-specific promoters). Such promoters are known in the art and include, e.g., intestinal fatty acid binding protein (IFABP), human mucin-2 promoter (HMUC2), human lysozyme promoter (HLY), human sucrose-isomaltase enhancer (HIS), CDX2, Villin, and PDX1.

Subjects that may be treated as described herein may include subjects having or at risk of developing inflammation of the gut, such as that associated with IBD. Whether a subject has or is at risk of developing inflammation of the gut (e.g., IBD) can be determined by identifying certain mutations that influence the presence and function of Vy4+ T cells in the gut.

A subject at risk of developing inflammation of the gut may have an increased risk of or susceptibility to the condition relative to control subjects.

A subject can be identified as having a mutation in a polynucleotide sequence encoding BTNL3 and BTNL8 (e.g., a ~56-kb deletion polymorphism (chr5:180375027-180430596 in hg19) by comparing a level of a polynucleotide sequence associated with a the BTNL8*3 mutation using the genotyping method described illustrated in FIG. 17B in a sample of the subject with a reference level of the polynucleotide sequence. The mutation can be characterized by reduced or ablated trafficking of BLTN3 and/or BTNL8 to a cell surface, e.g., relative to a reference sample, e.g., a wild-type sample. In some instances, the mutation is characterized by expression of a BTNL8*3 fusion protein. For example, the individual may be heterozygous or homozygous for a BTNL8*3 mutation.

The mutation can be a single nucleotide polymorphism (SNP), e.g., a SNP in a BTNL3 intron or a BTNL8 intron. Suitable SNPs include rs6868418 and rs4700772 (see for example the NCBI Short Genetic Variations database; dbSNP). For example, subject may be homozygous (TT) or heterozygous (CT) for T at rs6868418 (common allele CC) and/or homozygous (AA) or heterozygous (GA) for A at rs4700772 (common allele GG).

In some embodiments, the mutation may be an L3B30.2lc genotype. For example, the subject may be heterozygous (GT) or homozygous (TT) for T at rs73815153, heterozygous (CG) or homozygous (GG) for G at rs7726604, heterozygous (CG) or homozygous (GG) for G at rs7726607, and heterozygous (CG) or homozygous (GG) for G at rs59220426.

A subject identified as having a mutation that influences the presence and function of Vγ4+ T cells in the gut may be amenable to or suitable for treatment as described herein.

A subject identified as having or at risk of inflammation of the gut may be as described herein may be treated or selected for treatment in accordance with an aspect of the invention.

In some embodiments, identifying a subject as one having inflammation of the gut or likely or at risk of developing inflammation of the gut can be followed by providing a recommendation to pursue therapy (e.g., gene therapy or cell therapy, according to any of the methods described herein or known in the art).

In some preferred embodiments, an individual identified, treated or selected for treatment as described herein may lack a BTNL8*3 mutation and may be heterozygous (GT) at rs73815153, heterozygous (CG) at rs7726604, heterozygous (CG) at rs7726607, and heterozygous (CG) at rs59220426.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### EXAMPLES

The following examples are put forth so as to provide those of ordinary skill in the art with a description of how the compositions and methods described herein may be used, made, and evaluated, and are intended to be purely exemplary of the invention and are not intended to limit the scope of what the inventors regard as their invention.

### Example 1. Intestinal epithelial T cell selection

By flow cytometry of cells recovered from epithelium, and by confocal visualization of epithelial whole mounts, we found that the signature murine small intestinal Vγ7+ intraepithelial (IEL) compartment largely took shape at 2-3 weeks of age and remained stable for at least 9 months thereafter (FIGS. 1A and 1B). At day 21, Vγ7+ cells mostly phenocopied mature Skint1-selected dendritic epithelial T cells DETCs), expressing uniformly high levels of CD122 (the IL-2R/IL-15Rβ chain), TIGIT (an inhibitory co-receptor), and the TCR (detected with anti-CD3 antibodies) and low levels of RNA for Roryc and Sox13, two transcription factors contributing to γδ T cell differentiation (FIG. 1C). Vγ7- IELs (mostly Vγ1 or Vγ4) did not show this phenotype, and whereas both Vy7+ and Vγ7- IEL subsets were mostly CD45RBhi, CD44+, and CCR9+, Vy7+ IELs were distinct in being Lag3+, Thy1-, CD69+, CD5-, and CD8αα+ (FIGS. 1C and 2A).

Prior to day 21, however, Vy7+ IELs phenocopied Vγ7- IELs of adult mice. Thus, by sequential gating and radar plots of surface protein co-expression, one could clearly distinguish mature Vy7+ IELs (CD122hi[MFI > 500], Thy1-, TIGIT+, Lag3+, CD8αα+, CD5-, CD24-, TCRhi) from putative Vy7+ IEL progenitors (CD122lo[MFI < 200], Thy1+, TIGIT-, Lag3-, CD8αα-, CD5+, CD24+, TCRIo; FIGS. 1D, 1E, and 2B), with the latter also phenocopying DETC progenitors prior to Skint1 selection.

To further compare IELs with their putative progenitors, CD122hi Vγ7+ and CD122lo Vγ7+ IELs were purified from the same day 14-17 mice on four independent occasions and assessed by RNA sequencing (RNA-seq; FIG. 2C). Consistent with their distinct phenotypes, the cells showed significantly different expression of many genes for cell surface proteins (FIG. 2C). Furthermore, many genes upregulated (e.g., Tnfrsf9 [4-1BB/CD137], Xcl1 [lymphotactin], Nasp) or downregulated (e.g., sox13, Bel11b, Cx3cr1) in CD122hi versus CD122lo Vy7+ cells were likewise regulated by Skint1 selection of DETC progenitors (FIGS. 1F and 2C).

Additionally, CD122hi Vγ7+ cells were enriched in cell-cycle genes, consistent with which -100% of Vγ7+ IELs at day 21-24 were Ki67+ (i.e., outside of G0), compared to <40% of Vγ7- cells (p < 0.0001) (FIG. 1G). Likewise, Vy7+ IELs at day 28 phenocopied rapidly dividing thymocytes in that -10% incorporated ethynyldeoxyuridine (EdU) (a labeled nucleotide) during a 3-hour pulse, compared to only 4% of Vγ7-IELs (FIG. 2D). In sum, these data are consistent with the gut supporting the selective maturation and expansion of CD122hi, Thy1-, TIGIT+, Lag3+, CD8αα+, CD5-, CD24-, TCRhi Vy7+ cells that by weeks 3-4 dominate the γδ IEL compartment. After week 5, the fraction of cycling (Ki67+) Vy7+ IELs at steady state declined to levels comparable to Vγ7- IELs (FIG. 1G).

### Example 2. A gut epithelial selecting element

Because Skint1 selects for signature Vy5+ DETC progenitors in the thymus, DETCs are absent from athymic NU/NU mice. By contrast, intestinal IELs were present in NU/NU, and although there was some decrease in numbers (average of -1.3 X 106 cells compared to >2.0 X 106 cells in euthymic mice), the compartment was again dominated by CD122hi Vy7+ IELs. Moreover, -25% of Vy7+ IELs in NU/NU and in euthymic mice reacted with antibody GL2 that detects Vδ4 (TRDV2-2 encoded) chains. Consistent with this, TRDV2-2 sequences accounted for -25% of TCRδ chain RNAs expressed by purified Vy7+ IELs (FIGS. 3A and 4A). In sum, the shaping of the gut Vy7+ IEL compartment did not require a thymus.

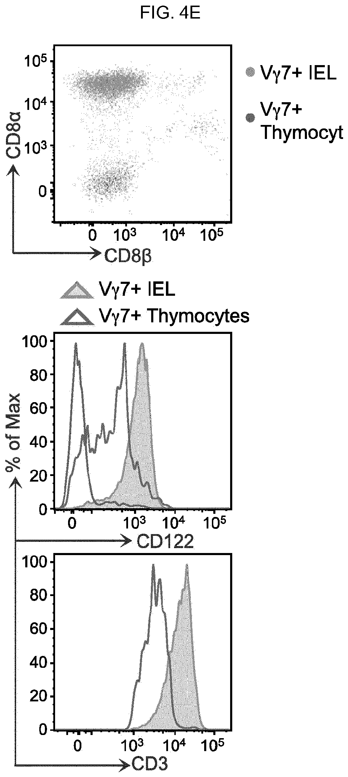

Consistent with this, Vy7+ thymocytes were rare, comprising <10% of TCRγδ+ cells in fetal and postnatal thymi across the first 8 weeks of life, the peak period of thymus function in mice (FIG. 4B). Furthermore, most Vy7+ thymocytes were CD45RBIo, Thy1+, CD5hi, CD122lo, TCRIo, and CD8αα-, thus offering no evidence for intrathymic maturation (FIGS. 4C and 4E). Likewise, neither lymph nodes nor Peyer's patches (PP) were required to shape the IEL compartment, since normal numbers of Vy7+ and Vy7GL2+ IELs with signature phenotypes were present in aly/aly (alymphoplasia) mice that following surgery were confirmed to lack PP and peripheral and mesenteric lymph nodes (MLNs; FIG. 4F).

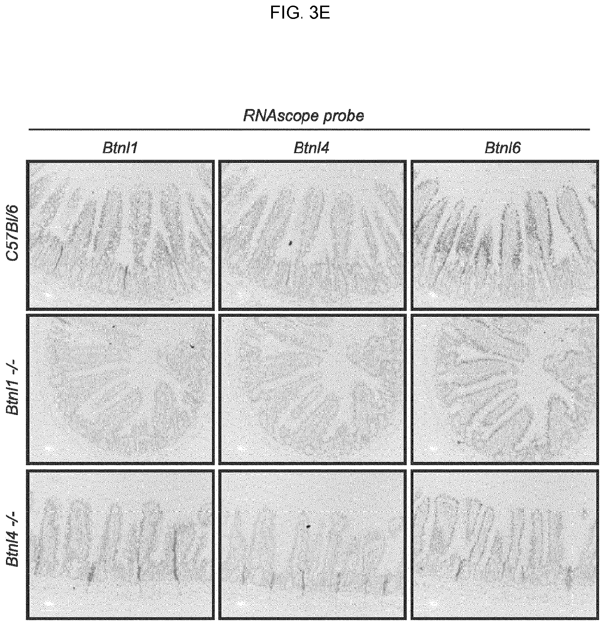

As intestinal driver(s) of IEL maturation in weanling mice, microbial and/or food antigens were logical candidates. However, C57BI/6 mice bred into and maintained in a germ-free environment and/or on elemental, protein-antigen-free diet displayed Vy7+ and Vy7GL2+ IEL compartments comparable to conventionally housed counterparts (FIG. 3B). The Vy7+ IELs were uniformly TCRhi, CD122hi, and absolute numbers were somewhat increased, partially compensating for the decline of TCRαβ IELs in germ-free and protein-antigen-free mice (FIG. 3B). Thus, the local T cell compartment is most likely shaped by an endogenous intestinal element(s). In seeking the element(s), we focused on three genes, Btnl1, Btnl4, and Btnl6, which are closely related to Skint1. Btnl4 was expressed at low levels in proximal small intestine, commencing in the fetus. Btnl1 and Btnl6 RNAs were detected at day 6 postpartum, and Btnl1 levels further increased at around day 14 before the expression of all three Btnl genes stabilized (FIG. 3C). Expression was in post-mitotic villus enterocytes that are interspersed with IELs and was essentially absent from villus crypts that house replicating epithelial cell progenitors and lack IELs (FIGS. 3D, 3E, and 4G). Btnl1 expression peaked in proximal and medial small intestine (FIG. 4H), where its expression was >107-fold higher than in the thymus (FIG. 4I). These expression patterns could permit Btnl1, Btnl4, and/or Btnl6 to act locally upon Vγ7+ IELs in weanling mice. To investigate this possibility, we obtained three independent strains of Btnl1-/- mice and one of Btnl4-/- mice, each generated by targeted mutagenesis of embryonic stem cells (ESCs), and a strain with an internally deleted Btnl1 locus generated by clustered regularly interspaced short palindromic repeats (CRISPR)-Cas9 in mouse eggs (FIG. 4J). The strains were confirmed as gene knockouts by DNA analysis and loss of respective Btnl RNAs (FIGS. 3E and 4I-4K).

### Example 3. Btnl1 shapes the intestinal IEL compartment

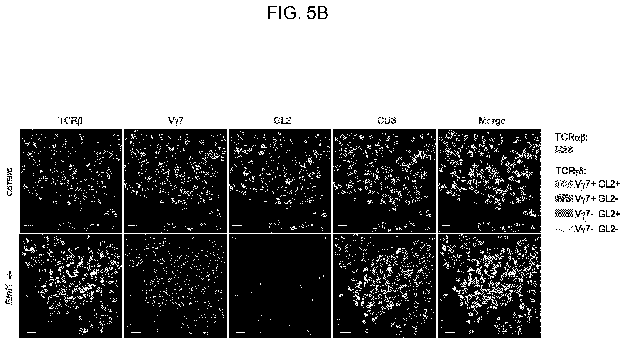

The four Btnl1-/- strains each displayed major, highly selective losses of Vγ7+ IELs, assessed by flow cytometry or confocal microscopy (FIGS. 5A, 5B, and 6A). Vγ7+ IEL numbers were depleted by -90%, with Vγ7GL2+ cells almost ablated. Because Vγ7- IEL numbers barely increased, the percentage representation of Vγ7+ cells among γδ IELs was reduced only by ~3-fold relative to wild-type (WT) mice, but this was set against a background of dramatically reduced γδ IEL numbers (FIGS. 5A and 6A). By contrast, TCRβ+CD8α+ IEL numbers increased significantly in Btnl1-/- mice (FIGS. 5A and 5B).

The specificity of Btnl1 for Vγ7+ IELs was emphasized by comprehensive immune phenotyping of Btnl1-/-, WT, and Btnl1+/- mice that showed comparable splenic or MLN immune cell subsets (including γδ cell repertoires) and comparable representation and phenotypes of Vy7+ thymocytes from day 4 to week 8 (FIGS. 6B-6E). Consistent with its expression pattern, Btnl1 acted extrathymically; thus, Btnl1 deficiency crossed onto NU/NU mice reduced the average number of Vy7+ IELs by -90%, with almost total loss of Vy7GL2+ IELs (FIG. 5C). Since NU/NU mice lack most αβ T cells, this result excludes the formal albeit unlikely possibility that Vγ7+ IEL losses in euthymic Btnl1-/- mice indirectly reflected expanded TCR αβ IELs. The specificity of Vy7+ IELs for Btnl1 was emphasized by the fact that Btnl4-/- mice displayed no overt defects in any major IEL subset (FIG. 5D).

### Example 4. Btnl1 selects Vv7+ IEL in trans

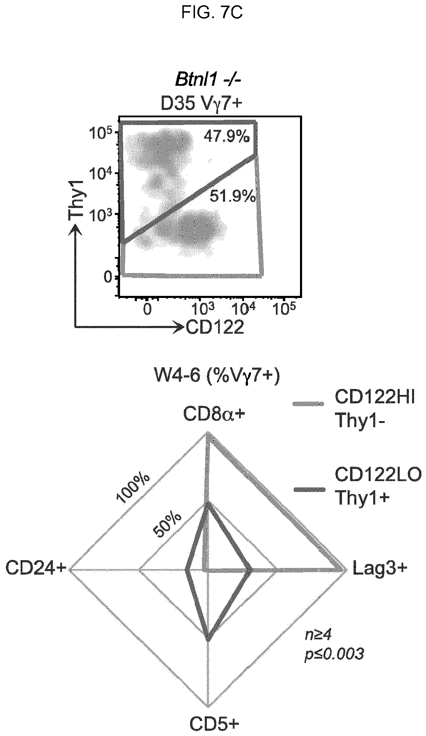

To determine how and when Btnl1 impacts IELs, we examined residual Vy7+ and Vy7GL2+ IELs in Btnl1-/- mice. Relative to those in WT mice, significantly fewer Vy7+ IELs incorporated EdU at D28 (p < 0.0001) or expressed Ki67, and this did not change until W7 when most Vy7+ IELs in WT mice moved out of cycling (FIGS. 7A and 8A). Thus, there was no selective expansion of Vγ7+ IELs in Btnl1-/- mice. Similarly, although their TCR levels were high, many residual Vy7+ and Vy7GL2+ IELs in week 3-5. Btnl1-/- mice were CD122lo, Thy1+, TIGIT-, Lag3-, CD8αα-, CD5+, CD24+, thereby phenocopying immature Vy7+ IELs of day 14-17 WT mice (FIGS. 7B, 7C, and 8B). To demonstrate that Btnl1 exerts its selective impact on Vy7+ T cells in trans, we established conditions in which the Vγ7+ and Vy7GL2+ IEL compartments of week 3-5 WT mice were reconstituted within ~5 weeks of donor bone marrow (BM) transfer to irradiated 8- to 10-week, γδ T cell-deficient TCRδ-/- mice (FIG. 8C). BM from either WT or Btnl1-/- mice proved equally effective at IEL reconstitution (FIG. 7D). WT BM reconstitution of Vγ7+ IELs in irradiated, congenic T cell-sufficient CD45.2+ WT recipients was less effective than it was in TCRδ-/- hosts (compare plots, top left in FIGS. 7D and 7E), but nonetheless, reconstitution of Btnl1-/- hosts was much less effective, and the few Vy7+ and Vy7GL2+ IELs that developed in Btnl1-/- recipients phenocopied residual Vy7+ cells in Btnl1-/- mice and immature IELs in day 14-17 WT mice (FIG. 7E). Complementing these findings, purified IELs from 4-week-old mice could reconstitute Vy7+ IELs in recipient TCRδ-/- mice, albeit very inefficiently, and this too was greatly impaired in Btnl1-/-TCRδ-/- hosts (FIG. 8D). Thus, Btnl1 acts in non-hematopoietic cells to support the selective expansion and maturation of Vγ7+ IEL.

To attempt to restore IEL selection, we rendered Btnl1-/- mice transgenic for Btnl1 expressed from a doxycycline (Dox)-inducible promoter (FIGS. 10A and 10B) and crossed them onto Btnl1-/- mice, into which was introduced a Dox-responsive trans-activator (rtTA) regulated by the ubiquitously expressed Rosa26 promoter. As reported, adding low-dose Dox to drinking water had little overt impact on the gut over a several-week period. Therefore, this system offered a means to globally induce Btnl1 de novo in Btnl1-/- bitransgenic (BiTg) mice that inherited both rtTA and the inducible Btnl1 transgene. Conversely, BiTg mice administered sugar water and Dox-treated single-transgenic (SiTg) Btnl1-/- mice (that inherited only the Btnl1 transgene) served as controls. Btnl1 induction in mice of appropriate genotypes was validated by qPCR (FIG. 10C).

When several W11 Btnl1-/- BiTg mice were treated in this way, the representation of Vy7+ and Vγ7+GL2+ IELs was unchanged relative to littermate controls, but the percentage of Vy7+ IELs that were CD122hi increased greatly, and most expressed Ki67. This was not true for Vγ7- or TCRβ+ IELs (FIGS. 10D-10F). Thus, Btnl1 induction de novo in adult mice partially phenocopied Vy7+ tEL-specific maturation in early life but did not reconstitute Vy7+ IEL numbers. Global Btnl1 induction also did not drive ectopic Vy7+ cell maturation in any other tissues.

### Example 5. A Temporal Window for Epithelial Btnl Activity

In further experiments, we restricted Btnl1 induction to mature enterocytes by generating Btnl1-/- mice transgenic for rtTA expressed from the villin promoter. Within 1-2 weeks of Dox-treatment of several W11 BiTg Btnl1-/- mice, most Vγ7+ IELs had become Lag3hi, Thy1-, and CD122hi, of which the majority were also Ki67+ (FIGS. 9A and 9B). Again, this phenotypic transition was Vy7+ IEL specific, albeit there were sometimes Btnl1-independent increases in Ki67+ TCRβ+ IELs in sugar-water-treated mice. (FIG.9B). Once more, the numbers and representation of Vy7+ IELs were unchanged, even in mice retained on Dox for 3-4 weeks (FIG. 9C). This establishes that Btnl1 can effect phenotypic conversion of immature Vy7+ IELs rather than merely promote a selective outgrowth of mature Vy7+ cells.

However, there was significantly increased representation of Vy7+ and Vγ7GL2+ IEL when Btnl1 expression was induced in Btnl1-/- mice in early-life by commencing Dox-treatment of nursing females at D7 or of weanlings at D21 and then maintaining treatment for 2-5 weeks (FIG. 9D). Moreover, the expanded Vy7+ cells phenocopied IEL of W4-5 WT mice, and were significantly different from the IEL of Dox-treated SiTg mice and Btnl1-/- mice (FIG. 9E). Thus, acute expression of Btnl1 purely in the gut epithelium induces the selective phenotypic maturation and expansion of Vy7+ IEL, but these effects are separable, with selective expansion mostly confined to a developmental window within the first five weeks of life.

### Example 6. Epithelial Btnl1 and Btnl6 regulate Vv7+ cells

Given that acute Btnl1 expression drives the selective maturation of Vy7+ IELs in vivo, we tested whether Btnl1 might show specificity for Vy7+ IEL ex vivo. Since primary intestinal epithelial cells reportedly harbor Btnl1 in a complex with Btnl6, we sought evidence for heterotypic interactions of Btnl proteins. Indeed, cell surface expression of Btnl1 on Btnl1-transfected MODE-K cells (an established intestinal epithelial cell line in which endogenous Btnl genes are negligibly expressed) was greatly enhanced by co-transfection with Btnl4 or Btnl6 (FIG. 12A). Likewise, surface Btnl6 expression was greatly enhanced by Btnl1, but there was no evidence for collaboration between Btnl6 and Btnl4 (FIG. 12A). Given the specificity of Btnl6 for Btnl1, and given that Btnl4-/- mice showed no IEL phenotype, we focused on Btnl1 and Btnl6.

MODE-K cells stably transduced with Btnl1 (L1), Btnl6 (L6), Btnl1 plus Btnl6 (L1+6), or empty vector (EV) were co-cultured with freshly explanted IELs that were then assayed for CD25 (IL-2Rα chain) upregulation, which is among the most robust readouts of TCR stimulation for systemic T cells. Whereas mixed TCRαβ+ and TCRγδ+ IELs showed minor CD25 upregulation upon co-culture with EV, L1, or L6 cells, IELs exposed to L1+6 cells showed highly significant CD25 upregulation, wholly attributable to -20% of Vy7+ cells (both Vy7GL2+ and Vy7GL2- cells; FIG. 11A). Significant CD25 upregulation was first evident within 4-6 hours, as is true for systemic TCR stimulation (FIG. 12B).

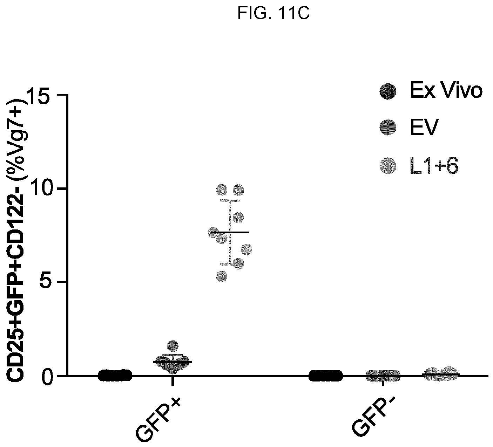

When L1+6 cells were co-cultured with primary IELs from Nur77.gfp mice in which GFP is upregulated by nuclear factor of activated T cells (NFAT) activation downstream of TCR signalling, essentially all IELs that upregulated CD25 were GFP+ (FIG. 11B). This phenotype was not shown by Vγ7- or TCRαβ+ IELs in the same co-cultures (FIG. 11B). IEL upregulating CD25 also downregulated CD122, an additional symptom of systemic TCR stimulation (FIG. 12C). Thus, CD25+GFP+CD122-cells arose uniquely among Vy7+ IEL co-cultured with L1+6 cells (FIG. 11C). Moreover, CD25 upregulation was accompanied by slight but significant TCR downregulation, another rapid response to TCR stimulation (FIG. 11D). Btnl1+Btnl6 acted directly on Vy7+ IEL, since CD25 was upregulated on cells that had been purified by flow cytometry prior to L1+6 cell coculture (FIG. 12D). Note that background CD25 expression was increased by TCR-dependent sorting, but this did not obscure the result.

When IEL were separated from L1+6 cells by transwells, CD25 upregulation was abrogated and could not be secondarily transactivated by IELs that were in contact with L1+6 cells, e.g., via secreted cytokines (FIG. 11E). CD25 upregulation by IELs in contact with L1+6 cells showed dose-dependent inhibition by PP2, which inhibits signalling by src-family kinases, such as Lck and Fyn, but was not inhibited by PP3, an established control for PP2 specificity (FIG. 12E).

Just as residual Vy7+ IEL in Btnl1-/- mice responded to acute transgenic Btnl1 induction in vivo, they were comparable to WT Vy7+ IELs in responding to Btnl1 plus Btnl6 ex vivo (FIG. 11F). In the same experiments, WT Vy7+ IELs showed relatively poor responses to anti-CD3, phenocopying the attenuated responsiveness imposed on Vy5+ DETC progenitors by Skint1, whereas Vy7+ IELs from Btnl1-/- mice, and TCRαβ+ and Vγ7- IELs from WT mice, none of which subsets had experienced prior Btnl1 selection in vivo, all showed strong responses to anti-CD3 (FIGS. 11F and 11G).

Finally, the supernatants of IEL co-cultures with L1+6 cells showed small but significant increases in interferon-y (IFN-γ), CCL4, and granulocyte macrophage colony-stimulating factor (GM-CSF) among 36 cytokines tested (FIG. 11H). These are typical IEL effectors, and although it was technically challenging to attribute production to Vy7+ IELs, such increases were not seen in supernatants of L1+6 cells cultured with IELs from TCRδ-/- mice. Note the higher background cytokine expression in TCRδ-/- mice may reflect spontaneous inflammation often associated with γδ deficiency. In sum, a range of metrics attested to a highly specific and direct interaction ex vivo of Vy7+ IELs with Btnl1 and Btnl6 co-expressed on gut epithelial cells.

### Example 7. Signature human intestinal γδ T cells

Because of limited tissue access, human gut T cells are understudied. Nonetheless, there are gutassociated γδ cells whose TCR usage differs markedly from Vγ9+Vδ2+ cells that dominate the peripheral blood. To better characterize such cells, we submitted biopsy specimens from healthy ascending colon to a modified version of a protocol used to isolate human skin T cells (Clark, et al., Journal of Investigational Dermatology. 2006. 126(5):1059-70, incorporated by reference herein). For 16 of 17 donors, the γδ cells were enriched in Vδ1+ cells, although Vδ1-Vδ2- cells were also present; hence, the term "Vδ2-" is used to distinguish tissue-associated γδ cells from Vδ2+ cells that could also be recovered from most gut samples, albeit in highly variable numbers (FIG. 13A).

Of six functional human Vγ chain genes (Vγ2, 3, 4, 5, 8, and 9), Vγ4 was reported to be the signature chain of intestinal Vδ2- cells. Indeed, for up to ten donors examined, most intestinal Vδ2- cells reacted with a Vy2/3/4-specific antibody, but not a Vγ5/3-specific antibody (FIG. 13B; Table 1), and TCR deep sequencing showed that Vγ4 sequences far outnumbered Vγ2 sequences (FIG. 14A). Thus, despite individual variation, most gut γδ T cell compartments included a substantial Vγ4+Vδ2- subset, while some donors also displayed relatively high representation of Vγ8+Vδ1+ cells (FIG. 13B; Table 1).

**Table 1: Reactivity of Vδ1, Vδ2, and Vδ1- and Vδ2- intestinal cells with Vγ antibodies**

| | Vδ1 | Vδ2 | Vδ1- Vδ2- |
|---|---|---|---|
| Vγ2/3/4 (n=10) % of | 60.9 (19.5) | 2.5 (3.1) | 64.7 (18.7) |
| Vγ3/5 (n=6) % of | 13.1 (10.9) | 1.4 (1.4) | 24.5 (25.8) |
| Vγ8 (n=7) % of | 37.7 (28.0) | 0.9 (1.0) | 14.4 (12.0) |
| Vγ9 (n=7) % of | 9.3 (16.0) | 88.5 (17.1) | 9.3 (11.2) |

### Example 8. BTNL3 and BTNL8 regulate human Vy4+ cells

There is no human equivalent of the Btnl2-proximal amplicon on mouse chromosome 17 that encodes Btnl1, Btnl4, and Btnl6. However, adjacent to human BTNL9 is an amplicon that encodes BTNL3 and BTNL8 whose expression is highly enriched in gut, particularly EpCAM+ epithelial cells (FIGS. 14B-14D). Interestingly, akin to the behavior of Btnl1 and Btnl6 described above, neither BTNL3 nor BTNL8 protein was efficiently expressed on cells transfected with their respective genes, unless both were co-expressed (FIG. 14E). Conversely, BTNL8S (a splice variant of BTNL8) failed to rescue surface BTNL3 expression (FIG. 14E).

Whereas we could not test for a developmental dependence of human gut γδ cells on BTNL3 and BTNL8, we could assess whether BTNL3 and BTNL8 phenocopied Btnl1 and Btnl6 by specifically activating signature gut γδ cells in a TCR-dependent fashion. Thus, we established short-term co- cultures of primary-gut-derived lymphocytes with HEK293T cells transduced with BTNL3 (L3), BTNL8 (L8), BTNL3 and BTNL8 (L3+8), or EV (FIG. 14F). For the representative donor shown, some of the discrete subsets of Vδ1- and Vδ1+ γδ cells that were apparent in T cell co-cultures with control (EV, L3, and L8) cells showed marked TCR downregulation when co-cultured with L3+8 cells (arrows FIG. 13C).

Emphasizing specificity, TCR downregulation occurred in response to L3+8 cells in 21 of 23 donors but was never seen in co-cultures with L3 or L8 cells, and was never shown by intestinal Vδ2+ or TCRαβ+ cells, even in the same cultures as responding Vδ2- cells (FIG. 13D). Although higher baseline CD25 expression reduced the sensitivity of this assay for human versus mouse gut T cell activation ex vivo, L3+8 cells induced significant CD25 upregulation vis-a-vis gut T cells co-cultured with control cells (FIG. 13E), and CD25 upregulation was most evident on cells with downregulated TCRs (FIG. 14G).

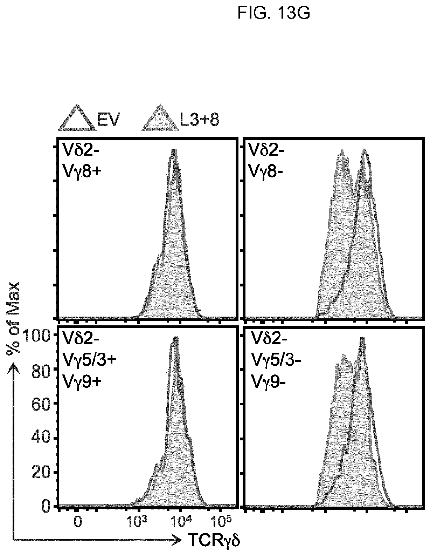

Not all Vδ2- cells responded to L3+8 (FIG. 13C). Thus, we considered that TCRγ chains might determine BTNL responsiveness, as is true in mice. Indeed, human Vδ2- populations that downregulated TCRs in co-cultures with L3+8 cells were detected by the Vy2/3/4-specific antibody, but not by antibodies to Vy8, Vy5/3, or Vγ9 (FIGS. 13F and 13G). Moreover, productively rearranged Vγ4 genes were prevalent when L3+8-responsive cells with downregulated TCRs were flow cytometry sorted from one donor (FIG. 14H) and their Vγ chains amplified without bias and sequenced (top four sequences, FIG. 15). By contrast, TCRγ transcripts from skin-derived TCRγδ+ cells (G234SK01) were biased toward Vγ3 (bottom four sequences, FIG. 15). Interestingly, of two donors showing no substantial response to BTNL3+8, one proved a posteriori to have an atypical intestinal γδ T cell repertoire dominated by Vγ8+ cells (FIG. 14I). Likewise, L3+8 cells induced no significant TCR down-modulation by primary γδ+ T cells from skin or blood among which Vγ4+ cells are rare (FIG. 13H). Thus, epithelial BTNL genes regulate human-tissue-resident γδ T cells in an organ-specific, TCRγ chain-specific manner.

### Example 9. Experimental methods

The following methods were performed as necessary to carry out the studies described in Examples 1-8.

### Mice

Wild-type (WT) C57BI/6 mice were obtained from Charles River and Harlan. Three independently derived embryonic stem (e.s.) cells for Btnl1-/- (Btnl1tm1(KOMP)Mbp) and e.s. cells for Btnl4-/- (Btnl4tm1(KOMP)Mbp) mice were obtained from the international mouse phenotyping consortium (IMPC) (project IDs: CSD67994 and CSD81524). Btnl1indel/indel mice were generated using CrisprCas Technology. Briefly, two independent short guide RNAs, targeting the intronic region between exon 1 and 2 and between exon 5 and 6 were identified using the online tool: crispr.mit.edu/ (CRISPR design platform, Broad Institute MA USA). Intron1/2: CCAGCTCCAAGATCCCCCTTGGG (SEQ ID NO: 13) Intron5/6: TCCATAGCACCTTATCCGGTTGG (SEQ ID NO: 14). The sg RNAs & PAM sequences were cloned into the g-RNA basic vector, translated in vitro, purified and co-injected with Cas9 into day 1 zygotes and transferred into pseudopregnant foster mice. WT and Btnl-knockout lines were generated and maintained at The Francis Crick Institute's Biological resource facilities. For timed pregnancies, mice were mated overnight and E0 was considered as the day a vaginal plug is observed. Both male and female mice aged between 1 and 35 weeks (as indicated) were used in this study. No gender-specific differences were observed.

### Germ-free mice and food antigen-free nutrition

Germ-free (GF) mice were kept in plastic isolators with autoclaved food, bedding, and water. Sterility of animals was checked bi-weekly by culturing faeces in thioglycollate medium under aerobic and anaerobic conditions for at least ten days. All handling procedures for GF mice were conducted in a laminar flow hood under sterile conditions. Food antigen-free (FAF) mice were raised on an amino acid-containing diet for up to five generations. Pellets of FAF diet (ssniff, S7242-E014/-E714) contained all essential vitamins, minerals, trace elements, fat, dextrin, sucrose and free amino acids equimolar to the protein content of normal rodent chow (LASQCdietRod16, LASvendi).

### Generation of doxycyclin inducible Btnl-1 transgenic (Tg) mice

Doxycycline (Dox)-inducible Btnl1-Tg mice were generated by injection of Btnl1-/- blastocysts with a linearized cassette containing a TRE/CMV-promoter upstream of the Btnl1-ORF. The TRE/CMV cassette has been previously described (Oppenheim et al., 2005). R26-rtTA2-M2 (Hochedlinger et al., 2005) or Villin-rtTA2-M2 (Roth et al., 2009) mice were bred to homozygosity for Btnl1-deficiency and backcrossed onto Btnl-Tg mice for 3 generations to facilitate global (R26) or local (Villin) induction of Btnl1 transgene expression by doxycycline administered to drinking water (1 mg/ml Dox, 2% sucrose). Animal experiments were undertaken in full compliance with UK Home Office regulations and under a project license to A.H. (80/2480).

### Flow Cytometry

Flow cytometry was performed using the following antibodies, coupled to the indicated fluorochromes. Antibodies for mouse: CD3 APC Cy7 (17A2); CD3 PerCPCy5.5 (145-2C11); TCRβ Brilliant Violet 421 (H57-597); TCRβ APC (H57-597); CD122 PE (TMβ1); CD122 Brilliant Violet 421 (TMβ1); CD122 APC (TMβ1); TIGIT PE (GIGD7); CD45RB APC Cy7 (C363-16A); Thy1.2 Brilliant Violet 510 (53-2.1); Lag3 PerCP-efluor 710 (C9B7W); CD5 PE (53-7.3); CD24 FITC (M1/69); CD24 PECy7 (M1/69); CD8α PECy7 (53-6.7); CD8α PECy7 (53-6.7); TCR Vδ4 FITC (GL-2); TCR Vδ4 PE (GL-2); CD8β PerCpCy5.5 (YTS156.7.7); CD25 PerCpCy5.5 (PC61); CD69 PECy7 (H1.2F3); CCR9 PECy7 (CW-1.2); CD44 PECy7 (IM7); TCRVγ7 (F2.67) was provided by Pablo Pereira (Institut Pasteur, Paris, France); TCRVγ1 APC (2.11); TCRVy4 APC (UC3-10A6); TCRδ BV421 (GL3); Ki67 FITC (B56/MOPC-21); CD45 Qdot 605 (30-35 F11); CD5 Brilliant Violet 510 (53-7.3); TCRδ PeCy7 (GL3); CD161/NK1.1 Brilliant Violet 650 (PK136); CD4 Brilliant Violet 786 (GK1.5); CD8α AlexaFluor 700 (53-6.7); CD25 APC (PC61); GITR PE (DTA-1); CD44 FITC (IM7); CD62L PerCP-Cy5.5 (MEL-14); KLRG1 BV421 (2F1); CD11c BV786 (HL3); CD11b BV510 (M1/70); F4/80 PerCPCy5.5 (BM8); Ly6G APC (1A8); Ly6C AlexaFluor 700 (AL-21); CD103 PE (M290); CD317 Brilliant Violet 650 (927); MHCII/IA/IE FITC (2G9); CD86 Pe-Cy7 (GL1); CD3 Brilliant Violet 421 (145-2C11); CD19 Brilliant Violet 421 (1D3); CD161/NK1.1 (lin) Brilliant Violet 421 (PK136); IgG1 PE (A85-1); B220 (CD45R) AlexaFluor 700 (RA3-6B2); IgM Brilliant Violet 786 (R6-60.2); IgD PerCPCy5.5 (11-26c.2a); GL-7 AlexaFluor 647 (GL7); CD95 PECy7 (Jo2); CD138 Brilliant Violet 650 (281-2); CD21/35 FITC (7G6); CD23 Brilliant Violet 421 (B-ly6).

Antibodies for human: CD25 Brilliant Violet^{™} 421 (BC96); CD25 PE (BC96); CD3 Brilliant Violet^{™} 510 (OKT3); CD3 BUV (UCHT 1); EpCAM eFlour^{®} 660 (1B7); Streptavidin APC-Cy7; Streptavidin Brilliant Violet^{™} 421; TCRγδ PeCy7 (IMMU510); Vγ9 PC5 (IMMU360); Vγ9 PE (B3); Vδ1 APC (REA173); Vδ2 PerCP (B6); Vγ2/3/4 biotin (23D12), Vy3/5 biotin (56.3) and Vγ8 biotin (R4.5.1) were provided by D. Kabelitz and D. Wesch (University of Kiel).

Other antibodies: DYKDDDDK-PE (Flag); DYKDDDDK-APC (Flag); HA-DyLight 650; 6x- Histidine-PE. Commercial antibodies were purchased from Biolegend, eBioscience, BD-Bioscience, Thermo Fisher Scientific or Miltenyi. Viability dyes (near IR or Blue) were from Invitrogen. Anti TCRVγ7 (F2.67) was purified from hybridoma supernatant using the mouse TCS purification system (Abcam) and conjugated to biotin or AF647.

Ki-67 staining was performed on cells fixed and permeabilized using the Foxp3 staining buffer set (eBioscience). BrdU (Sigma-Aldrich) and EdU incorporation was assessed 3h post-intraperitoneal injection (50mg/kg) by immunohistochemistry or by flow cytometry (Click-iT EdU AF647 Assay Kit, Invitrogen), respectively. Anti-TCRVγ7 (F2.67) was purified from hybridoma supernatant using the mouse TCS purification system (abcam-ab128749). Purified anti-TCRVγ7 was conjugated to biotin (EZ-Link Sulfo-NHS-LC Biotinylation Kit, Thermo Fisher Scientific) or to AF647 (labelling kit, Thermo Fisher Scientific). Other antibodies were anti-human Vγ2/3/4 (23D12, biotinylated), Vy5/3 (56.3, biotinylated) and Vγ8 (R4.5.1, biotinylated). Flow cytometry data analysis was performed on FlowJo (Version 9.9)

### Plasmids, cloning, RT-PCR, transfection and lentiviral transduction

The self-inactivating lentiviral vector pCSIGPW (SFFV promoter - Multiple Cloning Site [MCS] - IRES-GFP - CMV promoter - PuromycinR) was constructed by replacing the PuromycinR/mIR cassette from the pAPM vector (Pertel et al., 2011) by a custom EcoRI-Xhol-Pmel-Notl-BamHI-Xbal-Mlul MCS. The IRES-GFP cassette was cloned by PCR from the pIRES2-eGFP vector (Clonetech) using the BamHI/Xbal sites. The CMV promoter was cloned by PCR from the pCDNA3.1+ vector (Thermo Fischer Scientific) using the Mlul/Clal sites. The Puromycin resistance gene was cloned by PCR from the pGIPZ vector (Dharmacon) using the Clal/Agel sites. The pCSIGHW variant was generated by exchanging the puromycin resistance gene with a hygromycin B resistance gene, which was cloned by PCR from the pLHCX vector (Clontech).

cDNAs were (sub-)cloned into pCSIGPW or variant vectors. Btnl1, Btnl4 and Btnl6 were previously described (Bas et al., 2011). BTNL3, BTNL8S and BTNL8 (GenBank accession numbers NM_197975.2, NM_024850.2 and NM_001040462.2) were cloned from Caco-2 cells by conventional RT- PCR, using the following primers:

| | |
|---|---|
| BTNL3 For | 5'-GAATATCCATGGCTTTTGTGC-3' (SEQ ID NO: 15) |
| BTNL3 Rev | 5'-GTCTTCTCTGTCTCATCCCC-3' (SEQ ID NO: 16) |
| BTNL8 For | 5'-CCATTCACAGAACACATCCATG-3' (SEQ ID NO: 17) |
| BTNL8S Rev | 5'-TATGGGTTACAGTTTTCAGATCAG-3' (SEQ ID NO: 18) |
| BTNL8 Rev | 5'-GTGGGATGTGATTCATCCTAC-3' (SEQ ID NO: 19) |

FLAG, HA and HIS tags were added downstream of the putative leader peptides by overlapping PCR. Human full-length TCR γ and δ chains were cloned (Xhol / Notl, pCSIGPW) using the following primers:

| | |
|---|---|
| Vγ2/3/4 For | 5'-ATGCAGTGGGCCCTAGCG-3' (SEQ ID NO: 20) |
| Vγ8 For | 5'-ATGCTGTTGGCTCTAGCTCTGCTTC-3' (SEQ ID NO: 21) |
| Vγ9 For | 5'-ATGCTGTCACTGCTCCACACATC-3' (SEQ ID NO: 22) |
| Cy1/2 Rev | 5'-TTATGATTTCTCTCCATTGCAGCAG-3' (SEQ ID NO: 23) |
| Vδ1 For | 5'-ATGCTGTTCTCCAGCCTGCTG-3' (SEQ ID NO: 24) |
| Vδ2 For | 5'-ATGCAGAGGATCTCCTCCCTCAT-3' (SEQ ID NO: 25) |
| Vδ3 For | 5'-ATGATTCTTACTGTGGGCTTTAGCTTTTTG-3' (SEQ ID NO: 26) |
| Cδ Rev | 5'-TTACAAGAAAAATAACTTGGCAGTCAAGAG-3' (SEQ ID NO: 27) |

Expression of BTNL3 and BTNL8 was checked by conventional RT-PCR using the primers indicated above. BTN3A1, BTN3A2, EPCAM and GAPDH were used as control genes.

| | |
|---|---|
| BTN3A1 For | 5'-AGTATCTCCTGATATGCAGCATG-3' (SEQ ID NO: 28) |
| BTN3A1 Rev | 5'-GGAGGAACTCTCTTCTTCTTTTCAC-3' (SEQ ID NO: 29) |
| BTN3A2 For | 5'-TGGTATCTCTTGATATGCAGCATAG-3' (SEQ ID NO: 30) |
| BTN3A2 Rev | 5'-AGAGCATCAGGCTGACTTATTGG-3' (SEQ ID NO: 31) |
| EPCAM For | 5'-GCCGCCACCATGGCGCCCCCGCAG-3' (SEQ ID NO: 32) |
| EPCAM Rev | 5'-TTATGCATTGAGTTCCCTATGCA-3' (SEQ ID NO: 33) |
| GAPDH For | 5'-GAAGGTGAAGGTCGGAGTC-3' (SEQ ID NO: 34) |
| GAPDH Rev | 5'-GAAGATGGTGATGGGATTTC-3' (SEQ ID NO: 35) |

Transfections were carried out in HEK293T cells using PEI (3:1 PEI:DNA ratio, Polysciences). Btnl/BTNL expression was checked 48h post-transfection. Lentiviral particles were produced in HEK293T cells by co-transfection of pCSIGPW or pCSIGHW either empty or containing Btnl/BTNL cDNAs, pCMVΔR8.91 (HIV-1 tat/rev/gag/pol), and pHIT/G (MLV env). Transduced cells were treated with puromycin and hygromycin 48h post-transduction for 7 days, sorted on the basis of GFP expression and used for functional assays.

### Quantitative RT-PCR

Samples were stored in RNAlater (Ambion) or directly frozen in RLT buffer prior to RNA purification (Qiagen RNeasy kit). cDNA was generated using Superscript-II (Invitrogen) and analysed using Sybrgreen assay (Invitrogen) using a ViiA7 Real-time PCR machine (Applied Biosystems).

### Primers for murine qPCR:

| | |
|---|---|
| Btnl1 For: | 5'-TGACCAGGAGAAATCGAAGG-3' (SEQ ID NO: 36) |
| Btnl1 Rev: | 5'-CACCGAGCAGGACCAATAGT-3' (SEQ ID NO: 37) |
| Btnl4 For: | 5'-CATTCTCCTCAGAGACCCACACTA-3' (SEQ ID NO: 38) |
| Btnl4 Rev: | 5'-GAGAGGCCTGAGGGAAGAA-3' (SEQ ID NO: 39) |
| BTNL6 For: | 5'-GCACCTCTCTGGTGAAGGAG-3' (SEQ ID NO: 40) |
| Btnl6 Rev: | 5'-ACCGTCTTCTGGACCTTTGA-3' (SEQ ID NO: 41) |
| β-Actin For: | 5'-CAGCTTCTTTGCAGCTCCTT-3' (SEQ ID NO: 42) |
| β-Actin Rev: | 5'-CACGATGGAGGGGAATACAG-3' (SEQ ID NO: 43) |
| Sox-13 For: | 5'-CTCCAGGCCTTCCCAGAC-3' (SEQ ID NO: 44) |
| Sox-13 Rev: | 5'-CATGGACTTCCAGCGAGAAC-3' (SEQ ID NO: 45) |
| Rorγc For: | 5'-GGTGACCAGCTACCAGAGGA-3' (SEQ ID NO: 46) |
| Rorγc Rev: | 5'-CCACATACTGAATGGCCTCA-3' (SEQ ID NO: 47) |
| Tbp For: | 5'-GGGGAGCTGTGATGTGAAGT-3' (SEQ ID NO: 48) |
| Tbp Rev: | 5'-CCAGGAAATAATTCTGGCTCA-3' (SEQ ID NO: 49) |
| Cyclo For: | 5'-CAAATGCTGGACCAAACACAA-3' (SEQ ID NO: 50) |
| Cyclo Rev: | 5'-CCATCCAGCCATTCAGTCTTG-3' (SEQ ID NO: 51) |

### Southern Blotting

Southern blots were performed with probes generated using a Dig-Probe labelling kit; blots were hybridized in DIG-Easy-hyb buffer overnight, and developed using the DIG-Luminescence Detection Kit (Sigma-Aldrich). DIG labelled probes for Southern blotting were generated using the following primers:

| | |
|---|---|
| Btnl1 For: | 5'-ACTGGCTTCCTCAGAGTCAT-3' (SEQ ID NO: 52) |
| Btnl1 Rev: | 5'-CAGTAGTGAATGGCCCCTGA-3' (SEQ ID NO: 53) |
| Btnl4 For: | 5'-GACCAACGCTTCCCTACCTC-3' (SEQ ID NO: 54) |
| Btnl4 Rev: | 5'-GCCTTGGGTCCAACAAGACA-3' (SEQ ID NO: 55) |
| Btnl1-Tg-Ex3-For: | 5'-GGTTTTCTGTGAAGGGACCA-3' (SEQ ID NO: 56) |
| Btnl1-Tg-Ex4-Rev: | 5'-GGTCTGCAACTCAGAGGAGG-3' (SEQ ID NO: 57) |

### RNAscope

RNAscope was performed on paraffin embedded sections using probes and kits obtained from Advanced Cell Diagnostics Inc. using the RNAscope 2.0 HD Reagent Kit-BROWN. Reference sequences are as follows: Btnl1.NM_001111094.1 (576-1723); Btnl4 NM030746.1 (560-968); Btnl6.NM_030747.1 (245-1552).

### Isolation of murine intestinal intra-epithelial lymphocytes (IELs)

IELs were isolated from mouse small intestine as described in Wencker et al., Nat. Immunol. 2014, 15: 80-87, which is incorporated herein by reference in its entirety. Small intestine was opened and washed in PBS, cut into 1 cm pieces and incubated for 20min in RPMI 1640 supplemented with 1% penicillin/streptomycin (pen/strep), 10% fetal calf serum (FCS) and 1mM dithiothreitol on a turning wheel. Tissues were washed and vortexed in RPMI, then passed through a 70µm nylon cell strainer twice, and centrifuged on a 20/40/80% Percoll density gradient at 700g for 30min. IELs were harvested from the 40 to 80% Percoll interface.

### MODE-K co-culture assays

Cells were co-cultured in in RPMI 1640 supplemented with 10% FCS, Pen/Strep, 2.5% HEPES, 1% Glutamine, 1% non-essential amino acids, 1% sodium pyruvate, 0.2% β-mercapto-ethanol (Gibco) and cytokines including IL-2 (10 U/ml), IL-15 (10 ng/ml) (Immunotools), IL-3 (100 U/ml), IL-4 (200 U/ml) (R&D). 105 MODE-K were seeded in 48-well plates 24 hours prior to the addition of 105 unsorted or (where indicated) positively FACS-sorted (CD45+Vy7+) IELs and incubated for 16-18 hours in 10% CO2 unless indicated otherwise. For transwell assays, 2 × 105 MODE-K cells were seeded onto 24-well transwell plates (3 µm pore size - Corning) 24 hours prior to the addition of 3 × 105 IELs, either in direct contact (below), sequestered from (above), or split 50:50 with MODE-K cells (above and below the transwell).

### IEL Stimulation

96-well U bottom plates were coated overnight with 10 µg/ml LEAF-Purified anti-mouse CD3ε or Hamster IgG Isotype control (Biolegend) at 4oC and washed once with PBS 1x before seeding IEL.100,000 IELs were seeded per well. Cells were incubated at 37oC for 16-18 hours in 10% CO2 prior to analysis.

### Confocal Imaging

Proximal small intestine (SI) samples were fixed in Zamboni's fixative, blocked with normal goat serum and stained with antibodies against TCRβ, TCRδ, TCRVδ4 (encoded by TRDV2-2) (GL2), CD3 and Vγ7. Z-Sections were acquired on a confocal-LSM-710 microscope (Zeiss) and processed and analysed using Imaris Software (Bitplane Scientific Solutions).

### Bone Marrow Chimeras and Adoptive IEL Transfers

10-12 week old recipient mice were irradiated with 950Rads 24 hours, injected (IV) with 5-10x106 donor bone marrow cells and analysed 4-12 weeks later. IELs harvested from 4 week-old WT mice were column-purified using CD45 microbeads (MACS Miltenyi biotec) and IV-injected into 6 week-old TCRδ-/- and TCRδ-/-Btnl1-/- recipients. Analysis was performed 2-3 weeks later.

### RNA sequencing

Vy7+CD122hi and Vγ7+CD122lo IEL were sorted from pooled D14-17 pups directly into RLT buffer. RNA was prepared using the RNA-Micro-plus kit (Qiagen). RNA libraries were generated using the KAPA Stranded RNA-seq Kit with RiboErase (HMR) (KAPA BIOSYSTEMS). Paired-end sequencing on HiSeq 2500 (illumina) using rapid run chemistry (read length: 100bp).

### Human Samples and Primary Lymphocyte Isolation

Endoscopic biopsies were obtained from the ascending colon of adult donors undergoing routine diagnostic colonoscopy. Excess resected skin discarded at the time of cutaneous or reconstructive surgery was obtained from adult donors. Primary gut lymphocytes were obtained using an adaptation of the method of Kupper and Clarke (Clark, et al., Journal of Investigational Dermatology. 2006. 126(5):1059-70; FIG. 14F). Skin lymphocytes were isolated using the method as originally described. 9mm × 9mm × 1.5mm Cellfoam matrices (Cytomatrix PTY Ltd), were autoclaved and incubated in 100mg/mL rat tail collagen I (BD Biosciences) in PBS for 30min at 37°C, and washed twice in PBS. In compliance with local ethical approval, 12 endoscopic biopsies were taken from the ascending colon of donors. Biopsies were washed for 20min in 5mL wash medium (RPMI 1640 10% FCS, β-mercaptoethanol, penicillin [500 U/ml], streptomycin [500 µg/ml], metronidazole [5 µg/ml, Pharmacy department, Guy's Hospital], gentamicin [100µg/ml, Sigma- Aldrich] and amphotericin 12.5 µg/ml [Thermo Fisher Scientific]). One endoscopic biopsy was placed on top of each matrix, which was inverted, and pressure applied, to crush the biopsy into the matrix. The matrices were placed into a 24-well plate (1 per well) and covered with 2 mL RPMI 1640 (supplemented with 10% FCS, β-mercaptoethanol, penicillin [100 U/ml], streptomycin [100 µg/ml], metronidazole [1µg/ml], gentamicin [20 µg/ml], amphotericin [2.5 µg/ml]), IL-2 (100 U/mL, Novartis Pharmaceutical UK) and IL-15 (10 ng/mL, Biolegend). 1 ml of medium was aspirated every second day and replaced with complete medium containing 2x concentrated cytokines. Cells were harvested and residual biopsy and empty wells were washed with PBS 0.02 mM Hepes. The cell suspension was passed through a 70 µm nylon cell strainer, centrifuged at 400 g for 5 min and resuspended in complete medium without additional cytokine and placed into co-culture immediately. Lymphocytes were used after 5-7 days of culture. PBMCs were isolated by Ficoll gradient from blood obtained from the blood donation service.

### Human epithelial cell isolation

Colonic samples were incubated with 5 mM 1,4-dithiothreitol (Sigma), followed by enzymatic digestion with 1.5 mg/ml collagenase VIII (Sigma) and 0.05 mg/mL DNase I (Sigma). EpCAM+ cells were sorted by flow cytometry directly into RLT lysis buffer. RNA and cDNA were prepared as described above.

### HEK293T co-culture Assay

5x105 HEK293T cells, transduced with either empty vector (EV), BTNL3, BTNL8 or BTNL3+8 and 2x105 freshly harvested primary human lymphocytes were co-cultured in 96-well plates with complete medium without supplementary cytokine and incubated at 37°C at 5% CO2 for 16 hrs (FIG. 14F).

### Deep sequencing

Mouse TRDV gene: Amplification and sequencing of TCRδ CDR3 from RNA purified from sorted Vγ7+ IEL was performed using the Amp2Seq Platform (iRepertoire).Human TCRG Vγ gene: Amplification and sequencing of TCRγ CDR3 was performed using the immunoSEQ Platform (Adaptive Biotechnologies).

### Statistics

Unless stated otherwise, bar/spider charts display mean ±SD and p values were derived from unpaired two tailed t tests, assuming equivalent SD (ns>0.05).

### Imaris Image analysis

Confocal microscopy was performed using a LSM710 laser scanning confocal microscope (Zeiss) with a 40x oil objective (numerical aperture 1.3). 3D image analysis on z-stacks was carried out using Imaris (Bitplane). The surfaces tool was used to identify CD3+ cells. Voxels outside of these structures were set to zero in each of the channels to create masks.

### Bioinformatics analysis of RNA sequencing

101 base-pair paired-end reads were aligned and quantified using RSEM (v1.2.11) (Li and Dewey, 2011) with Bowtie2. Reads were aligned to a transcriptome constructed from the mm10 mouse genome and a UCSC known Gene gtf file. A mean alignment rate of 57.4 million fragments per sample was observed. Using the gene level quantification, only detected genes (mean TPM value across all samples > 1; 13,313 genes) were selected. Differential expression between the CD122hi and CD122lo Vγ7+ IEL groups using DESeq2 was identified by taking into account the paired structure within the replicate groups. Using an FDR of 0.01 2664 phenotype dependent gene expression effects were identified.

### Data Resources

RNA sequencing data: GEO Accession number - GSE85422.

### Example 10. Characterizing the role of BTNL3/BTNL8 on vδ cells in disease

The surface expression of TCR γδ (FIG. 16A, left three panels) or TCR.CD3 (FIG. 16A, right-most panel) was analysed for T cell subtypes indicated in each panel, following overnight human gut T cell co-cultures with 293 cells transduced with empty vector (EV; blue histograms) or 293 cells transduced with a vector expressing BTNL3+BTNL8 (red histograms). For each subtype the TCR/CD3 expression in the two co-culture situations essentially overlay, whereas for γδ cells expressing Vγ2, Vγ3, or Vγ4 (which are indistinguishable with the detection reagent used), TCRγδ expression by cells exposed to BTNL3 and BTNL8 (red histogram) was shifted to the left (down-regulated) relative to that expressed by cells exposed to EV (blue). TCR expression was quantified for 7 donors (FIG. 16B, left hand panel). Downregulation in TCRVγ2/3/4 cells is equivalent to that in all TCRVγ9(-) cells, and thus accounts for the complete down-regulation observed (FIG. 16B, second panel). Donor γδ and TCRVγ2/3/4 frequencies in these donors was characterized (FIG. 16B, right two panels).

A strategy to detect the common haplotype encoding BTNL8 and BTNL3 from the rarer haplotype encoding BTNL8*3 is illustrated in FIGS. 17A and 17B. Genotype-specific primers (L8R and L3R) were used in conjunction with a common forward primer (L8F) to generate a PCT product of about 1.3 kb. Results are shown in FIG. 18. About 46% of mutations were heterozygous, and about 8% were homozygous. Expression of transduced proteins was detected using FLAG-tagged proteins as shown in FIG.19. BTNL3 is poorly expressed at the cell surface unless it is co-expressed with either BTNL8 or BTNL8S179F, an allelic variant of BTNL8. However, coexpression with BTNL8*3 did not enhance its surface expression. Conversely, BTNL8*3 did enhance the cell surface expression of BTNL8 and BTNL8S179F.

FIG. 20 shows that following exposure of BTNL3+BTNL8 and BTNL3+BTNL8S179F, TCRγδ expression and TCRVγ2/3/4 expression are coordinately reduced, but not in other circumstances. Flow cytometry reveals that a homozygote patient diagnosed with IBD exhibited a very small γδ T cell compartment, of which the majority is Vδ1+. Similarly, in a homozygote patient having a family history of ulcerative colitis, the number of γδ T cells is also very low, and more than 85% of γδ T cells do not express TCRVγ2/3/4 (FIG. 21).

Two heterozygote patient samples were analysed and are shown in FIG. 22. Whereas patient GN017 expressed primarily TCRVγ2/3/4 and Vδ1, Vδ3, Vγ9, and Vδ2, patient GN019 expressed more diverse genes with readily detectable signals for Vγ8 and Vδ5, in addition to those detected in GN017.

Consistent with this, when TCRγ chain cDNAs were cloned from gut samples of the two donors, 31/31 sequences for GN017 were Vy4-Jy1, whereas for GN019, the γ chain sequences were much more diverse, with only 6/33 encoding Vγ4-Jγ1. Of note, GN017 was a "responder" whose γδ cells downregulated their TCRs on exposure to BTNL3+BTNL8, whereas GN019 was a non-responder. The effect on indicated SNPs on BTNL3 and BTNL8 expression are shown in FIGS. 23A and 23B.

Cells heterologously expressing Vγ4 were also found to be specifically reactive to BTNL3 and BTNL8. J76 cells, Jurkat cells that do not endogenously express any TCR, were transduced with vectors encoding either Vγ4Vδ1or Vγ9Vδ2, then cocultured with HEK293T cells expressing either empty vector, BTNL3 only, or BTNL3 and BTNL8. FIGS. 24A and 24B show that the TCR is downregulated and CD69 is upregulated specifically for J76 Vγ4Vδ1 cells exposed specifically to BTNL3+BTNL8 or PMA+ ionomycin. Conversely, J76 Vγ9Vδ2 cells showed TCR downregulation and CD69 upregulation only upon exposure to PMA-ionomycin. For the first time, heterologous expression of Vγ4 on a cell was shown to be sufficient to bind and respond to BTNL3+BTNL8.

In Caco2 polarization experiments shown in FIGS. 25A-25C, BTNL3 expression was notably only substantially expressed only in polarized Caco2 cells (FIGS. 25B and 25C). HNF4a expression and IL-8 expression were also both upregulated in polarized Caco2 cells (FIG. 25C). BTNL8 expression was upregulated in confluent/quiescent Caco2 cells, irrespective of polarization.

Studies on the effect of stress (e.g., stress mimicking the microenvironment of an inflamed gut, such as presence of TNFα and free radicals (e.g. H2O2)), revealed that TNFα reduced the expression of BTNL3, BTNL8, HNF4, and CDX2, while IL-8 was upregulated (FIG. 26A). Kinetic assays revealed that electrical resistance was lost over time in the presence of TNFα, relative to untreated controls (FIG. 26B).

### Example 11. Administration of a population of Vy4+ T cells to treat IBP

According to the methods disclosed herein, a physician of skill in the art can treat a patient, such as a human patient, so as to reduce or alleviate the symptoms of IBD. To this end, a physician of skill in the art administers to the human patient a population of Vγ4+ T cells in which 5% or more (e.g., 5%, 10%, 15%, 20%, 25%. 30%, 40%, 50%, 60%, 70%, 80%, 90%, or more) of the cells are Vγ4δ1+ or Vγ4δ3+ T cells. 10⁶ Vγ4+ T cells are administered to the patient by intravenous administration on a bimonthly frequency. The patient is evaluated, for instance, 2 weeks, 1 month, 2 months, 3 months, 4 months, 5 months, 6 months or more following administration of the population of IELs depending on the route of administration used for treatment. A finding of a reduction of one or more IBD symptoms following administration of a population of Vy4+ T cells provides an indication that the treatment has successfully treated IBD.

### Example 12. Generation of a vδ T cell expressing Vγ4δ1

Non-Vγ4δ1 γδ T cells, such as Vγ9δ2 cells, can be isolated from a patient sample and transduced to express Vγ4δ1. In an exemplary method for making Vγ4δ1+ γδ T cells, viral vectors (e.g., a lentiviral vector, adenoviral vector, or adeno-associated viral vector) containing a constitutively active promoter (e.g., any constitutively active known in the art, such as a human retroviral LTR, SFFV, EIF1α, or PGK) and the nucleic acid sequences encoding Vδ1 and Vγ4 are engineered using standard techniques known in the art. To express both Vδ1 and Vγ4, a bicistronic expression cassette is used in which an IRES sequence is placed between the nucleic acid sequence encoding Vδ1 and the nucleic acid sequence encoding Vγ4. After the viral vector is engineered, the virus can be used to transduce γδ T cells to generate a population of γδ T cells that express Vδ1 and Vγ4.

### Example 13. Generation of a Treq expressing Vγ4δ1

Regulatory T cells (Tregs) can be isolated from a patient sample and transduced to express Vγ4δ1. In an exemplary method for making Vγ4δ1+ Tregs, viral vectors (e.g., a lentiviral vector, adenoviral vector, or adeno-associated viral vector) containing a constitutively active promoter (e.g., any constitutively active known in the art, such as a human retroviral LTR, SFFV, EIF1α, or PGK) and the nucleic acid sequences encoding Vδ1 and Vγ4 are engineered using standard techniques known in the art. To express both Vδ1 and Vγ4, a bicistronic expression cassette is used in which an IRES sequence is placed between the nucleic acid sequence encoding Vδ1 and the nucleic acid sequence encoding Vγ4. After the viral vector is engineered, the virus can be used to transduce Tregs to generate a population of Tregs that express Vδ1 and Vγ4.

### Example 14. Generation of an NK cell expressing Vγ4δ1

Natural Killer (NK) cells can be isolated from a patient sample and transduced to express Vγ4δ1. In an exemplary method for making Vγ4δ1+ NK cells, viral vectors (e.g., a lentiviral vector, adenoviral vector, or adeno-associated viral vector) containing a constitutively active promoter (e.g., any constitutively active known in the art, such as a human retroviral LTR, SFFV, EIF1α, or PGK) and the nucleic acid sequences encoding Vδ1 and Vγ4 are engineered using standard techniques known in the art. To express both Vδ1 and Vγ4, a bicistronic expression cassette is used in which an IRES sequence is placed between the nucleic acid sequence encoding Vδ1 and the nucleic acid sequence encoding Vγ4. Viral vectors (e.g., a lentiviral vector, adenoviral vector, or adeno-associated viral vector) containing an NK cell promoter and the nucleic acid sequence encoding CD3 are also engineered using standard techniques known in the art. After the viral vectors are engineered, the Vδ1 and Vγ4 virus and the CD3 virus are used to cotransduce NK cells to generate a population of NK cells that express Vδ1, Vγ4, and CD3.

### Example 15. Gene therapy using BTNL3 and BTNL8

According to the methods disclosed herein, a physician of skill in the art can treat a patient, such as a human patient, so as to reduce or alleviate the symptoms of IBD. To this end, a physician of skill in the art administers to the human patient a virus expressing BTNL3 and BTNL8. Viral vectors (e.g., a lentiviral vector, adenoviral vector, or adeno-associated viral vector) containing the HNF4A promoter and the nucleic acid sequences encoding BTNL3 and BTNL8 are engineered using standard techniques known in the art. To express both BTNL3 and BTNL8, a bicistronic expression cassette is used in which an IRES sequence is placed between the nucleic acid sequence encoding BTNL3 and the nucleic acid sequence encoding BTNL8. A therapeutically effective amount of the virus is administered to the patient by intravenous administration to treat IBD. The treatment is administered once, or, optionally, repeated one or more times, e.g., once monthly, once bimonthly, three times annually, twice annually, or once annually. The patient is evaluated, for instance, 2 weeks, 1 month, 2 months, 3 months, 4 months, 5 months, 6 months or more following administration of the virus depending on the route of administration used for treatment. A finding of a reduction of one or more IBD symptoms following administration of a virus provides an indication that the treatment has successfully treated IBD.

### Example 16. Gene therapy using HNF4A

According to the methods disclosed herein, a physician of skill in the art can treat a patient, such as a human patient (e.g., a human expressing at least one WT copy of BTNL3 and BTNL8), so as to reduce or alleviate the symptoms of IBD. To this end, a physician of skill in the art administers to the human patient a virus expressing HNF4A. Viral vectors (e.g., a lentiviral vector, adenoviral vector, or adeno-associated viral vector) containing the HNF4A promoter and the nucleic acid sequence encoding HNF4A are engineered using standard techniques known in the art. The virus is administered in a therapeutically effective amount by intravenous administration to treat IBD. The treatment can be administered once, or, optionally, repeated one or more times, e.g., once monthly, once bimonthly, three times annually, twice annually, or once annually. The patient is evaluated, for instance, 2 weeks, 1 month, 2 months, 3 months, 4 months, 5 months, 6 months or more following administration of the virus depending on the route of administration used for treatment. A finding of a reduction of one or more IBD symptoms following administration of a virus provides an indication that the treatment has successfully treated IBD.

### Example 17. BTNL polymorphisms and association with disease

As shown in FIGS. 27A-27B, L8 *3 and L3B30.2cl were investigated for their ability to induce TCR downregulation. Donors with known disease states were genotyped for the indicated polymorphisms. Those donors who are either homozygous or compound heterozygous tended to be non-responders, while negative controls were responders. Responsiveness is shown in FIGS. 28A-28B.

Association with the polymorphisms was assessed by genotyping a cohort of 2048 Crohn's disease patients and 1879 healthy controls. As shown in FIG. 29, homozygosity for each of the polymorphisms did not show a significant association with Crohn's disease incidence, but carriage of two or more BTNL polymorphisms is associated with Crohn's disease. L3B30.2 frequency is also associated with Crohn's disease, as well as with ulcerative colitis (UC N=1932). L3B30.2 frequency: 3.4% Controls; 4.7% UC p=0.003; 4.9% Crohn's disease p=0.0009; 4.8% IBD p=0.0004.

### Example 18. Dvsregulation of colonic γδ T cell surface phenotype.

The expression of a gut-homing integrin, CD103, in γδ+Vδ2- T cells was measured in normal and active UC explant cultures and in different IBD disease subtypes, as shown in FIGS. 30A-30B. Similarly, the expression of 2B4, a costimulatory molecule, was also measured. Downregulation was associated with disease for both surface markers as compared with normal controls.

### Other Embodiments

Various modifications and variations of the described invention will be apparent to those skilled in the art without departing from the scope and spirit of the invention. Although the invention has been described in connection with specific embodiments, it should be understood that the invention as claimed should not be unduly limited to such specific embodiments. Indeed, various modifications of the described modes for carrying out the invention that are obvious to those skilled in the art are intended to be within the scope of the invention. Other embodiments are in the claims.

The following clauses, describing aspects of the invention, are part of the description
1. An isolated Vy4+ cell expressing a heterologous protein.
2. The isolated Vy4+ cell of clause 1, wherein Vγ4 is the heterologous protein, and the Vy4+ cell is derived from a Vγ4- cell.
3. The isolated Vy4+ cell of clause 1 or 2, wherein the Vγ4- cell is an immune cell.
4. The isolated Vy4+ cell of clause 3, wherein the immune cell is a lymphocyte.
5. The isolated Vy4+ cell of clause 4, wherein the lymphocyte is a T cell or an NK cell.
6. The isolated Vy4+ cell of clause 5, wherein the T cell is a γδ T cell.
7. The isolated Vy4+ cell of clause 6, wherein the γδ T cell is a Vδ2- cell.
8. The isolated Vy4+ cell of any one of clauses 1-7, wherein the Vy4+ cell is derived from a human cell.
9. The isolated Vy4+ cell of clause 1, wherein Vγ4 is an endogenously expressed protein.
10. The isolated Vy4+ cell of any one of clauses 1-9, further expressing a surface marker associated with Vy4+ γδ T cells.
11. The isolated Vy4+ cell of clause 10, wherein the surface marker is selected from CD103 or 2B4.
12. The isolated Vy4+ cell of clauses 10 or 11, wherein the surface marker is heterologous.
13. The isolated Vy4+ cell of any one of clauses 1 to 12, wherein the cell is derived from an induced pluripotent stem cell (iPSC).
14. A composition comprising a population of 10⁶-10¹⁰cells, wherein at least 10% of the population is a population of the isolated Vy4+ T cells of any of clauses 1-13.
15. A vector comprising a polynucleotide encoding a Vγ4 protein and optionally a polynucleotide encoding a Vδ1 protein.
16. The vector of clause 15, further comprising a polynucleotide sequence encoding a CD3 protein.
17. A vector comprising a polynucleotide encoding a BTNL3 protein and/or a polynucleotide sequence encoding a BTNL8 protein.
18. The vector of clause 17, wherein the vector encodes a BTNL3 protein and a BTNL8 protein.
19. A vector comprising a polynucleotide encoding an HNF4A protein.
20. The vector of any one of clauses 15-19, wherein the vector is a viral vector.
21. The vector of clause 20, wherein the viral vector is a lentiviral vector, an adenoviral vector, or an adeno-associated viral (AAV) vector.
22. A composition comprising the vector of any one of clauses 15-21.
23. The composition of clause 14 or 22 for use in a method of increasing the number or frequency of Vγ4+ cells in the gut of a subject.
24. The composition of clause 14 or 22 for use in a method of treating inflammation in the gut of a subject.
25. The composition of clause 24, wherein the inflammation in the gut of the subject is associated with inflammatory bowel disease (IBD).
26. The composition of clause 25 wherein the IBD is Crohn's disease, and/or ulcerative colitis.
27. A method of treating inflammation in the gut of a subject having a decreased expression level of BTNL3 and/or BTNL8, relative to a reference expression level, the method comprising administering to the subject the isolated Vγ4+ cell of any one of clauses 1-13, the vector of any one of clauses 14-21, or the composition of any one of clauses 22-26.
28. The method of clause 27, wherein the decreased expression level of BTNL3 and/or BTNL8 is the result of a mutation in a polynucleotide sequence encoding BTNL3 and/or BTNL8.
29. The method of clause 27 or 28, wherein the mutation is characterized by reduced or ablated trafficking of BTNL3 and/or BTNL8 to a cell surface.
30. The method of any one of clauses 27-29, wherein the mutation is a deletion variant, a fusion variant and/or a single nucleotide polymorphism (SNP).
31. The method of any one of clauses 27-30, wherein the mutation is one or more SNP in a BTNL3 intron.
32. The method of any one of clauses 27-30, wherein the mutation is a fusion of BTNL3 and BTNL8.
33. The method of any one of clauses 27-32, wherein the mutation is characterized by expression of a BTNL8*3 fusion protein and/or an L3B30.2lc genotype.
34. The method of any one of clauses 27-33, wherein the mutation is a heterozygous mutation.
35. The method of any one of clauses 27-34, wherein more than one type of mutation is present.
36. A method of treating inflammation in the gut of a subject having a decreased expression level of BTNL3 and/or BTNL8, relative to a reference expression level, the method comprising administering to the subject a polynucleotide encoding an HNF4A protein.
37. The method of clause 36, wherein the polynucleotide encoding an HNF4A protein is encoded by a viral vector.
38. The method of clause 36 or 37, wherein the viral vector is a lentiviral vector, an adenoviral vector, or an AAV vector.
39. A method of increasing the number of Vγ4+ cells in a population of γδ T cells in the gut of a subject, the method comprising administering a population of Vγ4+ cells to the subject, wherein the administered population of Vγ4+ cells has been screened for expression of Vγ4.
40. The method of clause 39, wherein the subject has been diagnosed with IBD.
41. The method of clause 10 wherein the IBD is Crohn's disease, and/or ulcerative colitis.
42. The method of clause 39, 40 or 41, wherein the subject has a mutation in a polynucleotide sequence encoding BTNL3 and/or BTNL8.
43. The method of any one of clauses 39-42, wherein the Vy4+ cells administered to the subject are the isolated Vγ4+ cells of any one of clauses 1-13.
44. The method of any one of clauses 39-42, wherein the Vγ4+ cells administered to the subject have not been modified to express a heterologous protein.
45. The method of any one of clauses 39-44, wherein the population of Vy4+ cells administered to the subject increases the number of Vy4+ cells in the gut of the subject to a number effective to alleviate one or more symptoms of inflammation in the gut.
46. The method of clause 45, wherein the inflammation in the gut is associated with IBD.
47. The method of clause 46 wherein the IBD is Crohn's disease, and/or ulcerative colitis.
48. The method of any one of clauses 39-47, further comprising administering one or more additional therapeutic agents to the subject.
49. A method of identifying a mutation in a polynucleotide sequence encoding BTNL3 and/or BTNL8, the method comprising:
   (a) comparing a level of a polynucleotide sequence associated with a deletion variant in a polynucleotide sequence encoding BTNL3 and/or BTNL8 in a sample of the subject with a reference level of the polynucleotide sequence associated with the deletion variant, wherein an increased level of the polynucleotide sequence associated with a deletion variant in a polynucleotide sequence encoding BTNL3 and BTNL8 in the sample of the subject, relative to the reference level, indicates the presence of the mutation in a polynucleotide sequence encoding BTNL3 and/or BTNL8; or
   (b) comparing a level of a polynucleotide sequence encoding BTNL3 and/or BTNL8 in a sample of the subject with a reference level of the polynucleotide sequence encoding BTNL3 and BTNL8,
   wherein a decreased level of the polynucleotide sequence encoding BTNL3 and/or BTNL8 in the sample of the subject, relative to the reference level, indicates the presence of the mutation in a polynucleotide sequence encoding BTNL3 and BTNL8.
50. The method of clause 49, wherein the reference level is of a sample having wild-type BTNL3 and BTNL8 genes.
51. The method of clause 49 or 50, wherein the mutation is characterized by reduced or ablated trafficking of BTNL3 and/or BTNL8 to a cell surface.
52. The method of any one of clauses 49-51, wherein the mutation is a deletion variant, a fusion variant, and/or an SNP.
53. The method of any one of clauses 49-52, wherein the mutation is an SNP in an intron in the BTNL3 intron.
54. The method of any one of clauses 49-52, wherein the mutation is a fusion of BTNL3 and BTNL8.
55. The method of any one of clauses 49-52, wherein the mutation is characterized by expression of a BTNL8*3 fusion protein and/or an L3B30.2lc genotype.
56. The method of any one of clauses 49-55, wherein the mutation is a heterozygous mutation.
57. The method of any one of clauses 49-56, wherein more than one type of mutation is present.
58. A method of identifying a subject as likely to develop IBD, the method comprising:
   (a) determining whether the subject has a mutation in a polynucleotide sequence encoding BTNL3 and/or BTNL8; and
   (b) based on the presence of the mutation, identifying the subject as likely to develop IBD.
59. The method of clause 58, wherein step (a) comprises the method of any one of clauses 49-57.
60. The method of clause 58 or 59 wherein the IBD is Crohn's disease, and/or ulcerative colitis.
61. The method of clause 58, 59 or 60, further comprising:
   (c) treating the subject according to the methods of any one of clauses 27-48.

## Claims

1. A population of Vy4+ cells for use in a method of increasing the number of Vy4+ cells in a population of γδ T cells in the gut of a subject, the method comprising administering the population of Vγ4+ cells to the subject, wherein the administered population of Vγ4+ cells has been screened for expression of Vγ4.

2. The population of Vγ4+ cells for use according to claim 1, wherein the subject has been diagnosed with inflammatory bowel disease (IBD).

3. The population of Vγ4+ cells for use according to claim 2, wherein the IBD is Crohn's disease and/or ulcerative colitis.

4. The population of Vγ4+ cells for use according to any one of claims 1-3, wherein the subject has a mutation in a polynucleotide sequence encoding BTNL3 and/or BTNL8.

5. The population of Vγ4+ cells for use according to claim 4, wherein the mutation is **characterized by** reduced or ablated trafficking of BTNL3 and/or BTNL8 to a cell surface.

6. The population of Vγ4+ cells for use according to claim 4 or 5, wherein the mutation is **characterized by** expression of a BTNL8*3 fusion protein and/or an L3B30.2lc genotype.

7. The population of Vγ4+ cells for use according to any one of claims 1-6, wherein the Vy4+ cells administered to the subject are isolated Vy4+ cells that express a heterologous protein.

8. The population of Vγ4+ cells for use according to any one of claims 1-7, wherein the population of Vγ4+ cells administered to the subject increases the number of Vy4+ cells in the gut of the subject to a number effective to alleviate one or more symptoms of inflammation in the gut.

9. The population of Vγ4+ cells for use according to claim 8, wherein the inflammation in the gut is associated with IBD.

10. The population of Vγ4+ cells for use according to claim 9, wherein the IBD is Crohn's disease and/or ulcerative colitis.

11. A method of identifying a mutation in a polynucleotide sequence encoding BTNL3 and/or BTNL8, the method comprising:
(a) comparing a level of a polynucleotide sequence associated with a deletion variant in a polynucleotide sequence encoding BTNL3 and/or BTNL8 in a sample of a subject with a reference level of the polynucleotide sequence associated with the deletion variant, wherein an increased level of the polynucleotide sequence associated with a deletion variant in a polynucleotide sequence encoding BTNL3 and BTNL8 in the sample of the subject, relative to the reference level, indicates the presence of the mutation in a polynucleotide sequence encoding BTNL3 and/or BTNL8; or
(b) comparing a level of a polynucleotide sequence encoding BTNL3 and/or BTNL8 in a sample of a subject with a reference level of the polynucleotide sequence encoding BTNL3 and BTNL8;
wherein a decreased level of the polynucleotide sequence encoding BTNL3 and/or BTNL8 in the sample of the subject, relative to the reference level, indicates the presence of the mutation in a polynucleotide sequence encoding BTNL3 and BTNL8.

12. The method of claim 11, wherein the mutation is **characterized by** reduced or ablated trafficking of BTNL3 and/or BTNL8 to a cell surface.

13. The method of claim 11 or 12, wherein the mutation is a single nucleotide polymorphism (SNP) in an intron in the BTNL3 intron.

14. The method of claim 11 or 12, wherein the mutation is a fusion of BTNL3 and BTNL8.

15. The method of claim 11 or 12, wherein the mutation is **characterized by** expression of a BTNL8*3 fusion protein and/or an L3B30.2lc genotype.
